(19) **Europäisches Patentamt / European Patent Office / Office européen des brevets**

(11) **EP 4 585 618 A1**

(12) # EUROPEAN PATENT APPLICATION
published in accordance with Art. 153(4) EPC

(43) Date of publication:
**16.07.2025 Bulletin 2025/29**

(21) Application number: **23862498.5**

(22) Date of filing: **08.09.2023**

(51) International Patent Classification (IPC):
**C07K 16/28** (2006.01)     **A61K 39/395** (2006.01)
**A61P 35/00** (2006.01)

(52) Cooperative Patent Classification (CPC):
**A61K 39/395; A61P 35/00; C07K 16/28**

(86) International application number:
**PCT/CN2023/117653**

(87) International publication number:
**WO 2024/051804 (14.03.2024 Gazette 2024/11)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **08.09.2022 CN 202211093055**

(71) Applicants:
• **Jiangsu Hengrui Pharmaceuticals Co., Ltd.
Lianyungang, Jiangsu 222047 (CN)**
• **Shanghai Hengrui Pharmaceutical Co., Ltd.
Shanghai 200245 (CN)**

(72) Inventors:
• **YE, Xin
Shanghai 200245 (CN)**
• **SUN, Le
Shanghai 200245 (CN)**
• **JIN, Xinsheng
Shanghai 200245 (CN)**
• **YING, Hua
Shanghai 200245 (CN)**

(74) Representative: **Regimbeau
20, rue de Chazelles
75847 Paris Cedex 17 (FR)**

Remarks:
The complete document including Reference Table(s) and the Sequence Listing(s) can be downloaded from the EPO website

(54) **ANTI-ILT4 ANTIBODY AND PHARMACEUTICAL USE THEREOF**

(57) The present invention provides an anti-ILT4 antibody and pharmaceutical use thereof.

EP 4 585 618 A1

## Description

The present application claims priority to Chinese Patent Application No. 202211093055.2 (filed on September 8, 2022).

## TECHNICAL FIELD

The present disclosure pertains to the field of biotechnology. Specifically, the present disclosure relates to an anti-ILT4 antibody and use thereof.

## BACKGROUND

The statements herein merely provide background information related to the present disclosure and may not necessarily constitute the prior art.

Immune checkpoints are molecules expressed on immune cells that can regulate the extent of the immune response. They play a crucial role in preventing autoimmune diseases. When the human body's immune function is stimulated, it becomes activated but does not become overly activated, thanks to immune checkpoints that act as a "brake" in the activation of the immune system to keep the activation within the normal range. Tumor cells can overexpress some immune checkpoint ligands to activate them, thereby inhibiting immune cell functions and evading immune surveillance. Common targeted therapies against T-cell immune checkpoints such as CTLA-4 and PD-1 have achieved significant success. However, due to compensatory mechanisms involving other immune checkpoints, these inhibitors also suffer from low efficiency or resistance. There is still a clinical need to develop other potential immune checkpoint targets.

Immunoglobulin-like transcript 4 (ILT4, also known as LILRB2) belongs to the LILR (leukocyte immunoglobulin-like receptor) family, which has 11 members in total. These members are classified into three categories based on whether their intracellular domains contain ITAM or ITIM. ILT4 is an inhibitory LILRB and is considered to function as an immune checkpoint. ILT4 is expressed on myeloid-derived immune cells, such as macrophages, dendritic cells, and granulocytes. It plays a physiological role in binding to MHC-I molecules to regulate immune balance and homeostasis. In tumor biology, the expression level of ILT4 is positively correlated with the number of myeloid-derived suppressor cells (MDSCs), which are a major cause of resistance to CTLA-4 and PD-1 antibodies. In addition, one of ILT4's ligands, HLA-G (non-classical MHC-I), is reported to be widely expressed in various tumor tissues and is considered an important mechanism for immune evasion of tumor cells. Targeting ILT4 can induce the transformation of macrophages to the pro-inflammatory M1 phenotype, enhance antigen presentation by APCs, and ultimately activate T cells. Meanwhile, ILT4 antibodies can alter the tumor microenvironment to reduce the suppression of T cells by MDSCs.

At present, Merck's MK-4830 (WO2018187518A1) and Jounce's JTX-8064 (WO2019126514A2) have entered phase 2 and phase 1 clinical trials, respectively. According to clinical data released by Merck, the combination of MK-4830 and the PD-1 antibody Keytruda achieved an ORR of 24% in 34 patients with metastatic or advanced solid tumors. Among 11 patients who had experienced disease progression after PD-1 targeted therapy, 5 patients (45%) were in remission, indicating that the ILT4 antibody has a certain potential to overcome resistance to PD-1 targeted therapy. In addition, the adverse reactions observed with the combination of MK-4830 and Keytruda were similar to those observed with Keytruda alone, suggesting that the ILT4 antibody does not raise significant safety concerns.

ILT4 plays an immune negative regulatory role. Inhibitory antibodies targeting it can effectively activate immune cells and can therefore be used as a beneficial complement to existing immune checkpoint therapies.

## SUMMARY

The present disclosure provides an anti-ILT4 antibody comprising a heavy chain variable region and a light chain variable region, wherein the heavy chain variable region comprises a HCDR1, a HCDR2, and a HCDR3, and the light chain variable region comprises a LCDR1, a LCDR2, and a LCDR3, wherein:

    a. the HCDR1, the HCDR2, and the HCDR3 of the heavy chain variable region comprise amino acid sequences of a HCDR1, a HCDR2, and a HCDR3, respectively, in SEQ ID NO: 184 or 185, and the LCDR1, the LCDR2, and the LCDR3 of the light chain variable region comprise amino acid sequences of a LCDR1, a LCDR2, and a LCDR3, respectively, in SEQ ID NO: 187 or 186; or the HCDR1, the HCDR2, and the HCDR3 of the heavy chain variable region comprise amino acid sequences of a HCDR1, a HCDR2, and a HCDR3, respectively, in SEQ ID NO: 32, and the LCDR1, the LCDR2, and the LCDR3 of the light chain variable region comprise amino acid sequences of a LCDR1, a LCDR2, and a LCDR3, respectively, in SEQ ID NO: 33; or

    b. the HCDR1, the HCDR2, and the HCDR3 of the heavy chain variable region comprise amino acid sequences of a HCDR1, a HCDR2, and a HCDR3, respectively, in any one of SEQ ID NO: 211, 205, 206, 207, 208, 209, or 210, and the LCDR1, the LCDR2, and the LCDR3 of the light chain variable region comprise amino acid sequences of a LCDR1, a LCDR2, and a LCDR3, respectively, in any one of SEQ ID NO: 212, 213, or 214; or the HCDR1, the HCDR2, and the HCDR3 of the heavy chain variable region comprise amino acid sequences of a HCDR1, a HCDR2, and a HCDR3, respectively, in SEQ ID NO: 40, and the LCDR1, the LCDR2, and the LCDR3 of the light chain variable region comprise amino acid sequences of a LCDR1, a LCDR2, and a LCDR3, respectively, in SEQ ID NO: 41; or

c. the HCDR1, the HCDR2, and the HCDR3 of the heavy chain variable region comprise amino acid sequences of a HCDR1, a HCDR2, and a HCDR3, respectively, in any one of SEQ ID NO: 181, 176, 177, 178, 179, or 180, and the LCDR1, the LCDR2, and the LCDR3 of the light chain variable region comprise amino acid sequences of a LCDR1, a LCDR2, and a LCDR3, respectively, in SEQ ID NO: 183 or 182; or the HCDR1, the HCDR2, and the HCDR3 of the heavy chain variable region comprise amino acid sequences of a HCDR1, a HCDR2, and a HCDR3, respectively, in SEQ ID NO: 28, and the LCDR1, the LCDR2, and the LCDR3 of the light chain variable region comprise amino acid sequences of a LCDR1, a LCDR2, and a LCDR3, respectively, in SEQ ID NO: 29; or

d. the HCDR1, the HCDR2, and the HCDR3 of the heavy chain variable region comprise amino acid sequences of a HCDR1, a HCDR2, and a HCDR3, respectively, in any one of SEQ ID NO: 193, 190, 191, or 192, and the LCDR1, the LCDR2, and the LCDR3 of the light chain variable region comprise amino acid sequences of a LCDR1, a LCDR2, and a LCDR3, respectively, in any one of SEQ ID NO: 194, 195, or 196; or the HCDR1, the HCDR2, and the HCDR3 of the heavy chain variable region comprise amino acid sequences of a HCDR1, a HCDR2, and a HCDR3, respectively, in SEQ ID NO: 10, and the LCDR1, the LCDR2, and the LCDR3 of the light chain variable region comprise amino acid sequences of a LCDR1, a LCDR2, and a LCDR3, respectively, in SEQ ID NO: 11; or

e. the HCDR1, the HCDR2, and the HCDR3 of the heavy chain variable region comprise amino acid sequences of a HCDR1, a HCDR2, and a HCDR3, respectively, in SEQ ID NO: 163, and the LCDR1, the LCDR2, and the LCDR3 of the light chain variable region comprise amino acid sequences of a LCDR1, a LCDR2, and a LCDR3, respectively, in SEQ ID NO: 225 or 164; or

f. the HCDR1, the HCDR2, and the HCDR3 of the heavy chain variable region comprise amino acid sequences of a HCDR1, a HCDR2, and a HCDR3, respectively, in SEQ ID NO: 197 or 198, and the LCDR1, the LCDR2, and the LCDR3 of the light chain variable region comprise amino acid sequences of a LCDR1, a LCDR2, and a LCDR3, respectively, in SEQ ID NO: 199 or 200; or the HCDR1, the HCDR2, and the HCDR3 of the heavy chain variable region comprise amino acid sequences of a HCDR1, a HCDR2, and a HCDR3, respectively, in SEQ ID NO: 36, and the LCDR1, the LCDR2, and the LCDR3 of the light chain variable region comprise amino acid sequences of a LCDR1, a LCDR2, and a LCDR3, respectively, in SEQ ID NO: 37; or

g. the HCDR1, the HCDR2, and the HCDR3 of the heavy chain variable region comprise amino acid sequences of a HCDR1, a HCDR2, and a HCDR3, respectively, in any one of SEQ ID NO: 217, 218, 219, 220, or 221, and the LCDR1, the LCDR2, and the LCDR3 of the light chain variable region comprise amino acid sequences of a LCDR1, a LCDR2, and a LCDR3, respectively, in SEQ ID NO: 222 or 223; or the HCDR1, the HCDR2, and the HCDR3 of the heavy chain variable region comprise amino acid sequences of a HCDR1, a HCDR2, and a HCDR3, respectively, in SEQ ID NO: 42, and the LCDR1, the LCDR2, and the LCDR3 of the light chain variable region comprise amino acid sequences of a LCDR1, a LCDR2, and a LCDR3, respectively, in SEQ ID NO: 43; or

h. the HCDR1, the HCDR2, and the HCDR3 of the heavy chain variable region comprise amino acid sequences of a HCDR1, a HCDR2, and a HCDR3, respectively, in SEQ ID NO: 34, and the LCDR1, the LCDR2, and the LCDR3 of the light chain variable region comprise amino acid sequences of a LCDR1, a LCDR2, and a LCDR3, respectively, in SEQ ID NO: 35; or

i. the HCDR1, the HCDR2, and the HCDR3 of the heavy chain variable region comprise amino acid sequences of a HCDR1, a HCDR2, and a HCDR3, respectively, in SEQ ID NO: 18, and the LCDR1, the LCDR2, and the LCDR3 of the light chain variable region comprise amino acid sequences of a LCDR1, a LCDR2, and a LCDR3, respectively, in SEQ ID NO: 19; or

j. the HCDR1, the HCDR2, and the HCDR3 of the heavy chain variable region comprise amino acid sequences of a HCDR1, a HCDR2, and a HCDR3, respectively, in SEQ ID NO: 20, and the LCDR1, the LCDR2, and the LCDR3 of the light chain variable region comprise amino acid sequences of a LCDR1, a LCDR2, and a LCDR3, respectively, in SEQ ID NO: 21; or

k. the HCDR1, the HCDR2, and the HCDR3 of the heavy chain variable region comprise amino acid sequences of a HCDR1, a HCDR2, and a HCDR3, respectively, in SEQ ID NO: 24, and the LCDR1, the LCDR2, and the LCDR3 of the light chain variable region comprise amino acid sequences of a LCDR1, a LCDR2, and a LCDR3, respectively, in SEQ ID NO: 25; or

l. the HCDR1, the HCDR2, and the HCDR3 of the heavy chain variable region comprise amino acid sequences of a HCDR1, a HCDR2, and a HCDR3, respectively, in SEQ ID NO: 44, and the LCDR1, the LCDR2, and the LCDR3 of the light chain variable region comprise amino acid sequences of a LCDR1, a LCDR2, and a LCDR3, respectively, in SEQ ID NO: 45; or

m. the HCDR1, the HCDR2, and the HCDR3 of the heavy chain variable region comprise amino acid sequences of a HCDR1, a HCDR2, and a HCDR3, respectively, in SEQ ID NO: 46, and the LCDR1, the LCDR2, and the LCDR3 of the light chain variable region comprise amino acid sequences of a LCDR1, a LCDR2, and a LCDR3, respectively, in SEQ ID NO: 47; or

n. the HCDR1, the HCDR2, and the HCDR3 of the heavy chain variable region comprise amino acid sequences of a HCDR1, a HCDR2, and a HCDR3, respectively, in SEQ ID NO: 48, and the LCDR1, the LCDR2, and the LCDR3 of the light chain variable region comprise amino acid sequences of a LCDR1, a LCDR2, and a LCDR3, respectively, in SEQ ID NO: 49; or

o. the HCDR1, the HCDR2, and the HCDR3 of the heavy chain variable region comprise amino acid sequences of a HCDR1, a HCDR2, and a HCDR3, respectively, in SEQ ID NO: 50, and the LCDR1, the LCDR2, and the LCDR3 of the light chain variable region comprise amino acid sequences of a LCDR1, a LCDR2, and a LCDR3, respectively, in SEQ ID NO: 51; or

p. the HCDR1, the HCDR2, and the HCDR3 of the heavy chain variable region comprise amino acid sequences of a HCDR1, a HCDR2, and a HCDR3, respectively, in SEQ ID NO: 4, and the LCDR1, the LCDR2, and the LCDR3 of the light chain variable region comprise amino acid sequences of a LCDR1, a LCDR2, and a LCDR3, respectively, in SEQ ID NO: 5; or

q. the HCDR1, the HCDR2, and the HCDR3 of the heavy chain variable region comprise amino acid sequences of a HCDR1, a HCDR2, and a HCDR3, respectively, in SEQ ID NO: 6, and the LCDR1, the LCDR2, and the LCDR3 of the light chain variable region comprise amino acid sequences of a LCDR1, a LCDR2, and a LCDR3, respectively, in SEQ ID NO: 7; or

r. the HCDR1, the HCDR2, and the HCDR3 of the heavy chain variable region comprise amino acid sequences of a HCDR1, a HCDR2, and a HCDR3, respectively, in SEQ ID NO: 8, and the LCDR1, the LCDR2, and the LCDR3 of the light chain variable region comprise amino acid sequences of a LCDR1, a LCDR2, and a LCDR3, respectively, in SEQ ID NO: 9; or

s. the HCDR1, the HCDR2, and the HCDR3 of the heavy chain variable region comprise amino acid sequences of a HCDR1, a HCDR2, and a HCDR3, respectively, in SEQ ID NO: 12, and the LCDR1, the LCDR2, and the LCDR3 of the light chain variable region comprise amino acid sequences of a LCDR1, a LCDR2, and a LCDR3, respectively, in SEQ ID NO: 13; or

t. the HCDR1, the HCDR2, and the HCDR3 of the heavy chain variable region comprise amino acid sequences of a HCDR1, a HCDR2, and a HCDR3, respectively, in SEQ ID NO: 14, and the LCDR1, the LCDR2, and the LCDR3 of the light chain variable region comprise amino acid sequences of a LCDR1, a LCDR2, and a LCDR3, respectively, in SEQ ID NO: 15; or

u. the HCDR1, the HCDR2, and the HCDR3 of the heavy chain variable region comprise amino acid sequences of a HCDR1, a HCDR2, and a HCDR3, respectively, in SEQ ID NO: 16, and the LCDR1, the LCDR2, and the LCDR3 of the light chain variable region comprise amino acid sequences of a LCDR1, a LCDR2, and a LCDR3, respectively, in SEQ ID NO: 17; or

v. the HCDR1, the HCDR2, and the HCDR3 of the heavy chain variable region comprise amino acid sequences of a HCDR1, a HCDR2, and a HCDR3, respectively, in SEQ ID NO: 22, and the LCDR1, the LCDR2, and the LCDR3 of the light chain variable region comprise amino acid sequences of a LCDR1, a LCDR2, and a LCDR3, respectively, in SEQ ID NO: 23; or

w. the HCDR1, the HCDR2, and the HCDR3 of the heavy chain variable region comprise amino acid sequences of a HCDR1, a HCDR2, and a HCDR3, respectively, in SEQ ID NO: 26, and the LCDR1, the LCDR2, and the LCDR3 of the light chain variable region comprise amino acid sequences of a LCDR1, a LCDR2, and a LCDR3, respectively, in SEQ ID NO: 27; or

x. the HCDR1, the HCDR2, and the HCDR3 of the heavy chain variable region comprise amino acid sequences of a HCDR1, a HCDR2, and a HCDR3, respectively, in SEQ ID NO: 30, and the LCDR1, the LCDR2, and the LCDR3 of the light chain variable region comprise amino acid sequences of a LCDR1, a LCDR2, and a LCDR3, respectively, in SEQ ID NO: 31; or

y. the HCDR1, the HCDR2, and the HCDR3 of the heavy chain variable region comprise amino acid sequences of a HCDR1, a HCDR2, and a HCDR3, respectively, in SEQ ID NO: 38, and the LCDR1, the LCDR2, and the LCDR3 of the light chain variable region comprise amino acid sequences of a LCDR1, a LCDR2, and a LCDR3, respectively, in SEQ ID NO: 39.

In some embodiments, provided is the aforementioned anti-ILT4 antibody, wherein:

a. the HCDR1, the HCDR2, and the HCDR3 of the heavy chain variable region comprise amino acid sequences of a HCDR1, a HCDR2, and a HCDR3, respectively, in SEQ ID NO: 184 or 185, and the LCDR1, the LCDR2, and the LCDR3 of the light chain variable region comprise amino acid sequences of a LCDR1, a LCDR2, and a LCDR3, respectively, in SEQ ID NO: 187 or 186; or the HCDR1, the HCDR2, and the HCDR3 of the heavy chain variable region comprise amino acid sequences of a HCDR1, a HCDR2, and a HCDR3, respectively, in SEQ ID NO: 32, and the LCDR1, the LCDR2, and the LCDR3 of the light chain variable region comprise amino acid sequences of a LCDR1, a LCDR2, and a LCDR3, respectively, in SEQ ID NO: 33; or

b. the HCDR1, the HCDR2, and the HCDR3 of the heavy chain variable region comprise amino acid sequences of a HCDR1, a HCDR2, and a HCDR3, respectively, in any one of SEQ ID NO: 211, 205, 206, 207, 208, 209, or 210, and the LCDR1, the LCDR2, and the LCDR3 of the light chain variable region comprise amino acid sequences of a LCDR1, a LCDR2, and a LCDR3, respectively, in any one of SEQ ID NO: 212, 213, or 214; or the HCDR1, the HCDR2, and the HCDR3 of the heavy chain variable region comprise amino acid sequences of a HCDR1, a HCDR2, and a HCDR3, respectively, in SEQ ID NO: 40, and the LCDR1, the LCDR2, and the LCDR3 of the light chain variable region comprise amino acid sequences of a LCDR1, a LCDR2, and a LCDR3, respectively, in SEQ ID NO: 41; or

c. the HCDR1, the HCDR2, and the HCDR3 of the heavy chain variable region comprise amino acid sequences of a HCDR1, a HCDR2, and a HCDR3, respectively, in any one of SEQ ID NO: 181, 176, 177, 178, 179, or 180, and the LCDR1, the LCDR2, and the LCDR3 of the light chain variable region comprise amino acid sequences of a LCDR1, a LCDR2, and a LCDR3, respectively, in SEQ ID NO: 183 or 182; or the HCDR1, the HCDR2, and the HCDR3 of the heavy chain variable region comprise amino acid sequences of a HCDR1, a HCDR2, and a HCDR3, respectively, in SEQ ID NO: 28, and the LCDR1, the LCDR2, and the LCDR3 of the light chain variable region comprise amino acid sequences of a LCDR1, a LCDR2, and a LCDR3, respectively, in SEQ ID NO: 29; or

d. the HCDR1, the HCDR2, and the HCDR3 of the heavy chain variable region comprise amino acid sequences of a HCDR1, a HCDR2, and a HCDR3, respectively, in any one of SEQ ID NO: 193, 190, 191, or 192, and the LCDR1, the LCDR2, and the LCDR3 of the light chain variable region comprise amino acid sequences of a LCDR1, a LCDR2, and a LCDR3, respectively, in any one of SEQ ID NO: 194, 195, or 196; or the HCDR1, the HCDR2, and the HCDR3 of the heavy chain variable region comprise amino acid sequences of a HCDR1, a HCDR2, and a HCDR3, respectively, in SEQ ID NO: 10, and the LCDR1, the LCDR2, and the LCDR3 of the light chain variable region comprise amino acid sequences of a LCDR1, a LCDR2, and a LCDR3, respectively, in SEQ ID NO: 11; or

e. the HCDR1, the HCDR2, and the HCDR3 of the heavy chain variable region comprise amino acid sequences of a HCDR1, a HCDR2, and a HCDR3, respectively, in SEQ ID NO: 163, and the LCDR1, the LCDR2, and the LCDR3 of the light chain variable region comprise amino acid sequences of a LCDR1, a LCDR2, and a LCDR3, respectively, in SEQ ID NO: 225 or 164; or

f. the HCDR1, the HCDR2, and the HCDR3 of the heavy chain variable region comprise amino acid sequences of a HCDR1, a HCDR2, and a HCDR3, respectively, in SEQ ID NO: 197 or 198, and the LCDR1, the LCDR2, and the LCDR3 of the light chain variable region comprise amino acid sequences of a LCDR1, a LCDR2, and a LCDR3, respectively, in SEQ ID NO: 199 or 200; or the HCDR1, the HCDR2, and the HCDR3 of the heavy chain variable region comprise amino acid sequences of a HCDR1, a HCDR2, and a HCDR3, respectively, in SEQ ID NO: 36, and the LCDR1, the LCDR2, and the LCDR3 of the light chain variable region comprise amino acid sequences of a LCDR1, a LCDR2, and a LCDR3, respectively, in SEQ ID NO: 37; or

g. the HCDR1, the HCDR2, and the HCDR3 of the heavy chain variable region comprise amino acid sequences of a HCDR1, a HCDR2, and a HCDR3, respectively, in any one of SEQ ID NO: 217, 218, 219, 220, or 221, and the LCDR1, the LCDR2, and the LCDR3 of the light chain variable region comprise amino acid sequences of a LCDR1, a LCDR2, and a LCDR3, respectively, in SEQ ID NO: 222 or 223; or the HCDR1, the HCDR2, and the HCDR3 of the heavy chain variable region comprise amino acid sequences of a HCDR1, a HCDR2, and a HCDR3, respectively, in SEQ ID NO: 42, and the LCDR1, the LCDR2, and the LCDR3 of the light chain variable region comprise amino acid sequences of a LCDR1, a LCDR2, and a LCDR3, respectively, in SEQ ID NO: 43; or

h. the HCDR1, the HCDR2, and the HCDR3 of the heavy chain variable region comprise amino acid sequences of a HCDR1, a HCDR2, and a HCDR3, respectively, in SEQ ID NO: 34, and the LCDR1, the LCDR2, and the LCDR3 of the light chain variable region comprise amino acid sequences of a LCDR1, a LCDR2, and a LCDR3, respectively, in SEQ ID NO: 35; or

i. the HCDR1, the HCDR2, and the HCDR3 of the heavy chain variable region comprise amino acid sequences of a HCDR1, a HCDR2, and a HCDR3, respectively, in SEQ ID NO: 18, and the LCDR1, the LCDR2, and the LCDR3 of the light chain variable region comprise amino acid sequences of a LCDR1, a LCDR2, and a LCDR3, respectively, in SEQ ID NO: 19; or

j. the HCDR1, the HCDR2, and the HCDR3 of the heavy chain variable region comprise amino acid sequences of a HCDR1, a HCDR2, and a HCDR3, respectively, in SEQ ID NO: 20, and the LCDR1, the LCDR2, and the LCDR3 of the light chain variable region comprise amino acid sequences of a LCDR1, a LCDR2, and a LCDR3, respectively, in SEQ ID NO: 21; or

k. the HCDR1, the HCDR2, and the HCDR3 of the heavy chain variable region comprise amino acid sequences of a HCDR1, a HCDR2, and a HCDR3, respectively, in SEQ ID NO: 24, and the LCDR1, the LCDR2, and the LCDR3 of the light chain variable region comprise amino acid sequences of a LCDR1, a LCDR2, and a LCDR3, respectively, in SEQ ID NO: 25; or

l. the HCDR1, the HCDR2, and the HCDR3 of the heavy chain variable region comprise amino acid sequences of a HCDR1, a HCDR2, and a HCDR3, respectively, in SEQ ID NO: 44, and the LCDR1, the LCDR2, and the LCDR3 of the light chain variable region comprise amino acid sequences of a LCDR1, a LCDR2, and a LCDR3, respectively, in SEQ ID NO: 45; or

m. the HCDR1, the HCDR2, and the HCDR3 of the heavy chain variable region comprise amino acid sequences of a HCDR1, a HCDR2, and a HCDR3, respectively, in SEQ ID NO: 46, and the LCDR1, the LCDR2, and the LCDR3 of the light chain variable region comprise amino acid sequences of a LCDR1, a LCDR2, and a LCDR3, respectively, in SEQ ID NO: 47; or

n. the HCDR1, the HCDR2, and the HCDR3 of the heavy chain variable region comprise amino acid sequences of a HCDR1, a HCDR2, and a HCDR3, respectively, in SEQ ID NO: 48, and the LCDR1, the LCDR2, and the LCDR3 of the light chain variable region comprise amino acid sequences of a LCDR1, a LCDR2, and a LCDR3, respectively, in SEQ ID NO: 49; or

o. the HCDR1, the HCDR2, and the HCDR3 of the heavy chain variable region comprise amino acid sequences of a HCDR1, a HCDR2, and a HCDR3, respectively, in SEQ ID NO: 50, and the LCDR1, the LCDR2, and the LCDR3 of the light chain variable region comprise amino acid sequences of a LCDR1, a LCDR2, and a LCDR3, respectively, in SEQ ID NO: 51.

In some embodiments, provided is the aforementioned anti-ILT4 antibody, wherein:

a. the HCDR1, the HCDR2, and the HCDR3 of the heavy chain variable region comprise amino acid sequences of a HCDR1, a HCDR2, and a HCDR3, respectively, in SEQ ID NO: 184, and the LCDR1, the LCDR2, and the LCDR3 of the light chain variable region comprise amino acid sequences of a LCDR1, a LCDR2, and a LCDR3, respectively, in SEQ ID NO: 187; or

b. the HCDR1, the HCDR2, and the HCDR3 of the heavy chain variable region comprise amino acid sequences of a HCDR1, a HCDR2, and a HCDR3, respectively, in SEQ ID NO: 211, and the LCDR1, the LCDR2, and the LCDR3 of the light chain variable region comprise amino acid sequences of a LCDR1, a LCDR2, and a LCDR3, respectively, in SEQ ID NO: 212; or

c. the HCDR1, the HCDR2, and the HCDR3 of the heavy chain variable region comprise amino acid sequences of a HCDR1, a HCDR2, and a HCDR3, respectively, in SEQ ID NO: 181, and the LCDR1, the LCDR2, and the LCDR3 of the light chain variable region comprise amino acid sequences of a LCDR1, a LCDR2, and a LCDR3, respectively, in SEQ ID NO: 183; or

d. the HCDR1, the HCDR2, and the HCDR3 of the heavy chain variable region comprise amino acid sequences of a HCDR1, a HCDR2, and a HCDR3, respectively, in SEQ ID NO: 193, and the LCDR1, the LCDR2, and the LCDR3 of the light chain variable region comprise amino acid sequences of a LCDR1, a LCDR2, and a LCDR3, respectively, in SEQ ID NO: 194; or

e. the HCDR1, the HCDR2, and the HCDR3 of the heavy chain variable region comprise amino acid sequences of a HCDR1, a HCDR2, and a HCDR3, respectively, in SEQ ID NO: 163, and the LCDR1, the LCDR2, and the LCDR3 of the light chain variable region comprise amino acid sequences of a LCDR1, a LCDR2, and a LCDR3, respectively, in SEQ ID NO: 225; or

f. the HCDR1, the HCDR2, and the HCDR3 of the heavy chain variable region comprise amino acid sequences of a HCDR1, a HCDR2, and a HCDR3, respectively, in SEQ ID NO: 197, and the LCDR1, the LCDR2, and the LCDR3 of the light chain variable region comprise amino acid sequences of a LCDR1, a LCDR2, and a LCDR3, respectively, in SEQ ID NO: 199; or

g. the HCDR1, the HCDR2, and the HCDR3 of the heavy chain variable region comprise amino acid sequences of a HCDR1, a HCDR2, and a HCDR3, respectively, in SEQ ID NO: 217, and the LCDR1, the LCDR2, and the LCDR3 of the light chain variable region comprise amino acid sequences of a LCDR1, a LCDR2, and a LCDR3, respectively, in SEQ ID NO: 222.

In some embodiments, provided is the aforementioned anti-ILT4 antibody, wherein:

a. the HCDR1, the HCDR2, and the HCDR3 of the heavy chain variable region comprise amino acid sequences of a HCDR1, a HCDR2, and a HCDR3, respectively, in SEQ ID NO: 184, and the LCDR1, the LCDR2, and the LCDR3 of the light chain variable region comprise amino acid sequences of a LCDR1, a LCDR2, and a LCDR3, respectively, in SEQ ID NO: 187; or

b. the HCDR1, the HCDR2, and the HCDR3 of the heavy chain variable region comprise amino acid sequences of a HCDR1, a HCDR2, and a HCDR3, respectively, in SEQ ID NO: 211, and the LCDR1, the LCDR2, and the LCDR3 of the light chain variable region comprise amino acid sequences of a LCDR1, a LCDR2, and a LCDR3, respectively, in SEQ ID NO: 212.

In some embodiments, provided is the aforementioned anti-ILT4 antibody, wherein:

p. the HCDR1, the HCDR2, and the HCDR3 of the heavy chain variable region comprise amino acid sequences of a HCDR1, a HCDR2, and a HCDR3, respectively, in SEQ ID NO: 4, and the LCDR1, the LCDR2, and the LCDR3 of the light chain variable region comprise amino acid sequences of a LCDR1, a LCDR2, and a LCDR3, respectively, in SEQ ID NO: 5; or

q. the HCDR1, the HCDR2, and the HCDR3 of the heavy chain variable region comprise amino acid sequences of a HCDR1, a HCDR2, and a HCDR3, respectively, in SEQ ID NO: 6, and the LCDR1, the LCDR2, and the LCDR3 of the light chain variable region comprise amino acid sequences of a LCDR1, a LCDR2, and a LCDR3, respectively, in SEQ ID NO: 7; or

r. the HCDR1, the HCDR2, and the HCDR3 of the heavy chain variable region comprise amino acid sequences of a HCDR1, a HCDR2, and a HCDR3, respectively, in SEQ ID NO: 8, and the LCDR1, the LCDR2, and the LCDR3 of the light chain variable region comprise amino acid sequences of a LCDR1, a LCDR2, and a LCDR3, respectively, in SEQ ID NO: 9; or

s. the HCDR1, the HCDR2, and the HCDR3 of the heavy chain variable region comprise amino acid sequences of a HCDR1, a HCDR2, and a HCDR3, respectively, in SEQ ID NO: 12, and the LCDR1, the LCDR2, and the LCDR3 of the light chain variable region comprise amino acid sequences of a LCDR1, a LCDR2, and a LCDR3, respectively, in SEQ ID NO: 13; or

t. the HCDR1, the HCDR2, and the HCDR3 of the heavy chain variable region comprise amino acid sequences of a HCDR1, a HCDR2, and a HCDR3, respectively, in SEQ ID NO: 14, and the LCDR1, the LCDR2, and the LCDR3 of the light chain variable region comprise amino acid sequences of a LCDR1, a LCDR2, and a LCDR3, respectively, in SEQ ID NO: 15; or

u. the HCDR1, the HCDR2, and the HCDR3 of the heavy chain variable region comprise amino acid sequences of a HCDR1, a HCDR2, and a HCDR3, respectively, in SEQ ID NO: 16, and the LCDR1, the LCDR2, and the LCDR3 of the light chain variable region comprise amino acid sequences of a LCDR1, a LCDR2, and a LCDR3, respectively, in SEQ ID NO: 17; or

v. the HCDR1, the HCDR2, and the HCDR3 of the heavy chain variable region comprise amino acid sequences of a HCDR1, a HCDR2, and a HCDR3, respectively, in SEQ ID NO: 22, and the LCDR1, the LCDR2, and the LCDR3 of the light chain variable region comprise amino acid sequences of a LCDR1, a LCDR2, and a LCDR3, respectively, in SEQ ID NO: 23; or

w. the HCDR1, the HCDR2, and the HCDR3 of the heavy chain variable region comprise amino acid sequences of a HCDR1, a HCDR2, and a HCDR3, respectively, in SEQ ID NO: 26, and the LCDR1, the LCDR2, and the LCDR3 of the light chain variable region comprise amino acid sequences of a LCDR1, a LCDR2, and a LCDR3, respectively, in SEQ ID NO: 27; or

x. the HCDR1, the HCDR2, and the HCDR3 of the heavy chain variable region comprise amino acid sequences of a HCDR1, a HCDR2, and a HCDR3, respectively, in SEQ ID NO: 30, and the LCDR1, the LCDR2, and the LCDR3 of the light chain variable region comprise amino acid sequences of a LCDR1, a LCDR2, and a LCDR3, respectively, in SEQ ID NO: 31; or

y. the HCDR1, the HCDR2, and the HCDR3 of the heavy chain variable region comprise amino acid sequences of a HCDR1, a HCDR2, and a HCDR3, respectively, in SEQ ID NO: 38, and the LCDR1, the LCDR2, and the LCDR3 of the light chain variable region comprise amino acid sequences of a LCDR1, a LCDR2, and a LCDR3, respectively, in SEQ ID NO: 39.

In some embodiments, provided is the anti-ILT4 antibody according to any one of the above, wherein the HCDR1, the HCDR2, and the HCDR3 of the heavy chain variable region and the LCDR1, the LCDR2, and the LCDR3 of the light chain variable region are defined according to the same numbering scheme selected from the group consisting of Kabat, IMGT, Chothia, AbM, and Contact. In some embodiments, the HCDR1, the HCDR2, and the HCDR3 of the heavy chain variable region and the LCDR1, the LCDR2, and the LCDR3 of the light chain variable region are defined according to the Kabat numbering scheme. In some embodiments, the HCDR1, the HCDR2, and the HCDR3 of the heavy chain variable region and the LCDR1, the LCDR2, and the LCDR3 of the light chain variable region are defined according to the IMGT numbering scheme. In some embodiments, the HCDR1, the HCDR2, and the HCDR3 of the heavy chain variable region and the LCDR1, the LCDR2, and the LCDR3 of the light chain variable region are defined according to the Chothia numbering scheme. In some embodiments, the HCDR1, the HCDR2, and the HCDR3 of the heavy chain variable region and the LCDR1, the LCDR2, and the LCDR3 of the light chain variable region are defined according to the AbM numbering scheme. In some embodiments, the HCDR1, the HCDR2, and the HCDR3 of the heavy chain variable region and the LCDR1, the LCDR2, and the LCDR3 of the light chain variable region are defined according to the Contact numbering scheme.

In some embodiments, provided is the anti-ILT4 antibody according to any one of the above, wherein:

a. in the heavy chain variable region, the HCDR1 comprises an amino acid sequence of SEQ ID NO: 105, the HCDR2 comprises an amino acid sequence of SEQ ID NO: 120, and the HCDR3 comprises an amino acid sequence of SEQ ID NO: 121; and in the light chain variable region, the LCDR1 comprises an amino acid sequence of SEQ ID NO: 114, the LCDR2 comprises an amino acid sequence of SEQ ID NO: 109, and the LCDR3 comprises an amino acid sequence of SEQ ID NO: 119; or

b. in the heavy chain variable region, the HCDR1 comprises an amino acid sequence of SEQ ID NO: 135, the HCDR2 comprises an amino acid sequence of any one of SEQ ID NO: 203, 136, 201, 202, or 204, and the HCDR3 comprises an amino acid sequence of SEQ ID NO: 137; and in the light chain variable region, the LCDR1 comprises an amino acid sequence of SEQ ID NO: 138, the LCDR2 comprises an amino acid sequence of SEQ ID NO: 139, and the LCDR3 comprises an amino acid sequence of SEQ ID NO: 140; or

c. in the heavy chain variable region, the HCDR1 comprises an amino acid sequence of SEQ ID NO: 111, the HCDR2 comprises an amino acid sequence of any one of SEQ ID NO: 173, 112, 174, or 175, and the HCDR3 comprises an amino acid sequence of SEQ ID NO: 113; and in the light chain variable region, the LCDR1 comprises an amino acid sequence of SEQ ID NO: 114, the LCDR2 comprises an amino acid sequence of SEQ ID NO: 115, and the LCDR3 comprises an amino acid sequence of SEQ ID NO: 116; or

d. in the heavy chain variable region, the HCDR1 comprises an amino acid sequence of SEQ ID NO: 69, the HCDR2 comprises an amino acid sequence of any one of SEQ ID NO: 189, 70, or 188, and the HCDR3 comprises an amino acid sequence of SEQ ID NO: 71; and in the light chain variable region, the LCDR1 comprises an amino acid sequence of SEQ ID NO: 61, the LCDR2 comprises an amino acid sequence of SEQ ID NO: 62, and the LCDR3 comprises an amino acid sequence of SEQ ID NO: 72; or

e. in the heavy chain variable region, the HCDR1 comprises an amino acid sequence of SEQ ID NO: 165, the HCDR2 comprises an amino acid sequence of SEQ ID NO: 166, and the HCDR3 comprises an amino acid sequence of SEQ ID NO: 167; and in the light chain variable region, the LCDR1 comprises an amino acid sequence of SEQ ID NO: 224 or 168, the LCDR2 comprises an amino acid sequence of SEQ ID NO: 169, and the LCDR3 comprises an amino acid sequence of SEQ ID NO: 170; or

f. in the heavy chain variable region, the HCDR1 comprises an amino acid sequence of SEQ ID NO: 105, the HCDR2 comprises an amino acid sequence of SEQ ID NO: 125, and the HCDR3 comprises an amino acid sequence of SEQ ID NO: 126; and in the light chain variable region, the LCDR1 comprises an amino acid sequence of SEQ ID NO: 127, the LCDR2 comprises an amino acid sequence of SEQ ID NO: 109, and the LCDR3 comprises an amino acid sequence of SEQ ID NO: 128; or

g. in the heavy chain variable region, the HCDR1 comprises an amino acid sequence of SEQ ID NO: 141, the HCDR2 comprises an amino acid sequence of SEQ ID NO: 215 or 142, and the HCDR3 comprises an amino acid sequence of SEQ ID NO: 216 or 143; and in the light chain variable region, the LCDR1 comprises an amino acid sequence of SEQ ID NO: 144, the LCDR2 comprises an amino acid sequence of SEQ ID NO: 145, and the LCDR3 comprises an amino acid sequence of SEQ ID NO: 146; or

h. in the heavy chain variable region, the HCDR1 comprises an amino acid sequence of SEQ ID NO: 99, the HCDR2 comprises an amino acid sequence of SEQ ID NO: 122, and the HCDR3 comprises an amino acid sequence of SEQ ID NO: 123; and in the light chain variable region, the LCDR1 comprises an amino acid sequence of SEQ ID NO: 114, the LCDR2 comprises an amino acid sequence of SEQ ID NO: 109, and the LCDR3 comprises an amino acid sequence of SEQ ID NO: 124; or

i. in the heavy chain variable region, the HCDR1 comprises an amino acid sequence of SEQ ID NO: 73, the HCDR2 comprises an amino acid sequence of SEQ ID NO: 87, and the HCDR3 comprises an amino acid sequence of SEQ ID NO: 88; and in the light chain variable region, the LCDR1 comprises an amino acid sequence of SEQ ID NO: 61, the LCDR2 comprises an amino acid sequence of SEQ ID NO: 89, and the LCDR3 comprises an amino acid sequence of SEQ ID NO: 90; or

j. in the heavy chain variable region, the HCDR1 comprises an amino acid sequence of SEQ ID NO: 91, the HCDR2 comprises an amino acid sequence of SEQ ID NO: 92, and the HCDR3 comprises an amino acid sequence of SEQ ID NO: 93; and in the light chain variable region, the LCDR1 comprises an amino acid sequence of SEQ ID NO: 85, the LCDR2 comprises an amino acid sequence of SEQ ID NO: 62, and the LCDR3 comprises an amino acid sequence of SEQ ID NO: 94; or

k. in the heavy chain variable region, the HCDR1 comprises an amino acid sequence of SEQ ID NO: 99, the HCDR2 comprises an amino acid sequence of SEQ ID NO: 100, and the HCDR3 comprises an amino acid sequence of SEQ ID NO: 101; and in the light chain variable region, the LCDR1 comprises an amino acid sequence of SEQ ID NO: 102, the LCDR2 comprises an amino acid sequence of SEQ ID NO: 103, and the LCDR3 comprises an amino acid sequence of SEQ ID NO: 104; or

l. in the heavy chain variable region, the HCDR1 comprises an amino acid sequence of SEQ ID NO: 147, the HCDR2 comprises an amino acid sequence of SEQ ID NO: 148, and the HCDR3 comprises an amino acid sequence of SEQ ID NO: 149; and in the light chain variable region, the LCDR1 comprises an amino acid sequence of SEQ ID NO: 150, the LCDR2 comprises an amino acid sequence of SEQ ID NO: 151, and the LCDR3 comprises an amino acid sequence of SEQ ID NO: 152; or

m. in the heavy chain variable region, the HCDR1 comprises an amino acid sequence of SEQ ID NO: 153, the HCDR2 comprises an amino acid sequence of SEQ ID NO: 154, and the HCDR3 comprises an amino acid sequence of SEQ ID NO: 155; and in the light chain variable region, the LCDR1 comprises an amino acid sequence of SEQ ID NO: 114, the LCDR2 comprises an amino acid sequence of SEQ ID NO: 133, and the LCDR3 comprises an amino acid sequence of SEQ ID NO: 119; or

n. in the heavy chain variable region, the HCDR1 comprises an amino acid sequence of SEQ ID NO: 58, the HCDR2 comprises an amino acid sequence of SEQ ID NO: 156, and the HCDR3 comprises an amino acid sequence of SEQ ID NO: 157; and in the light chain variable region, the LCDR1 comprises an amino acid sequence of SEQ ID NO: 158, the LCDR2 comprises an amino acid sequence of SEQ ID NO: 62, and the LCDR3 comprises an amino acid sequence of SEQ ID NO: 159; or

o. in the heavy chain variable region, the HCDR1 comprises an amino acid sequence of SEQ ID NO: 105, the HCDR2 comprises an amino acid sequence of SEQ ID NO: 160, and the HCDR3 comprises an amino acid sequence of SEQ ID NO: 161; and in the light chain variable region, the LCDR1 comprises an amino acid sequence of SEQ ID NO: 114, the LCDR2 comprises an amino acid sequence of SEQ ID NO: 109, and the LCDR3 comprises an amino acid sequence of SEQ ID NO: 162; or

p. in the heavy chain variable region, the HCDR1 comprises an amino acid sequence of SEQ ID NO: 52, the HCDR2 comprises an amino acid sequence of SEQ ID NO: 53, and the HCDR3 comprises an amino acid sequence of SEQ ID NO: 54; and in the light chain variable region, the LCDR1 comprises an amino acid sequence of SEQ ID NO: 55, the LCDR2 comprises an amino acid sequence of SEQ ID NO: 56, and the LCDR3 comprises an amino acid sequence of SEQ ID NO: 57; or

q. in the heavy chain variable region, the HCDR1 comprises an amino acid sequence of SEQ ID NO: 58, the HCDR2 comprises an amino acid sequence of SEQ ID NO: 59, and the HCDR3 comprises an amino acid sequence of SEQ ID NO: 60; and in the light chain variable region, the LCDR1 comprises an amino acid sequence of SEQ ID NO: 61, the LCDR2 comprises an amino acid sequence of SEQ ID NO: 62, and the LCDR3 comprises an amino acid sequence of SEQ ID NO: 63; or

r. in the heavy chain variable region, the HCDR1 comprises an amino acid sequence of SEQ ID NO: 64, the HCDR2 comprises an amino acid sequence of SEQ ID NO: 65, and the HCDR3 comprises an amino acid sequence of SEQ ID NO: 66; and in the light chain variable region, the LCDR1 comprises an amino acid sequence of SEQ ID NO: 67, the LCDR2 comprises an amino acid sequence of SEQ ID NO: 56, and the LCDR3 comprises an amino acid sequence of SEQ ID NO: 68; or

s. in the heavy chain variable region, the HCDR1 comprises an amino acid sequence of SEQ ID NO: 73, the HCDR2 comprises an amino acid sequence of SEQ ID NO: 74, and the HCDR3 comprises an amino acid sequence of SEQ ID NO: 75; and in the light chain variable region, the LCDR1 comprises an amino acid sequence of SEQ ID NO: 76, the LCDR2 comprises an amino acid sequence of SEQ ID NO: 62, and the LCDR3 comprises an amino acid sequence of SEQ ID NO: 77; or

t. in the heavy chain variable region, the HCDR1 comprises an amino acid sequence of SEQ ID NO: 78, the HCDR2 comprises an amino acid sequence of SEQ ID NO: 79, and the HCDR3 comprises an amino acid sequence of SEQ ID NO: 80; and in the light chain variable region, the LCDR1 comprises an amino acid sequence of SEQ ID NO: 81, the LCDR2 comprises an amino acid sequence of SEQ ID NO: 62, and the LCDR3 comprises an amino acid sequence of SEQ ID NO: 82; or

u. in the heavy chain variable region, the HCDR1 comprises an amino acid sequence of SEQ ID NO: 52, the HCDR2 comprises an amino acid sequence of SEQ ID NO: 83, and the HCDR3 comprises an amino acid sequence of SEQ ID NO: 84; and in the light chain variable region, the LCDR1 comprises an amino acid sequence of SEQ ID NO: 85, the LCDR2 comprises an amino acid sequence of SEQ ID NO: 62, and the LCDR3 comprises an amino acid sequence of SEQ ID NO: 86; or

v. in the heavy chain variable region, the HCDR1 comprises an amino acid sequence of SEQ ID NO: 95, the HCDR2 comprises an amino acid sequence of SEQ ID NO: 96, and the HCDR3 comprises an amino acid sequence of SEQ ID NO: 97; and in the light chain variable region, the LCDR1 comprises an amino acid sequence of SEQ ID NO: 85, the LCDR2 comprises an amino acid sequence of SEQ ID NO: 98, and the LCDR3 comprises an amino acid sequence of SEQ ID NO: 68; or

w. in the heavy chain variable region, the HCDR1 comprises an amino acid sequence of SEQ ID NO: 105, the HCDR2 comprises an amino acid sequence of SEQ ID NO: 106, and the HCDR3 comprises an amino acid sequence of SEQ ID NO: 107; and in the light chain variable region, the LCDR1 comprises an amino acid sequence of SEQ ID NO: 108, the LCDR2 comprises an amino acid sequence of SEQ ID NO: 109, and the LCDR3 comprises an amino acid sequence of SEQ ID NO: 110; or

x. in the heavy chain variable region, the HCDR1 comprises an amino acid sequence of SEQ ID NO: 105, the HCDR2 comprises an amino acid sequence of SEQ ID NO: 117, and the HCDR3 comprises an amino acid sequence of SEQ ID NO: 118; and in the light chain variable region, the LCDR1 comprises an amino acid sequence of SEQ ID NO: 114, the LCDR2 comprises an amino acid sequence of SEQ ID NO: 109, and the LCDR3 comprises an amino acid sequence of SEQ ID NO: 119; or

y. in the heavy chain variable region, the HCDR1 comprises an amino acid sequence of SEQ ID NO: 129, the HCDR2 comprises an amino acid sequence of SEQ ID NO: 130, and the HCDR3 comprises an amino acid sequence of SEQ ID NO: 131; and in the light chain variable region, the LCDR1 comprises an amino acid sequence of SEQ ID NO: 132, the LCDR2 comprises an amino acid sequence of SEQ ID NO: 133, and the LCDR3 comprises an amino acid sequence of SEQ ID NO: 134.

In some embodiments, provided is the anti-ILT4 antibody according to any one of the above, wherein:

a. in the heavy chain variable region, the HCDR1 comprises an amino acid sequence of SEQ ID NO: 105, the HCDR2 comprises an amino acid sequence of SEQ ID NO: 120, and the HCDR3 comprises an amino acid sequence of SEQ ID NO: 121; and in the light chain variable region, the LCDR1 comprises an amino acid sequence of SEQ ID NO: 114, the LCDR2 comprises an amino acid sequence of SEQ ID NO: 109, and the LCDR3 comprises an amino acid sequence of SEQ ID NO: 119; or

b. in the heavy chain variable region, the HCDR1 comprises an amino acid sequence of SEQ ID NO: 135, the HCDR2 comprises an amino acid sequence of any one of SEQ ID NO: 203, 136, 201, 202, or 204, and the HCDR3 comprises an amino acid sequence of SEQ ID NO: 137; and in the light chain variable region, the LCDR1 comprises an amino acid sequence of SEQ ID NO: 138, the LCDR2 comprises an amino acid sequence of SEQ ID NO: 139, and the LCDR3 comprises an amino acid sequence of SEQ ID NO: 140; or

c. in the heavy chain variable region, the HCDR1 comprises an amino acid sequence of SEQ ID NO: 111, the HCDR2 comprises an amino acid sequence of any one of SEQ ID NO: 173, 112, 174, or 175, and the HCDR3 comprises an amino acid sequence of SEQ ID NO: 113; and in the light chain variable region, the LCDR1 comprises an amino acid sequence of SEQ ID NO: 114, the LCDR2 comprises an amino acid sequence of SEQ ID NO: 115, and the LCDR3 comprises an amino acid sequence of SEQ ID NO: 116; or

d. in the heavy chain variable region, the HCDR1 comprises an amino acid sequence of SEQ ID NO: 69, the HCDR2 comprises an amino acid sequence of any one of SEQ ID NO: 189, 70, or 188, and the HCDR3 comprises an amino acid sequence of SEQ ID NO: 71; and in the light chain variable region, the LCDR1 comprises an amino acid sequence of SEQ ID NO: 61, the LCDR2 comprises an amino acid sequence of SEQ ID NO: 62, and the LCDR3 comprises an amino acid sequence of SEQ ID NO: 72; or

e. in the heavy chain variable region, the HCDR1 comprises an amino acid sequence of SEQ ID NO: 165, the HCDR2 comprises an amino acid sequence of SEQ ID NO: 166, and the HCDR3 comprises an amino acid sequence of SEQ ID NO: 167; and in the light chain variable region, the LCDR1 comprises an amino acid sequence of SEQ ID NO: 224 or 168, the LCDR2 comprises an amino acid sequence of SEQ ID NO: 169, and the LCDR3 comprises an amino acid sequence of SEQ ID NO: 170; or

f. in the heavy chain variable region, the HCDR1 comprises an amino acid sequence of SEQ ID NO: 105, the HCDR2 comprises an amino acid sequence of SEQ ID NO: 125, and the HCDR3 comprises an amino acid sequence of SEQ ID NO: 126; and in the light chain variable region, the LCDR1 comprises an amino acid sequence of SEQ ID NO: 127, the LCDR2 comprises an amino acid sequence of SEQ ID NO: 109, and the LCDR3 comprises an amino acid sequence of SEQ ID NO: 128; or

g. in the heavy chain variable region, the HCDR1 comprises an amino acid sequence of SEQ ID NO: 141, the HCDR2 comprises an amino acid sequence of SEQ ID NO: 215 or 142, and the HCDR3 comprises an amino acid sequence of SEQ ID NO: 216 or 143; and in the light chain variable region, the LCDR1 comprises an amino acid sequence of SEQ ID NO: 144, the LCDR2 comprises an amino acid sequence of SEQ ID NO: 145, and the LCDR3 comprises an amino acid sequence of SEQ ID NO: 146; or

h. in the heavy chain variable region, the HCDR1 comprises an amino acid sequence of SEQ ID NO: 99, the HCDR2 comprises an amino acid sequence of SEQ ID NO: 122, and the HCDR3 comprises an amino acid sequence of SEQ ID NO: 123; and in the light chain variable region, the LCDR1 comprises an amino acid sequence of SEQ ID NO: 114, the LCDR2 comprises an amino acid sequence of SEQ ID NO: 109, and the LCDR3 comprises an amino acid sequence of SEQ ID NO: 124; or

i. in the heavy chain variable region, the HCDR1 comprises an amino acid sequence of SEQ ID NO: 73, the HCDR2 comprises an amino acid sequence of SEQ ID NO: 87, and the HCDR3 comprises an amino acid sequence of SEQ ID NO: 88; and in the light chain variable region, the LCDR1 comprises an amino acid sequence of SEQ ID NO: 61, the LCDR2 comprises an amino acid sequence of SEQ ID NO: 89, and the LCDR3 comprises an amino acid sequence of SEQ ID NO: 90; or

j. in the heavy chain variable region, the HCDR1 comprises an amino acid sequence of SEQ ID NO: 91, the HCDR2 comprises an amino acid sequence of SEQ ID NO: 92, and the HCDR3 comprises an amino acid sequence of SEQ ID NO: 93; and in the light chain variable region, the LCDR1 comprises an amino acid sequence of SEQ ID NO: 85, the LCDR2 comprises an amino acid sequence of SEQ ID NO: 62, and the LCDR3 comprises an amino acid sequence of SEQ ID NO: 94; or

k. in the heavy chain variable region, the HCDR1 comprises an amino acid sequence of SEQ ID NO: 99, the HCDR2 comprises an amino acid sequence of SEQ ID NO: 100, and the HCDR3 comprises an amino acid sequence of SEQ ID NO: 101; and in the light chain variable region, the LCDR1 comprises an amino acid sequence of SEQ ID NO: 102, the LCDR2 comprises an amino acid sequence of SEQ ID NO: 103, and the LCDR3 comprises an amino acid sequence of SEQ ID NO: 104; or

l. in the heavy chain variable region, the HCDR1 comprises an amino acid sequence of SEQ ID NO: 147, the HCDR2 comprises an amino acid sequence of SEQ ID NO: 148, and the HCDR3 comprises an amino acid sequence of SEQ ID NO: 149; and in the light chain variable region, the LCDR1 comprises an amino acid sequence of SEQ ID NO: 150, the LCDR2 comprises an amino acid sequence of SEQ ID NO: 151, and the LCDR3 comprises an amino acid sequence of SEQ ID NO: 152; or

m. in the heavy chain variable region, the HCDR1 comprises an amino acid sequence of SEQ ID NO: 153, the HCDR2 comprises an amino acid sequence of SEQ ID NO: 154, and the HCDR3 comprises an amino acid sequence of SEQ ID NO: 155; and in the light chain variable region, the LCDR1 comprises an amino acid sequence of SEQ ID NO: 114, the LCDR2 comprises an amino acid sequence of SEQ ID NO: 133, and the LCDR3 comprises an amino acid sequence of SEQ ID NO: 119; or

n. in the heavy chain variable region, the HCDR1 comprises an amino acid sequence of SEQ ID NO: 58, the HCDR2 comprises an amino acid sequence of SEQ ID NO: 156, and the HCDR3 comprises an amino acid sequence of SEQ ID NO: 157; and in the light chain variable region, the LCDR1 comprises an amino acid sequence of SEQ ID NO: 158, the LCDR2 comprises an amino acid sequence of SEQ ID NO: 62, and the LCDR3 comprises an amino acid sequence of SEQ ID NO: 159; or

o. in the heavy chain variable region, the HCDR1 comprises an amino acid sequence of SEQ ID NO: 105, the HCDR2 comprises an amino acid sequence of SEQ ID NO: 160, and the HCDR3 comprises an amino acid sequence of SEQ ID NO: 161; and in the light chain variable region, the LCDR1 comprises an amino acid sequence of SEQ ID NO: 114, the LCDR2 comprises an amino acid sequence of SEQ ID NO: 109, and the LCDR3 comprises an amino acid sequence of SEQ ID NO: 162.

In some embodiments, provided is the anti-ILT4 antibody according to any one of the above, wherein:

a. in the heavy chain variable region, the HCDR1 comprises an amino acid sequence of SEQ ID NO: 105, the HCDR2 comprises an amino acid sequence of SEQ ID NO: 120, and the HCDR3 comprises an amino acid sequence of SEQ ID NO: 121; and in the light chain variable region, the LCDR1 comprises an amino acid sequence of SEQ ID NO: 114, the LCDR2 comprises an amino acid sequence of SEQ ID NO: 109, and the LCDR3 comprises an amino acid sequence of SEQ ID NO: 119; or

b. in the heavy chain variable region, the HCDR1 comprises an amino acid sequence of SEQ ID NO: 135, the HCDR2 comprises an amino acid sequence of SEQ ID NO: 203, and the HCDR3 comprises an amino acid sequence of SEQ ID NO: 137; and in the light chain variable region, the LCDR1 comprises an amino acid sequence of SEQ ID NO: 138, the LCDR2 comprises an amino acid sequence of SEQ ID NO: 139, and the LCDR3 comprises an amino acid sequence of SEQ ID NO: 140; or

c. in the heavy chain variable region, the HCDR1 comprises an amino acid sequence of SEQ ID NO: 111, the HCDR2 comprises an amino acid sequence of SEQ ID NO: 173, and the HCDR3 comprises an amino acid sequence of SEQ ID NO: 113; and in the light chain variable region, the LCDR1 comprises an amino acid sequence of SEQ ID NO: 114, the LCDR2 comprises an amino acid sequence of SEQ ID NO: 115, and the LCDR3 comprises an amino acid sequence of SEQ ID NO: 116; or

d. in the heavy chain variable region, the HCDR1 comprises an amino acid sequence of SEQ ID NO: 69, the HCDR2 comprises an amino acid sequence of SEQ ID NO: 189, and the HCDR3 comprises an amino acid sequence of SEQ ID NO: 71; and in the light chain variable region, the LCDR1 comprises an amino acid sequence of SEQ ID NO: 61, the LCDR2 comprises an amino acid sequence of SEQ ID NO: 62, and the LCDR3 comprises an amino acid sequence of SEQ ID NO: 72; or

e. in the heavy chain variable region, the HCDR1 comprises an amino acid sequence of SEQ ID NO: 165, the HCDR2 comprises an amino acid sequence of SEQ ID NO: 166, and the HCDR3 comprises an amino acid sequence of SEQ ID NO: 167; and in the light chain variable region, the LCDR1 comprises an amino acid sequence of SEQ ID NO: 224, the LCDR2 comprises an amino acid sequence of SEQ ID NO: 169, and the LCDR3 comprises an amino acid sequence of SEQ ID NO: 170; or

f. in the heavy chain variable region, the HCDR1 comprises an amino acid sequence of SEQ ID NO: 105, the HCDR2 comprises an amino acid sequence of SEQ ID NO: 125, and the HCDR3 comprises an amino acid sequence of SEQ ID NO: 126; and in the light chain variable region, the LCDR1 comprises an amino acid sequence of SEQ ID NO: 127, the LCDR2 comprises an amino acid sequence of SEQ ID NO: 109, and the LCDR3 comprises an amino acid sequence of SEQ ID NO: 128; or

g. in the heavy chain variable region, the HCDR1 comprises an amino acid sequence of SEQ ID NO: 141, the HCDR2 comprises an amino acid sequence of SEQ ID NO: 215, and the HCDR3 comprises an amino acid sequence of SEQ ID NO: 216; and in the light chain variable region, the LCDR1 comprises an amino acid sequence of SEQ ID NO: 144, the LCDR2 comprises an amino acid sequence of SEQ ID NO: 145, and the LCDR3 comprises an amino acid sequence of SEQ ID NO: 146.

In some embodiments, provided is the anti-ILT4 antibody according to any one of the above, wherein:

a. in the heavy chain variable region, the HCDR1 comprises an amino acid sequence of SEQ ID NO: 105, the HCDR2 comprises an amino acid sequence of SEQ ID NO: 120, and the HCDR3 comprises an amino acid sequence of SEQ ID NO: 121; and in the light chain variable region, the LCDR1 comprises an amino acid sequence of SEQ ID NO: 114, the LCDR2 comprises an amino acid sequence of SEQ ID NO: 109, and the LCDR3 comprises an amino acid sequence of SEQ ID NO: 119; or

b. in the heavy chain variable region, the HCDR1 comprises an amino acid sequence of SEQ ID NO: 135, the HCDR2 comprises an amino acid sequence of SEQ ID NO: 203, and the HCDR3 comprises an amino acid sequence of SEQ ID NO: 137; and in the light chain variable region, the LCDR1 comprises an amino acid sequence of SEQ ID NO: 138, the LCDR2 comprises an amino acid sequence of SEQ ID NO: 139, and the LCDR3 comprises an amino acid sequence of SEQ ID NO: 140.

In some embodiments, provided is the anti-ILT4 antibody according to any one of the above, wherein: in the heavy chain variable region, the HCDR1 comprises an amino acid sequence set forth in SEQ ID NO: 105, the HCDR2 comprises an amino acid sequence set forth in SEQ ID NO: 120, and the HCDR3 comprises an amino acid sequence set forth in SEQ ID NO: 121; and in the light chain variable region, the LCDR1 comprises an amino acid sequence set forth in SEQ ID NO: 114, the LCDR2 comprises an amino acid sequence set forth in SEQ ID NO: 109, and the LCDR3 comprises an amino acid sequence set forth in SEQ ID NO: 119.

In some embodiments, provided is the anti-ILT4 antibody according to any one of the above, wherein:

p. in the heavy chain variable region, the HCDR1 comprises an amino acid sequence of SEQ ID NO: 52, the HCDR2 comprises an amino acid sequence of SEQ ID NO: 53, and the HCDR3 comprises an amino acid sequence of SEQ ID NO: 54; and in the light chain variable region, the LCDR1 comprises an amino acid sequence of SEQ ID NO: 55, the LCDR2 comprises an amino acid sequence of SEQ ID NO: 56, and the LCDR3 comprises an amino acid sequence of SEQ ID NO: 57; or

q. in the heavy chain variable region, the HCDR1 comprises an amino acid sequence of SEQ ID NO: 58, the HCDR2 comprises an amino acid sequence of SEQ ID NO: 59, and the HCDR3 comprises an amino acid sequence of SEQ ID NO: 60; and in the light chain variable region, the LCDR1 comprises an amino acid sequence of SEQ ID NO: 61, the LCDR2 comprises an amino acid sequence of SEQ ID NO: 62, and the LCDR3 comprises an amino acid sequence of SEQ ID NO: 63; or

r. in the heavy chain variable region, the HCDR1 comprises an amino acid sequence of SEQ ID NO: 64, the HCDR2 comprises an amino acid sequence of SEQ ID NO: 65, and the HCDR3 comprises an amino acid sequence of SEQ ID NO: 66; and in the light chain variable region, the LCDR1 comprises an amino acid sequence of SEQ ID NO: 67, the LCDR2 comprises an amino acid sequence of SEQ ID NO: 56, and the LCDR3 comprises an amino acid sequence of SEQ ID NO: 68; or

s. in the heavy chain variable region, the HCDR1 comprises an amino acid sequence of SEQ ID NO: 73, the HCDR2 comprises an amino acid sequence of SEQ ID NO: 74, and the HCDR3 comprises an amino acid sequence of SEQ ID NO: 75; and in the light chain variable region, the LCDR1 comprises an amino acid sequence of SEQ ID NO: 76, the LCDR2 comprises an amino acid sequence of SEQ ID NO: 62, and the LCDR3 comprises an amino acid sequence of SEQ ID NO: 77; or

t. in the heavy chain variable region, the HCDR1 comprises an amino acid sequence of SEQ ID NO: 78, the HCDR2 comprises an amino acid sequence of SEQ ID NO: 79, and the HCDR3 comprises an amino acid sequence of SEQ ID NO: 80; and in the light chain variable region, the LCDR1 comprises an amino acid sequence of SEQ ID NO: 81, the LCDR2 comprises an amino acid sequence of SEQ ID NO: 62, and the LCDR3 comprises an amino acid sequence of SEQ ID NO: 82; or

u. in the heavy chain variable region, the HCDR1 comprises an amino acid sequence of SEQ ID NO: 52, the HCDR2 comprises an amino acid sequence of SEQ ID NO: 83, and the HCDR3 comprises an amino acid sequence of SEQ ID NO: 84; and in the light chain variable region, the LCDR1 comprises an amino acid sequence of SEQ ID NO: 85, the LCDR2 comprises an amino acid sequence of SEQ ID NO: 62, and the LCDR3 comprises an amino acid sequence of SEQ ID NO: 86; or

v. in the heavy chain variable region, the HCDR1 comprises an amino acid sequence of SEQ ID NO: 95, the HCDR2 comprises an amino acid sequence of SEQ ID NO: 96, and the HCDR3 comprises an amino acid sequence of SEQ ID NO: 97; and in the light chain variable region, the LCDR1 comprises an amino acid sequence of SEQ ID NO: 85, the LCDR2 comprises an amino acid sequence of SEQ ID NO: 98, and the LCDR3 comprises an amino acid sequence of SEQ ID NO: 68; or

w. in the heavy chain variable region, the HCDR1 comprises an amino acid sequence of SEQ ID NO: 105, the HCDR2 comprises an amino acid sequence of SEQ ID NO: 106, and the HCDR3 comprises an amino acid sequence of SEQ ID NO: 107; and in the light chain variable region, the LCDR1 comprises an amino acid sequence of SEQ ID NO: 108, the LCDR2 comprises an amino acid sequence of SEQ ID NO: 109, and the LCDR3 comprises an amino acid sequence of SEQ ID NO: 110; or

x. in the heavy chain variable region, the HCDR1 comprises an amino acid sequence of SEQ ID NO: 105, the HCDR2 comprises an amino acid sequence of SEQ ID NO: 117, and the HCDR3 comprises an amino acid sequence of SEQ ID NO: 118; and in the light chain variable region, the LCDR1 comprises an amino acid sequence of SEQ ID NO: 114, the LCDR2 comprises an amino acid sequence of SEQ ID NO: 109, and the LCDR3 comprises an amino acid sequence of SEQ ID NO: 119; or

y. in the heavy chain variable region, the HCDR1 comprises an amino acid sequence of SEQ ID NO: 129, the HCDR2 comprises an amino acid sequence of SEQ ID NO: 130, and the HCDR3 comprises an amino acid sequence of SEQ ID NO: 131; and in the light chain variable region, the LCDR1 comprises an amino acid sequence of SEQ ID NO: 132, the LCDR2 comprises an amino acid sequence of SEQ ID NO: 133, and the LCDR3 comprises an amino acid sequence of SEQ ID NO: 134.

In some embodiments, provided is the anti-ILT4 antibody according to any one of the above, wherein the HCDR1, the HCDR2, and the HCDR3 of the heavy chain variable region and the LCDR1, the LCDR2, and the LCDR3 of the light chain variable region are defined according to the Kabat numbering scheme.

In some embodiments, provided is the anti-ILT4 antibody according to any one of the above, wherein:

a. in the heavy chain variable region, the HCDR1 comprises an amino acid sequence of SEQ ID NO: 260, the HCDR2 comprises an amino acid sequence of SEQ ID NO: 261, and the HCDR3 comprises an amino acid sequence of SEQ ID NO: 262; and in the light chain variable region, the LCDR1 comprises an amino acid sequence of SEQ ID NO: 263, the LCDR2 comprises an amino acid sequence of SEQ ID NO: 264, and the LCDR3 comprises an amino acid sequence of SEQ ID NO: 119; or

b. in the heavy chain variable region, the HCDR1 comprises an amino acid sequence of SEQ ID NO: 267, the HCDR2 comprises an amino acid sequence of SEQ ID NO: 268, and the HCDR3 comprises an amino acid sequence of SEQ ID NO: 269; and in the light chain variable region, the LCDR1 comprises an amino acid sequence of SEQ ID NO: 270, the LCDR2 comprises an amino acid sequence of SEQ ID NO: 271, and the LCDR3 comprises an amino acid sequence of SEQ ID NO: 140;

wherein the CDRs are defined according to the IMGT numbering scheme.

In some embodiments, provided is the anti-ILT4 antibody according to any one of the above, wherein:

in the heavy chain variable region, the HCDR1 comprises an amino acid sequence set forth in SEQ ID NO: 260, the HCDR2 comprises an amino acid sequence set forth in SEQ ID NO: 261, and the HCDR3 comprises an amino acid sequence set forth in SEQ ID NO: 262; and in the light chain variable region, the LCDR1 comprises an amino acid sequence set forth in SEQ ID NO: 263, the LCDR2 comprises an amino acid sequence set forth in SEQ ID NO: 264, and the LCDR3 comprises an amino acid sequence set forth in SEQ ID NO: 119;

wherein the CDRs are defined according to the IMGT numbering scheme.

In some embodiments, provided is the anti-ILT4 antibody according to any one of the above, wherein:

a. in the heavy chain variable region, the HCDR1 comprises an amino acid sequence of SEQ ID NO: 265, the HCDR2 comprises an amino acid sequence of SEQ ID NO: 266, and the HCDR3 comprises an amino acid sequence of SEQ ID NO: 121; and in the light chain variable region, the LCDR1 comprises an amino acid sequence of SEQ ID NO: 114, the LCDR2 comprises an amino acid sequence of SEQ ID NO: 109, and the LCDR3 comprises an amino acid sequence of SEQ ID NO: 119; or

b. in the heavy chain variable region, the HCDR1 comprises an amino acid sequence of SEQ ID NO: 272, the HCDR2 comprises an amino acid sequence of SEQ ID NO: 273, and the HCDR3 comprises an amino acid sequence of SEQ ID NO: 137; and in the light chain variable region, the LCDR1 comprises an amino acid sequence of SEQ ID NO: 138, the LCDR2 comprises an amino acid sequence of SEQ ID NO: 139, and the LCDR3 comprises an amino acid sequence of SEQ ID NO: 140;

wherein the CDRs are defined according to the Chothia numbering scheme.

In some embodiments, provided is the anti-ILT4 antibody according to any one of the above, wherein:

in the heavy chain variable region, the HCDR1 comprises an amino acid sequence set forth in SEQ ID NO: 265, the HCDR2 comprises an amino acid sequence set forth in SEQ ID NO: 266, and the HCDR3 comprises an amino acid sequence set forth in SEQ ID NO: 121; and in the light chain variable region, the LCDR1 comprises an amino acid sequence set forth in SEQ ID NO: 114, the LCDR2 comprises an amino acid sequence set forth in SEQ ID NO: 109, and the LCDR3 comprises an amino acid sequence set forth in SEQ ID NO: 119;

wherein the CDRs are defined according to the Chothia numbering scheme.

In some embodiments, provided is the anti-ILT4 antibody according to any one of the above, wherein the anti-ILT4 antibody is a murine antibody, a chimeric antibody, a humanized antibody, or a fully human-derived antibody. In some embodiments, the antibody is a chimeric antibody. In some embodiments, the antibody is a fully human-derived antibody. In some embodiments, the antibody is a humanized antibody.

In some embodiments, provided is the anti-ILT4 antibody according to any one of the above, wherein the anti-ILT4 antibody comprises a framework region (FR) of a human antibody.

In some embodiments, provided is the anti-ILT4 antibody according to any one of the above, wherein:

a. the heavy chain variable region comprises an amino acid sequence having at least 70% (e.g., at least 70%, 80%, 85%, 90%, 95%, 98%, or 99%) sequence identity to SEQ ID NO: 184 or 185, and the light chain variable region comprises an amino acid sequence having at least 70% (e.g., at least 70%, 80%, 85%, 90%, 95%, 98%, or 99%) sequence identity to SEQ ID NO: 187 or 186; or the heavy chain variable region comprises an amino acid sequence having at least 70% (e.g., at least 70%, 80%, 85%, 90%, 95%, 98%, or 99%) sequence identity to SEQ ID NO: 32, and the light chain variable region comprises an amino acid sequence having at least 70% (e.g., at least 70%, 80%, 85%, 90%, 95%, 98%, or 99%) sequence identity to SEQ ID NO: 33; or

b. the heavy chain variable region comprises an amino acid sequence having at least 70% (e.g., at least 70%, 80%, 85%, 90%, 95%, 98%, or 99%) sequence identity to SEQ ID NO: 211, 205, 206, 207, 208, 209, or 210, and the light chain variable region comprises an amino acid sequence having at least 70% (e.g., at least 70%, 80%, 85%, 90%, 95%, 98%, or 99%) sequence identity to SEQ ID NO: 212, 213, or 214; or the heavy chain variable region comprises an amino acid sequence having at least 70% (e.g., at least 70%, 80%, 85%, 90%, 95%, 98%, or 99%) sequence identity to SEQ ID NO: 40, and the light chain variable region comprises an amino acid sequence having at least 70% (e.g., at least 70%, 80%, 85%, 90%, 95%, 98%, or 99%) sequence identity to SEQ ID NO: 41; or

c. the heavy chain variable region comprises an amino acid sequence having at least 70% (e.g., at least 70%, 80%, 85%, 90%, 95%, 98%, or 99%) sequence identity to SEQ ID NO: 181, 176, 177, 178, 179, or 180, and the light chain variable region comprises an amino acid sequence having at least 70% (e.g., at least 70%, 80%, 85%, 90%, 95%, 98%, or 99%) sequence identity to SEQ ID NO: 183 or 182; or the heavy chain variable region comprises an amino acid sequence having at least 70% (e.g., at least 70%, 80%, 85%, 90%, 95%, 98%, or 99%) sequence identity to SEQ ID NO: 28, and the light chain variable region comprises an amino acid sequence having at least 70% (e.g., at least 70%, 80%, 85%, 90%, 95%, 98%, or 99%) sequence identity to SEQ ID NO: 29; or

d. the heavy chain variable region comprises an amino acid sequence having at least 70% (e.g., at least 70%, 80%, 85%, 90%, 95%, 98%, or 99%) sequence identity to SEQ ID NO: 193, 190, 191, or 192, and the light chain variable region comprises an amino acid sequence having at least 70% (e.g., at least 70%, 80%, 85%, 90%, 95%, 98%, or 99%) sequence identity to SEQ ID NO: 194, 195, or 196; or the heavy chain variable region comprises an amino acid sequence having at least 70% (e.g., at least 70%, 80%, 85%, 90%, 95%, 98%, or 99%) sequence identity to SEQ ID NO: 10, and the light chain variable region comprises an amino acid sequence having at least 70% (e.g., at least 70%, 80%, 85%, 90%, 95%, 98%, or 99%) sequence identity to SEQ ID NO: 11; or

e. the heavy chain variable region comprises an amino acid sequence having at least 70% (e.g., at least 70%, 80%, 85%, 90%, 95%, 98%, or 99%) sequence identity to SEQ ID NO: 163, and the light chain variable region comprises an amino acid sequence having at least 70% (e.g., at least 70%, 80%, 85%, 90%, 95%, 98%, or 99%) sequence identity to SEQ ID NO: 225 or 164;

f. the heavy chain variable region comprises an amino acid sequence having at least 70% (e.g., at least 70%, 80%, 85%, 90%, 95%, 98%, or 99%) sequence identity to SEQ ID NO: 197 or 198, and the light chain variable region comprises an amino acid sequence having at least 70% (e.g., at least 70%, 80%, 85%, 90%, 95%, 98%, or 99%) sequence identity to SEQ ID NO: 199 or 200; or the heavy chain variable region comprises an amino acid sequence having at least 70% (e.g., at least 70%, 80%, 85%, 90%, 95%, 98%, or 99%) sequence identity to SEQ ID NO: 36, and the light chain variable region comprises an amino acid sequence having at least 70% (e.g., at least 70%, 80%, 85%, 90%, 95%, 98%, or 99%) sequence identity to SEQ ID NO: 37; or

g. the heavy chain variable region comprises an amino acid sequence having at least 70% (e.g., at least 70%, 80%, 85%, 90%, 95%, 98%, or 99%) sequence identity to SEQ ID NO: 217, 218, 219, 220, or 221, and the light chain variable region comprises an amino acid sequence having at least 70% (e.g., at least 70%, 80%, 85%, 90%, 95%, 98%, or 99%) sequence identity to SEQ ID NO: 222 or 223; or the heavy chain variable region comprises an amino acid sequence having at least 70% (e.g., at least 70%, 80%, 85%, 90%, 95%, 98%, or 99%) sequence identity to SEQ ID NO: 42, and the light chain variable region comprises an amino acid sequence having at least 70% (e.g., at least 70%, 80%, 85%, 90%, 95%, 98%, or 99%) sequence identity to SEQ ID NO: 43.

In some embodiments, provided is the anti-ILT4 antibody according to any one of the above, wherein:

a. the heavy chain variable region comprises an amino acid sequence of SEQ ID NO: 184 or 185, and the light chain variable region comprises an amino acid sequence of SEQ ID NO: 187 or 186; or
the heavy chain variable region comprises an amino acid sequence of SEQ ID NO: 32, and the light chain variable region comprises an amino acid sequence of SEQ ID NO: 33; or
b. the heavy chain variable region comprises an amino acid sequence of SEQ ID NO: 211, 205, 206, 207, 208, 209, or 210, and the light chain variable region comprises an amino acid sequence of SEQ ID NO: 212, 213, or 214; or
the heavy chain variable region comprises an amino acid sequence of SEQ ID NO: 40, and the light chain variable region comprises an amino acid sequence of SEQ ID NO: 41; or
c. the heavy chain variable region comprises an amino acid sequence of SEQ ID NO: 181, 176, 177, 178, 179, or 180, and the light chain variable region comprises an amino acid sequence of SEQ ID NO: 183 or 182; or
the heavy chain variable region comprises an amino acid sequence of SEQ ID NO: 28, and the light chain variable region comprises an amino acid sequence of SEQ ID NO: 29; or
d. the heavy chain variable region comprises an amino acid sequence of SEQ ID NO: 193, 190, 191, or 192, and the light chain variable region comprises an amino acid sequence of SEQ ID NO: 194, 195, or 196; or
the heavy chain variable region comprises an amino acid sequence of SEQ ID NO: 10, and the light chain variable region comprises an amino acid sequence of SEQ ID NO: 11; or
e. the heavy chain variable region comprises an amino acid sequence of SEQ ID NO: 163, and the light chain variable region comprises an amino acid sequence of SEQ ID NO: 225 or 164;
f. the heavy chain variable region comprises an amino acid sequence of SEQ ID NO: 197 or 198, and the light chain variable region comprises an amino acid sequence of SEQ ID NO: 199 or 200; or
the heavy chain variable region comprises an amino acid sequence of SEQ ID NO: 36, and the light chain variable region comprises an amino acid sequence of SEQ ID NO: 37; or
g. the heavy chain variable region comprises an amino acid sequence of SEQ ID NO: 217, 218, 219, 220, or 221, and the light chain variable region comprises an amino acid sequence of SEQ ID NO: 222 or 223; or
the heavy chain variable region comprises an amino acid sequence of SEQ ID NO: 42, and the light chain variable region comprises an amino acid sequence of SEQ ID NO: 43.

In some embodiments, provided is the anti-ILT4 antibody according to any one of the above, wherein:

a. the heavy chain variable region comprises an amino acid sequence of SEQ ID NO: 184 or 185, and the light chain variable region comprises an amino acid sequence of SEQ ID NO: 187 or 186; or
b. the heavy chain variable region comprises an amino acid sequence of SEQ ID NO: 211, 205, 206, 207, 208, 209, or 210, and the light chain variable region comprises an amino acid sequence of SEQ ID NO: 212, 213, or 214; or
c. the heavy chain variable region comprises an amino acid sequence of SEQ ID NO: 181, 176, 177, 178, 179, or 180, and the light chain variable region comprises an amino acid sequence of SEQ ID NO: 183 or 182; or
d. the heavy chain variable region comprises an amino acid sequence of SEQ ID NO: 193, 190, 191, or 192, and the light chain variable region comprises an amino acid sequence of SEQ ID NO: 194, 195, or 196; or
e. the heavy chain variable region comprises an amino acid sequence of SEQ ID NO: 163, and the light chain variable region comprises an amino acid sequence of SEQ ID NO: 225 or 164;
f. the heavy chain variable region comprises an amino acid sequence of SEQ ID NO: 197 or 198, and the light chain variable region comprises an amino acid sequence of SEQ ID NO: 199 or 200; or
g. the heavy chain variable region comprises an amino acid sequence of SEQ ID NO: 217, 218, 219, 220, or 221, and the light chain variable region comprises an amino acid sequence of SEQ ID NO: 222 or 223.

In some embodiments, provided is the anti-ILT4 antibody according to any one of the above, wherein:

> a. the heavy chain variable region comprises an amino acid sequence of SEQ ID NO: 184, and the light chain variable region comprises an amino acid sequence of SEQ ID NO: 187; or
> b. the heavy chain variable region comprises an amino acid sequence of SEQ ID NO: 211, and the light chain variable region comprises an amino acid sequence of SEQ ID NO: 212; or
> c. the heavy chain variable region comprises an amino acid sequence of SEQ ID NO: 181, and the light chain variable region comprises an amino acid sequence of SEQ ID NO: 183; or
> d. the heavy chain variable region comprises an amino acid sequence of SEQ ID NO: 193, and the light chain variable region comprises an amino acid sequence of SEQ ID NO: 194; or
> e. the heavy chain variable region comprises an amino acid sequence of SEQ ID NO: 163, and the light chain variable region comprises an amino acid sequence of SEQ ID NO: 225;
> f. the heavy chain variable region comprises an amino acid sequence of SEQ ID NO: 197, and the light chain variable region comprises an amino acid sequence of SEQ ID NO: 199; or
> g. the heavy chain variable region comprises an amino acid sequence of SEQ ID NO: 217, and the light chain variable region comprises an amino acid sequence of SEQ ID NO: 222.

In some embodiments, provided is the anti-ILT4 antibody according to any one of the above, wherein:

> a. the heavy chain variable region comprises an amino acid sequence of SEQ ID NO: 184, and the light chain variable region comprises an amino acid sequence of SEQ ID NO: 187; or
> b. the heavy chain variable region comprises an amino acid sequence of SEQ ID NO: 211, and the light chain variable region comprises an amino acid sequence of SEQ ID NO: 212.

In some embodiments, provided is the anti-ILT4 antibody according to any one of the above, wherein: the heavy chain variable region comprises an amino acid sequence set forth in SEQ ID NO: 184, and the light chain variable region comprises an amino acid sequence set forth in SEQ ID NO: 187.

In some embodiments, provided is the anti-ILT4 antibody according to any one of the above, wherein:

> a. the heavy chain variable region comprises an amino acid sequence of SEQ ID NO: 32, and the light chain variable region comprises an amino acid sequence of SEQ ID NO: 33; or
> b. the heavy chain variable region comprises an amino acid sequence of SEQ ID NO: 40, and the light chain variable region comprises an amino acid sequence of SEQ ID NO: 41; or
> c. the heavy chain variable region comprises an amino acid sequence of SEQ ID NO: 28, and the light chain variable region comprises an amino acid sequence of SEQ ID NO: 29; or
> d. the heavy chain variable region comprises an amino acid sequence of SEQ ID NO: 10, and the light chain variable region comprises an amino acid sequence of SEQ ID NO: 11; or
> f. the heavy chain variable region comprises an amino acid sequence of SEQ ID NO: 36, and the light chain variable region comprises an amino acid sequence of SEQ ID NO: 37; or
> g. the heavy chain variable region comprises an amino acid sequence of SEQ ID NO: 42, and the light chain variable region comprises an amino acid sequence of SEQ ID NO: 43.

In some embodiments, provided is the anti-ILT4 antibody according to any one of the above, wherein the heavy chain variable region comprises a FR1, a FR2, and a FR3 that are derived from IGHV4-31*01 and a FR4 derived from IGHJ1*01, and the FRs are unsubstituted or have one or more amino acid substitutions selected from the group consisting of 1E, 27S, 30T, 44K, 71R, and 76S; and/or the light chain variable region comprises a FR1, a FR2, and a FR3 that are derived from IGKV1-39*01 and a FR4 derived from IGKJ4*01, and the FRs are unsubstituted or have one or more amino acid substitutions selected from the group consisting of 43S, 48V, and 71Y In some embodiments, provided is the anti-ILT4 antibody, wherein in the heavy chain variable region, the HCDR1 comprises an amino acid sequence of SEQ ID NO: 105, the HCDR2 comprises an amino acid sequence of SEQ ID NO: 120, and the HCDR3 comprises an amino acid sequence of SEQ ID NO: 121, and the FRs of the heavy chain variable region comprise one or more amino acid substitutions selected from the group consisting of 1E, 27S, 30T, 44K, 71R, and 76S; and in the light chain variable region, the LCDR1 comprises an amino acid sequence of SEQ ID NO: 114, the LCDR2 comprises an amino acid sequence of SEQ ID NO: 109, and the LCDR3 comprises an amino acid sequence of SEQ ID NO: 119, and the FRs of the light chain variable region comprise one or more amino acid substitutions selected from the group consisting of 43S, 48V, and 71Y In some embodiments, the variable regions and CDRs described above are defined according to the Kabat numbering scheme.

In some embodiments, provided is the anti-ILT4 antibody according to any one of the above, wherein the heavy chain variable region comprises a FR1, a FR2, and a FR3 that are derived from IGHV1-18*01 and a FR4 derived from IGHJ6*01, and the FRs are unsubstituted or have one or more amino acid substitutions selected from the group consisting of 1E, 43E, 69L, 71V, 73K, 76N, and 93T; and/or the light chain variable region comprises a FR1, a FR2, and a FR3 that are derived from IGKV4-1*01 or IGKV3-11 *01 and a FR4 derived from IGKJ4*01, and the FRs are unsubstituted or have one or more amino acid substitutions selected from the group consisting of 4L, 43P, 45K, 58I, 68R, and 85T. In some embodiments, provided is the anti-ILT4 antibody, wherein in the heavy chain variable region, the HCDR1 comprises an amino acid sequence of SEQ ID NO: 135, the HCDR2 comprises an amino acid sequence of SEQ ID NO: 203, 136, 201, 202, or 204, and the HCDR3 comprises an amino acid sequence of SEQ ID NO: 137, and the FRs of the heavy chain variable region comprise one or more amino acid substitutions selected from the group consisting of 1E, 43E, 69L, 71V, 73K, 76N, and 93T; and in the light chain variable region, the LCDR1 comprises an amino acid sequence of SEQ ID NO: 138, the LCDR2 comprises an amino acid sequence of SEQ ID NO: 139, and the LCDR3 comprises an amino acid sequence of SEQ ID NO: 140, and the FRs of the light chain variable region comprise one or more amino acid substitutions selected from the group consisting of 4L, 43P, 45K, 58I, 68R, and 85T. In some embodiments, the variable regions and CDRs described above are defined according to the Kabat numbering scheme.

In some embodiments, provided is the anti-ILT4 antibody according to any one of the above, wherein the heavy chain variable region comprises a FR1, a FR2, and a FR3 that are derived from IGHV1-3*01 and a FR4 derived from IGHJ6*01, and the FRs are unsubstituted or have one or more amino acid substitutions selected from the group consisting of 1E, 38K, 44S, 69L, 71V, 73K, and 75G; and/or the light chain variable region comprises a FR1, a FR2, and a FR3 that are derived from IGKV1-39*01 and a FR4 derived from IGKJ2*01, and the FRs are unsubstituted or have one or more amino acid substitutions selected from the group consisting of 43S, 48V, and 71Y. In some embodiments, provided is the anti-ILT4 antibody, wherein in the heavy chain variable region, the HCDR1 comprises an amino acid sequence of SEQ ID NO: 111, the HCDR2 comprises an amino acid sequence of SEQ ID NO: 173, 112, 174, or 175, and the HCDR3 comprises an amino acid sequence of SEQ ID NO: 113, and the FRs of the heavy chain variable region comprise one or more amino acid substitutions selected from the group consisting of 1E, 38K, 44S, 69L, 71V, 73K, and 75G; and in the light chain variable region, the LCDR1 comprises an amino acid sequence of SEQ ID NO: 114, the LCDR2 comprises an amino acid sequence of SEQ ID NO: 115, and the LCDR3 comprises an amino acid sequence of SEQ ID NO: 116, and the FRs of the light chain variable region comprise one or more amino acid substitutions selected from the group consisting of 43S, 48V, and 71Y. In some embodiments, the variable regions and CDRs described above are defined according to the Kabat numbering scheme.

In some embodiments, provided is the anti-ILT4 antibody according to any one of the above, wherein the heavy chain variable region comprises a FR1, a FR2, and a FR3 that are derived from IGHV3-21*01 and a FR4 derived from IGHJ1*01, and the FRs are unsubstituted or have a 49A substitution; and/or the light chain variable region comprises a FR1, a FR2, and a FR3 that are derived from IGKV1-33*01 and a FR4 derived from IGKJ4*01, and the FRs are unsubstituted or have one or more amino acid substitutions selected from the group consisting of 39R, 42Q, 43S, and 85V. In some embodiments, provided is the anti-ILT4 antibody, wherein in the heavy chain variable region, the HCDR1 comprises an amino acid sequence of SEQ ID NO: 69, the HCDR2 comprises an amino acid sequence of SEQ ID NO: 189, 70, or 188, and the HCDR3 comprises an amino acid sequence of SEQ ID NO: 71, and the FRs of the heavy chain variable region comprise a 49A substitution; and in the light chain variable region, the LCDR1 comprises an amino acid sequence of SEQ ID NO: 61, the LCDR2 comprises an amino acid sequence of SEQ ID NO: 62, and the LCDR3 comprises an amino acid sequence of SEQ ID NO: 72, and the FRs of the light chain variable region comprise one or more amino acid substitutions selected from the group consisting of 39R, 42Q, 43S, and 85V. In some embodiments, the variable regions and CDRs described above are defined according to the Kabat numbering scheme.

In some embodiments, provided is the anti-ILT4 antibody according to any one of the above, wherein the heavy chain variable region comprises a FR1, a FR2, and a FR3 that are derived from IGHV4-31*02 and a FR4 derived from IGHJ6*01, and the FRs are unsubstituted or have one or more amino acid substitutions selected from the group consisting of 1E, 27Y, 30T, 42E, 44K, and 71R; and/or the light chain variable region comprises a FR1, a FR2, and a FR3 that are derived from IGKV1-39*01 and a FR4 derived from IGKJ4*01, and the FRs are unsubstituted or have one or more amino acid substitutions selected from the group consisting of 43S, 48L, and 71Y. In some embodiments, provided is the anti-ILT4 antibody, wherein in the heavy chain variable region, the HCDR1 comprises an amino acid sequence of SEQ ID NO: 105, the HCDR2 comprises an amino acid sequence of SEQ ID NO: 125, and the HCDR3 comprises an amino acid sequence of SEQ ID NO: 126, and the FRs of the heavy chain variable region comprise one or more amino acid substitutions selected from the group consisting of 1E, 27Y, 30T, 42E, 44K, and 71R; and in the light chain variable region, the LCDR1 comprises an amino acid sequence of SEQ ID NO: 127, the LCDR2 comprises an amino acid sequence of SEQ ID NO: 109, and the LCDR3 comprises an amino acid sequence of SEQ ID NO: 128, and the FRs of the light chain variable region comprise one or more amino acid substitutions selected from the group consisting of 43S, 48L, and 71Y. In some embodiments, the variable regions and CDRs described above are defined according to the Kabat numbering scheme.

In some embodiments, provided is the anti-ILT4 antibody according to any one of the above, wherein the heavy chain variable region comprises a FR1, a FR2, and a FR3 that are derived from IGHV1-18*01 and a FR4 derived from IGHJ6*01, and the FRs are unsubstituted or have one or more amino acid substitutions selected from the group consisting of 1E, 2I, 44A, 46K, and 76T; and/or the light chain variable region comprises a FR1, a FR2, and a FR3 that are derived from IGKV4-1*01 or IGKV2-28*01 and a FR4 derived from IGKJ4*01, and the FRs are unsubstituted or have one or more amino acid substitutions selected from the group consisting of 43 S and 48L. In some embodiments, provided is the anti-ILT4 antibody, wherein in the heavy chain variable region, the HCDR1 comprises an amino acid sequence of SEQ ID NO: 141, the HCDR2 comprises an amino acid sequence of SEQ ID NO: 215 or 142, and the HCDR3 comprises an amino acid sequence of SEQ ID NO: 216 or 143, and the FRs of the heavy chain variable region comprise one or more amino acid substitutions selected from the group consisting of 1E, 2I, 44A, 46K, and 76T; and in the light chain variable region, the LCDR1 comprises an amino acid sequence of SEQ ID NO: 144, the LCDR2 comprises an amino acid sequence of SEQ ID NO: 145, and the LCDR3 comprises an amino acid sequence of SEQ ID NO: 146, and the FRs of the light chain variable region comprise one or more amino acid substitutions selected from the group consisting of 43 S and 48L. In some embodiments, the variable regions and CDRs described above are defined according to the Kabat numbering scheme.

In some embodiments, provided is the anti-ILT4 antibody according to any one of the above, wherein the anti-ILT4 antibody is an antibody fragment; in some embodiments, the antibody fragment is selected from the group consisting of Fab, Fab', F(ab')2, Fd, Fv, scFv, dsFv, and dAb.

In some embodiments, provided is the anti-ILT4 antibody according to any one of the above, wherein the anti-ILT4 antibody comprises a heavy chain constant region and a light chain constant region. In some embodiments, the heavy chain constant region is a human IgG1 or IgG4 heavy chain constant region. In some embodiments, the heavy chain constant region comprises 234A and 235A mutations; the mutations are numbered according to the EU index. In some embodiments, the light chain constant region is a human κ light chain constant region. In some embodiments, the heavy chain constant region comprises an amino acid sequence of SEQ ID NO: 240 or 171, and the light chain constant region comprises an amino acid sequence of SEQ ID NO: 172. In some embodiments, the heavy chain constant region comprises an amino acid sequence of SEQ ID NO: 240, and the light chain constant region comprises an amino acid sequence of SEQ ID NO: 172.

In some embodiments, provided is the anti-ILT4 antibody according to any one of the above, wherein the anti-ILT4 antibody comprises a heavy chain and a light chain, wherein:

    a. the heavy chain comprises an amino acid sequence having at least 70% (e.g., at least 70%, 80%, 85%, 90%, 95%, 98%, or 99%) sequence identity to SEQ ID NO: 228, and the light chain comprises an amino acid sequence having at least 70% (e.g., at least 70%, 80%, 85%, 90%, 95%, 98%, or 99%) sequence identity to SEQ ID NO: 229; or

    b. the heavy chain comprises an amino acid sequence having at least 70% (e.g., at least 70%, 80%, 85%, 90%, 95%, 98%, or 99%) sequence identity to SEQ ID NO: 234, and the light chain comprises an amino acid sequence having at least 70% (e.g., at least 70%, 80%, 85%, 90%, 95%, 98%, or 99%) sequence identity to SEQ ID NO: 235; or

    c. the heavy chain comprises an amino acid sequence having at least 70% (e.g., at least 70%, 80%, 85%, 90%, 95%, 98%, or 99%) sequence identity to SEQ ID NO: 226, and the light chain comprises an amino acid sequence having at least 70% (e.g., at least 70%, 80%, 85%, 90%, 95%, 98%, or 99%) sequence identity to SEQ ID NO: 227; or

    d. the heavy chain comprises an amino acid sequence having at least 70% (e.g., at least 70%, 80%, 85%, 90%, 95%, 98%, or 99%) sequence identity to SEQ ID NO: 230, and the light chain comprises an amino acid sequence having at least 70% (e.g., at least 70%, 80%, 85%, 90%, 95%, 98%, or 99%) sequence identity to SEQ ID NO: 231; or

    e. the heavy chain comprises an amino acid sequence having at least 70% (e.g., at least 70%, 80%, 85%, 90%, 95%, 98%, or 99%) sequence identity to SEQ ID NO: 238, and the light chain comprises an amino acid sequence having at least 70% (e.g., at least 70%, 80%, 85%, 90%, 95%, 98%, or 99%) sequence identity to SEQ ID NO: 239; or

    f. the heavy chain comprises an amino acid sequence having at least 70% (e.g., at least 70%, 80%, 85%, 90%, 95%, 98%, or 99%) sequence identity to SEQ ID NO: 232, and the light chain comprises an amino acid sequence having at least 70% (e.g., at least 70%, 80%, 85%, 90%, 95%, 98%, or 99%) sequence identity to SEQ ID NO: 233; or

    g. the heavy chain comprises an amino acid sequence having at least 70% (e.g., at least 70%, 80%, 85%, 90%, 95%, 98%, or 99%) sequence identity to SEQ ID NO: 236, and the light chain comprises an amino acid sequence having at least 70% (e.g., at least 70%, 80%, 85%, 90%, 95%, 98%, or 99%) sequence identity to SEQ ID NO: 237.

In some embodiments, provided is the anti-ILT4 antibody according to any one of the above, wherein the anti-ILT4 antibody comprises a heavy chain and a light chain, wherein:

    a. the heavy chain comprises an amino acid sequence of SEQ ID NO: 228, and the light chain comprises an amino acid sequence of SEQ ID NO: 229;

    b. the heavy chain comprises an amino acid sequence of SEQ ID NO: 234, and the light chain comprises an amino acid sequence of SEQ ID NO: 235;

    c. the heavy chain comprises an amino acid sequence of SEQ ID NO: 226, and the light chain comprises an amino acid sequence of SEQ ID NO: 227;

    d. the heavy chain comprises an amino acid sequence of SEQ ID NO: 230, and the light chain comprises an amino acid sequence of SEQ ID NO: 231;

e. the heavy chain comprises an amino acid sequence of SEQ ID NO: 238, and the light chain comprises an amino acid sequence of SEQ ID NO: 239;

f. the heavy chain comprises an amino acid sequence of SEQ ID NO: 232, and the light chain comprises an amino acid sequence of SEQ ID NO: 233;

g. the heavy chain comprises an amino acid sequence of SEQ ID NO: 236, and the light chain comprises an amino acid sequence of SEQ ID NO: 237.

In some embodiments, provided is the anti-ILT4 antibody according to any one of the above, wherein the anti-ILT4 antibody comprises a heavy chain and a light chain, wherein:

a. the heavy chain comprises an amino acid sequence of SEQ ID NO: 228, and the light chain comprises an amino acid sequence of SEQ ID NO: 229;

b. the heavy chain comprises an amino acid sequence of SEQ ID NO: 234, and the light chain comprises an amino acid sequence of SEQ ID NO: 235.

In some embodiments, provided is the anti-ILT4 antibody according to any one of the above, wherein the anti-ILT4 antibody comprises a heavy chain and a light chain, wherein:

the heavy chain comprises an amino acid sequence set forth in SEQ ID NO: 228, and
the light chain comprises an amino acid sequence set forth in SEQ ID NO: 229.

In some embodiments, the present disclosure further provides an isolated anti-ILT4 antibody that competes for binding to human ILT4 or an epitope thereof with the anti-ILT4 antibody according to any one of the foregoing.

In some embodiments, the anti-ILT4 antibody provided by the present disclosure specifically binds to human ILT4 and does not bind to other LILR family members: ILT2, ILT3, LILRB5, LILRA1, LILRA2, LILRA3, LILRA4, LILRA5, and/or LILRA6. In some embodiments, the ability of the isolated anti-ILT4 antibody of the present disclosure to bind to ILT4 protein is higher than that of a control antibody. Specifically, the isolated anti-ILT4 antibody of the present disclosure binds to human ILT4 with a KD value of less than $1 \times 10^{-8}$ M (e.g., less than $8 \times 10^{-9}$ M, less than $5 \times 10^{-9}$ M, less than $2 \times 10^{-9}$ M, less than $1 \times 10^{-9}$ M, less than $8 \times 10^{-10}$ M, less than $5 \times 10^{-10}$ M, less than $2 \times 10^{-10}$ M, less than $1 \times 10^{-10}$ M, less than $8 \times 10^{-11}$ M, or less than $5 \times 10^{-11}$ M), as measured by Biacore.

In some embodiments, a humanized anti-ILT4 antibody provided by the present disclosure is capable of effectively removing the inhibitory effect of ILT4 and promoting the secretion of higher levels of mIL-2 by 3A9 cells.

In some embodiments, a humanized anti-ILT4 antibody provided by the present disclosure is capable of effectively promoting the transformation of macrophages to the M1 phenotype and the secretion of high levels of the proinflammatory cytokine TNF-$\alpha$.

In some embodiments, a humanized anti-ILT4 antibody provided by the present disclosure (e.g., huP2-G9) is capable of inducing dendritic cells to secrete higher levels of proinflammatory cytokines or chemokines in an immunosuppressive environment with IL-10.

In some embodiments, a humanized anti-ILT4 antibody provided by the present disclosure is capable of effectively blocking the binding of ILT4 to HLA-G.

In some embodiments, a humanized anti-ILT4 antibody provided by the present disclosure is capable of effectively blocking the binding of ILT4 to HLA-G, and the blocking activity of the humanized anti-ILT4 antibody is stronger than that of the control antibodies MK and JTX.

In another aspect, the present disclosure provides a pharmaceutical composition comprising the anti-ILT4 antibody according to any one of the above and one or more pharmaceutically acceptable carriers, diluents, or excipients. In some embodiments, the pharmaceutical composition further comprises at least one second therapeutic agent. In some embodiments, the second therapeutic agent is an anti-PD-1 antibody.

In another aspect, the present disclosure provides an isolated nucleic acid encoding the anti-ILT4 antibody according to any one of the above.

In another aspect, the present disclosure provides a vector comprising the aforementioned nucleic acid molecule.

In another aspect, the present disclosure provides a host cell comprising the isolated nucleic acid according to any one of the above.

In another aspect, the present disclosure provides a method for blocking the binding of ILT4 to HLA-G in a human subject in need thereof, the method comprising administering to the human subject an effective amount of the anti-ILT4 antibody according to any one of the above.

In another aspect, the present disclosure provides a method for detecting an ILT4 peptide or a fragment thereof in a sample, the method comprising contacting the sample with the anti-ILT4 antibody according to any one of the above and detecting the presence of a complex between the anti-ILT4 antibody and the ILT4 peptide or the fragment thereof, wherein that the complex is detected indicates the presence of the ILT4 peptide or the fragment thereof.

In another aspect, the present disclosure provides a method for treating a disease, the method comprising a step of administering to a subject the anti-ILT4 antibody or the pharmaceutical composition according to any one of the above.

In another aspect, the present disclosure provides use of the anti-ILT4 antibody or the pharmaceutical composition according to any one of the above in the preparation of a medicament for treating a disease.

In another aspect, the present disclosure provides the anti-ILT4 antibody or the pharmaceutical composition according to any one of the above for use as a medicament. In some embodiments, the medicament is used for treating a disease.

In some embodiments, the disease according to any one of the above is a tumor. In some embodiments, the disease is selected from the group consisting of astrocytoma (e.g., anaplastic astrocytoma), glioblastoma, bladder cancer, bone cancer, brain cancer, breast cancer (e.g., breast cancer characterized by BRCA1 and/or BRCA2 mutations), carcinoid, cervical cancer, choroid plexus papilloma, colorectal cancer (e.g., colon cancer and rectal cancer), fallopian tube cancer, ependymoma, gallbladder cancer, gastric cancer, head and neck cancer, idiopathic myelofibrosis, renal cancer or renal cell carcinoma (e.g., rhabdoid tumor of the kidney and Wilms' tumor), renal pelvis tumor, leukemia, liver cancer (e.g., hepatocellular carcinoma), esophageal cancer (also known as "oesophageal cancer", e.g., esophageal squamous cell carcinoma), lung cancer (e.g., non-small cell lung cancer and small cell lung cancer), lymphoma, medulloblastoma, melanoma, meningioma, Merkel cell carcinoma, mesothelioma, multiple myeloma, neuroblastoma, oligodendroglioma, ovarian cancer, peritoneal tumor, pancreatic cancer, polycythemia vera, primary neuroectodermal tumor, prostate cancer, retinoblastoma, salivary gland cancer, sarcoma (e.g., chondrosarcoma, Ewing sarcoma, osteosarcoma, rhabdomyosarcoma, synovial sarcoma, and soft tissue sarcoma), small intestine cancer, colorectal cancer, squamous cell carcinoma (e.g., cutaneous squamous cell carcinoma), thyroid cancer, endometrial cancer, vestibular schwannoma, blastoma, teratoma, pituitary cancer, vulva cancer, thymoma, testicular cancer, bile duct cancer, pheochromocytoma, paraganglioma, and adenoid cystic carcinoma. In some embodiments, the disease is selected from the group consisting of ovarian cancer, lung cancer, esophageal squamous cell carcinoma, colorectal cancer, renal cell carcinoma, and melanoma.

In some embodiments, provided is the aforementioned method for treating a disease, wherein the method further comprises using a second therapeutic agent. In some embodiments, the second therapeutic agent comprises an immune checkpoint inhibitor. In some embodiments, the second therapeutic agent is an anti-PD-1 antibody.

In some embodiments, provided is the aforementioned method for treating a disease, wherein the anti-PD-1 antibody comprises a heavy chain variable region PD-1-VH and a light chain variable region PD-1-VL, wherein:
the PD-1-VH comprises: a PD-1-HCDR1 comprising an amino acid sequence of SEQ ID NO: 250, a PD-1-HCDR2 comprising an amino acid sequence of SEQ ID NO: 251, and a PD-1-HCDR3 comprising an amino acid sequence of SEQ ID NO: 252, and the PD-1-VL comprises: a PD-1-LCDR1 comprising an amino acid sequence of SEQ ID NO: 253, a PD-1-LCDR2 comprising an amino acid sequence of SEQ ID NO: 254, and a PD-1-LCDR3 comprising an amino acid sequence of SEQ ID NO: 255.

In some embodiments, provided is the aforementioned method for treating a disease, wherein the anti-PD-1 antibody comprises a heavy chain variable region PD-1-VH and a light chain variable region PD-1-VL, wherein:
the PD-1-VH comprises an amino acid sequence of SEQ ID NO: 256, and the PD-1-VL comprises an amino acid sequence of SEQ ID NO: 257.

In some embodiments, provided is the aforementioned method for treating a disease, wherein the anti-PD-1 antibody comprises a heavy chain and a light chain, wherein:
the heavy chain comprises an amino acid sequence of SEQ ID NO: 248, and the light chain comprises an amino acid sequence of SEQ ID NO: 249.

In another aspect, provided is use of a combination of the anti-ILT4 antibody and the anti-PD-1 antibody according to any one of the above in the preparation of a medicament for treating a disease.

In some embodiments, the aforementioned medicament is used for treating a disease; preferably, the disease is a tumor; more preferably, the disease is a disease selected from the group consisting of astrocytoma (e.g., anaplastic astrocytoma), glioblastoma, bladder cancer, bone cancer, brain cancer, breast cancer (e.g., breast cancer characterized by BRCA1 and/or BRCA2 mutations), carcinoid, cervical cancer, choroid plexus papilloma, colorectal cancer (e.g., colon cancer and rectal cancer), fallopian tube cancer, ependymoma, gallbladder cancer, gastric cancer, head and neck cancer, idiopathic myelofibrosis, renal cancer or renal cell carcinoma (e.g., rhabdoid tumor of the kidney and Wilms' tumor), renal pelvis tumor, leukemia, liver cancer (e.g., hepatocellular carcinoma), esophageal cancer (also known as "oesophageal cancer", e.g., esophageal squamous cell carcinoma), lung cancer (e.g., non-small cell lung cancer and small cell lung cancer), lymphoma, medulloblastoma, melanoma, meningioma, Merkel cell carcinoma, mesothelioma, multiple myeloma, neuroblastoma, oligodendroglioma, ovarian cancer, peritoneal tumor, pancreatic cancer, polycythemia vera, primary neuroectodermal tumor, prostate cancer, retinoblastoma, salivary gland cancer, sarcoma (e.g., chondrosarcoma, Ewing sarcoma, osteosarcoma, rhabdomyosarcoma, synovial sarcoma, and soft tissue sarcoma), small intestine cancer, colorectal cancer, squamous cell carcinoma (e.g., cutaneous squamous cell carcinoma), thyroid cancer, endometrial cancer, vestibular schwannoma, blastoma, teratoma, pituitary cancer, vulva cancer, thymoma, testicular cancer, bile duct cancer, pheochromocytoma, paraganglioma, and adenoid cystic carcinoma. In some embodiments, the aforementioned medicament is used for treating a disease selected from the group consisting of ovarian cancer, lung cancer, esophageal squamous cell carcinoma, colorectal cancer, renal cell carcinoma, and melanoma.

In some embodiments, provided is the aforementioned use of a combination of the anti-ILT4 antibody and the anti-PD-1 antibody in the preparation of a medicament for treating a disease, wherein the anti-ILT4 antibody and the anti-PD-1 antibody are in the same container or different containers.

In some embodiments, provided is the aforementioned use of a combination of the anti-ILT4 antibody and the anti-PD-1 antibody in the preparation of a medicament for treating a disease, wherein the anti-PD-1 antibody comprises a heavy chain variable region PD-1-VH and a light chain variable region PD-1-VL, wherein:

the PD-1-VH comprises: a PD-1-HCDR1 comprising an amino acid sequence of SEQ ID NO: 250, a PD-1-HCDR2 comprising an amino acid sequence of SEQ ID NO: 251, and a PD-1-HCDR3 comprising an amino acid sequence of SEQ ID NO: 252, and the PD-1-VL comprises: a PD-1-LCDR1 comprising an amino acid sequence of SEQ ID NO: 253, a PD-1-LCDR2 comprising an amino acid sequence of SEQ ID NO: 254, and a PD-1-LCDR3 comprising an amino acid sequence of SEQ ID NO: 255.

In some embodiments, provided is the aforementioned use of a combination of the anti-ILT4 antibody and the anti-PD-1 antibody in the preparation of a medicament for treating a disease, wherein the anti-PD-1 antibody comprises a heavy chain variable region PD-1-VH and a light chain variable region PD-1-VL, wherein:

the PD-1-VH comprises an amino acid sequence of SEQ ID NO: 256, and the PD-1-VL comprises an amino acid sequence of SEQ ID NO: 257.

In some embodiments, provided is the aforementioned use of a combination of the anti-ILT4 antibody and the anti-PD-1 antibody in the preparation of a medicament for treating a disease, wherein the anti-PD-1 antibody comprises a heavy chain and a light chain, wherein:

the heavy chain comprises an amino acid sequence of SEQ ID NO: 248, and the light chain comprises an amino acid sequence of SEQ ID NO: 249.

In another aspect, provided is a combination of the anti-ILT4 antibody and the anti-PD-1 antibody according to any one of the above for use in the treatment of a disease; preferably, the disease is a tumor; more preferably, the disease is a disease selected from the group consisting of astrocytoma (e.g., anaplastic astrocytoma), glioblastoma, bladder cancer, bone cancer, brain cancer, breast cancer (e.g., breast cancer characterized by BRCA1 and/or BRCA2 mutations), carcinoid, cervical cancer, choroid plexus papilloma, colorectal cancer (e.g., colon cancer and rectal cancer), fallopian tube cancer, ependymoma, gallbladder cancer, gastric cancer, head and neck cancer, idiopathic myelofibrosis, renal cancer or renal cell carcinoma (e.g., rhabdoid tumor of the kidney and Wilms' tumor), renal pelvis tumor, leukemia, liver cancer (e.g., hepatocellular carcinoma), esophageal cancer (also known as "oesophageal cancer", e.g., esophageal squamous cell carcinoma), lung cancer (e.g., non-small cell lung cancer and small cell lung cancer), lymphoma, medulloblastoma, melanoma, meningioma, Merkel cell carcinoma, mesothelioma, multiple myeloma, neuroblastoma, oligodendroglioma, ovarian cancer, peritoneal tumor, pancreatic cancer, polycythemia vera, primary neuroectodermal tumor, prostate cancer, retinoblastoma, salivary gland cancer, sarcoma (e.g., chondrosarcoma, Ewing sarcoma, osteosarcoma, rhabdomyosarcoma, synovial sarcoma, and soft tissue sarcoma), small intestine cancer, colorectal cancer, squamous cell carcinoma (e.g., cutaneous squamous cell carcinoma), thyroid cancer, endometrial cancer, vestibular schwannoma, blastoma, teratoma, pituitary cancer, vulva cancer, thymoma, testicular cancer, bile duct cancer, pheochromocytoma, paraganglioma, and adenoid cystic carcinoma. In some embodiments, the aforementioned medicament is used for treating a disease selected from the group consisting of ovarian cancer, lung cancer, esophageal squamous cell carcinoma, colorectal cancer, renal cell carcinoma, and melanoma.

## BRIEF DESCRIPTION OF THE DRAWINGS

FIG. 1A shows the blocking effect of the anti-ILT4 antibody huP2-E3 on ILT4 and its downstream signaling pathways.
FIG. 1B shows the blocking effect of the anti-ILT4 antibody huP2-G9 on ILT4 and its downstream signaling pathways.
FIG. 1C shows the blocking effect of the anti-ILT4 antibody huP2-C6 on ILT4 and its downstream signaling pathways.
FIG. 1D shows the blocking effect of the anti-ILT4 antibody P2m on ILT4 and its downstream signaling pathways.
FIG. 2A shows the effect of the anti-ILT4 antibody huP2-G9 on the secretion of the cytokine TNF-$\alpha$ by macrophages.
FIG. 2B shows the effect of the anti-ILT4 antibody huP2-C6 on the secretion of the cytokine TNF-$\alpha$ by macrophages.
FIG. 2C shows the effect of the anti-ILT4 antibody huP2-E8 on the secretion of the cytokine TNF-$\alpha$ by macrophages.
FIG. 2D shows the effect of the anti-ILT4 antibody P2m on the secretion of the cytokine TNF-$\alpha$ by macrophages.

FIG. 3A shows the combined effect of the anti-ILT4 antibody huP2-E3 and the anti-PD-1 antibody in allogeneic MLR.
FIG. 3B shows the combined effect of the anti-ILT4 antibody huP2-G9 and the anti-PD-1 antibody in allogeneic MLR.
FIG. 3C shows the combined effect of the anti-ILT4 antibody huP2-C6 and the anti-PD-1 antibody in allogeneic MLR.
FIG. 3D shows the combined effect of the anti-ILT4 antibody huP2-E8 and the anti-PD-1 antibody in allogeneic MLR.
FIG. 3E shows the combined effect of the anti-ILT4 antibody P2m and the anti-PD-1 antibody in allogeneic MLR.

## DETAILED DESCRIPTION

### Terminology

To facilitate the understanding of the present disclosure, some technical and scientific terms are described below. Unless otherwise specifically defined herein, all technical and scientific terms used herein have the same meaning as commonly understood by those of ordinary skill in the art.

The singular forms "a", "an", and "the" used in the description and claims include plural reference unless the context clearly dictates otherwise.

Unless otherwise clearly stated in the context, throughout the description and the claims, the words "comprise", "have", "include", and the like, should be construed in an inclusive sense as opposed to an exclusive or exhaustive sense.

The term "cytokine" is a general term for proteins that are released by a cell population to act on other cells as intercellular mediators. Examples of such cytokines include lymphokines, monokines, chemokines, and traditional polypeptide hormones. Exemplary cytokines include: mIL-2, IFNγ, TNFα, CCL-2, and IL-6.

The term "and/or" is meant to include the two meanings "and" and "or". For example, the phrase "A, B, and/or C" is intended to encompass each of the following: A, B, and C; A, B, or C; A or C; A or B; B or C; A and C; A and B; B and C; A (alone); B (alone); and C (alone).

The three-letter and single-letter codes for amino acids used herein are as described in J. biol. chem. 243, p3558 (1968).

The term "ILT4" refers to full-length ILT4 protein. Human ILT4 comprises an amino acid sequence of SEQ ID NO: 3. The amino acid sequences of ILT4 molecules from non-human species (e.g., mice, monkeys, rabbits, dogs, and pigs) may be obtained from public sources.

The term "αPD-1" refers to an anti-PD-1 antibody.

The term "amino acid" refers to naturally occurring and synthetic amino acids, as well as amino acid analogs and amino acid mimics that function in a manner similar to the naturally occurring amino acids. Naturally occurring amino acids are those encoded by genetic codes and those amino acids later modified, e.g., hydroxyproline, γ-carboxyglutamic acid, and O-phosphoserine. Amino acid analogs refer to compounds that have a substantially identical chemical structure (i.e., an α carbon that binds to hydrogen, carboxyl, amino, and an R group) to naturally occurring amino acids, e.g., homoserine, norleucine, methionine sulfoxide, and methioninemethyl sulfonium. Such analogs have a modified R group (e.g., norleucine) or a modified peptide skeleton, but retain a substantially identical chemical structure to naturally occurring amino acids. Amino acid mimics refer to chemical compounds that have a different structure from amino acids, but function in a manner similar to naturally occurring amino acids.

The term "amino acid mutation" includes amino acid substitutions (also known as amino acid replacements), deletions, insertions, and modifications. Any combination of substitutions, deletions, insertions, and modifications can be made to obtain a final construct, as long as the final construct possesses the desired properties, such as reduced binding to the Fc receptor. Amino acid sequence deletions and insertions include deletions and insertions at the amino terminus and/or the carboxyl terminus of a polypeptide chain. Specific amino acid mutations may be amino acid substitutions. In one embodiment, the amino acid mutation is a non-conservative amino acid substitution, i.e., the replacement of one amino acid with another amino acid having different structural and/or chemical properties. Amino acid substitutions include replacement with non-naturally occurring amino acids or with derivatives of the 20 natural amino acids (e.g., 4-hydroxyproline, 3-methylhistidine, ornithine, homoserine, and 5-hydroxylysine). Amino acid mutations can be generated using genetic or chemical methods well known in the art. Genetic methods may include site-directed mutagenesis, PCR, gene synthesis, and the like. It is contemplated that methods for altering amino acid side chain groups other than genetic engineering, such as chemical modification, may also be used. Various names may be used herein to indicate the same amino acid mutation. Herein, the expression of "position + amino acid residue" may be used to denote an amino acid residue at a specific position. For example, 102S indicates that the amino acid residue at position 102 is S. C102S indicates that the amino acid residue at position 102 is mutated from the original C into S. It should be understood that when an amino acid sequence is defined in the claims using a position + residue, the amino acid at that position before mutation does not limit the technical solutions. The term "antibody" is used in the broadest sense and encompasses a variety of antibody structures, including but not limited to monoclonal antibodies, polyclonal antibodies; monospecific antibodies, multispecific antibodies (e.g., bispecific antibodies); and full-length antibodies, and antibody fragments (or antigen-binding fragments, or antigen-binding moieties) so long as they exhibit the desired antigen-binding activity.

"Natural antibody" refers to a naturally occurring immunoglobulin molecule. For example, a natural IgG antibody is a heterotetrameric glycoprotein of about 150,000 Daltons composed of two light chains and two heavy chains linked by a disulfide bond. From the N-terminus to the C-terminus, each heavy chain has one variable region (VH), also known as variable heavy domain or heavy chain variable region, followed by heavy chain constant regions. Generally, a natural IgG heavy chain constant region comprises three constant domains (CH1, CH2, and CH3). Similarly, from the N-terminus to the C-terminus, each light chain has one variable region (VL), also known as variable light domain or light chain variable domain, followed by one constant light domain (light chain constant region, CL). The term "full-length antibody", "intact antibody", and "whole antibody" are used herein interchangeably and refer to an antibody having a substantially similar structure to a natural antibody structure or having heavy chains in an Fc region as defined herein. In a natural intact antibody, the light chain comprises light chain variable region VL and constant region CL, wherein the VL is positioned at the amino terminus of the light chain, and the light chain constant region comprises a κ chain and a λ chain; the heavy chain comprises a variable region VH and a constant region (CH1, CH2, and CH3), wherein the VH is positioned at the amino terminus of the heavy chain, and the constant region is positioned at the carboxyl terminus, wherein the CH3 is closest to the carboxyl terminus of the polypeptide, and the heavy chain can be of any isotype, including IgG (including subtypes IgG1, IgG2, IgG3, and IgG4), IgA (including subtypes IgA1 and IgA2), IgM, and IgE.

The term "variable region" or "variable domain" of an antibody refers to a domain in an antibody heavy or light chain that is involved in the binding of the antibody to an antigen. Herein, the heavy chain variable region (VH) and light chain variable region (VL) of the antibody each comprise four conserved framework regions (FRs) and three complementarity determining regions (CDRs). The term "complementarity determining region" or "CDR" refers to a region in the variable domain that primarily contributes to antigen binding; "framework" or "FR" refers to variable domain residues other than CDR residues. A VH comprises 3 CDRs: HCDR1, HCDR2, and HCDR3; a VL comprises 3 CDRs: LCDR1, LCDR2, and LCDR3. Each VH and VL is composed of three CDRs and four FRs arranged from the amino terminus (also known as N-terminus) to the carboxyl terminus (also known as C-terminus) in the following order: FR1, CDR1, FR2, CDR2, FR3, CDR3, and FR4.

The amino acid sequence boundaries of the CDRs can be determined by a variety of well-known schemes, for example, the "Kabat" numbering scheme (see Kabat et al., (1991), "Sequences of Proteins of Immunological Interest", 5th ed., Public Health Service, National Institutes of Health, Bethesda, MD), the "Chothia" numbering scheme, the "ABM" numbering scheme, the "contact" numbering scheme (see Martin, ACR. Protein Sequence and Structure Analysis of Antibody Variable Domains [J]. 2001), and the ImMunoGenTics (IMGT) numbering scheme (Lefranc, M.P. et al., Dev. Comp. Immunol., 27, 55-77 (2003); Front Immunol. 2018 Oct 16; 9: 2278), and the like. The corresponding relationships between the various numbering schemes are well known to those skilled in the art and, illustratively, are shown in Table 1 below.

Table 1. Relationshios between CDR numbering schemes

| CDR | IMGT | Kabat | AbM | Chothia | Contact |
|---|---|---|---|---|---|
| HCDR1 | 27-38 | 31-35 | 26-35 | 26-32 | 30-35 |
| HCDR2 | 56-65 | 50-65 | 50-58 | 52-56 | 47-58 |
| HCDR3 | 105-117 | 95-102 | 95-102 | 95-102 | 93-101 |
| LCDR1 | 27-38 | 24-34 | 24-34 | 24-34 | 30-36 |
| LCDR2 | 56-65 | 50-56 | 50-56 | 50-56 | 46-55 |
| LCDR3 | 105-117 | 89-97 | 89-97 | 89-97 | 89-96 |

Unless otherwise stated, the "Kabat" numbering scheme is applied to the variable regions and CDRs in examples of the present disclosure. Although one numbering scheme (e.g., Kabat) is employed to define amino acid residues in specific embodiments, corresponding technical solutions for other numbering schemes are to be considered as equivalent technical solutions.

The term "antibody fragment" refers to a molecule different from an intact antibody, which comprises a moiety of an intact antibody that binds to an antigen to which the intact antibody binds. Examples of antibody fragments include, but are not limited to, Fv, Fab, Fab', Fab'-SH, F(ab')2, single-domain antibodies, single-chain Fab (scFab), diabodies, linear antibodies, single-chain antibodies (e.g., scFv), and multispecific antibodies formed from antibody fragments.

The term "Fc region" or "fragment crystallizable region" is used to define the C-terminal region of the heavy chain of an antibody, including native Fc regions and engineered Fc regions. In some embodiments, the Fc region comprises two subunits, which may be identical or different. In some embodiments, the Fc region of the human IgG heavy chain is defined as extending from the amino acid residue at position Cys226 or from Pro230 to its carboxyl terminus. Suitable Fc regions used for the antibodies described herein include Fc regions of human IgG1, IgG2 (IgG2A and IgG2B), IgG3, and IgG4. In some embodiments, the boundaries of the Fc region may also be varied, for example, by deleting the C-terminal lysine of the Fc region (residue 447 according to the EU numbering scheme) or deleting the C-terminal glycine and lysine of the Fc region (residues 446 and 447 according to the EU numbering scheme). Unless otherwise stated, the numbering scheme for the Fc region is the EU numbering scheme, also known as the EU index.

The term "chimeric" antibody refers to an antibody in which a portion of the heavy and/or light chain in the antibody is derived from a particular source or species, while the remainder of the heavy and/or light chain is derived from another different source or species.

The term "humanized" antibody refers to an antibody that retains the reactivity of a non-human antibody while having low immunogenicity in humans. For example, this can be achieved by retaining the non-human CDRs and replacing the remainder of the antibody with its human counterparts (i.e., the constant regions and the framework region portion of the variable regions).

The terms "human antibody", "human-derived antibody", "fully human antibody", and "complete human antibody" are used interchangeably and refer to antibodies in which the variable and constant regions are human sequences. The term encompasses antibodies that are derived from human genes but have, for example, sequences that have been altered to, e.g., reduce possible immunogenicity, increase affinity, and eliminate cysteines or glycosylation sites that may cause undesired folding. The term encompasses antibodies recombinantly produced in non-human cells (that may confer glycosylation not characteristic of human cells). The term also encompasses antibodies that have been produced in transgenic mice comprising some or all of the human immunoglobulin heavy and light chain loci. The meaning of human antibodies explicitly excludes humanized antibodies.

The term "affinity" refers to the overall strength of the non-covalent interaction between a single binding site of a molecule (e.g., an antibody) and its binding ligand (e.g., an antigen). Unless otherwise indicated, as used herein, binding "affinity" refers to an internal binding affinity that reflects the 1:1 interaction between members of a binding pair (e.g., an antibody and an antigen). The affinity of a molecule X for its ligand Y can be generally denoted by the dissociation constant (KD). Affinity can be determined by conventional methods known in the art, including those described herein.

As used herein, the term "kassoc" or "ka" refers to the association rate of a particular antibody-antigen interaction, while the term "kdis" or "kd" refers to the dissociation rate of a particular antibody-antigen interaction. The term "KD" refers to the dissociation constant, which is obtained from the ratio of kd to ka (i.e., kd/ka) and denoted by a molar concentration (M). The KD value of an antibody can be determined using methods well known in the art. For example, surface plasmon resonance is determined using a biosensing system such as a system (e.g., Biacore), or affinity in a solution is determined by solution equilibrium titration (SET).

The term "surface plasmon resonance" refers to an optical phenomenon that allows for the analysis of real-time interactions by detecting changes in protein concentrations within a biosensor matrix, for example, using the BIAcoreTM system (Biacore Life Sciences division of GE Healthcare, Piscataway, NJ).

The term "effector function" refers to biological activities that can be attributed to the Fc region of an antibody (either the natural sequence Fc region or the amino acid sequence variant Fc region) and vary with the antibody isotype. Examples of effector functions of an antibody include, but are not limited to: C1q binding and complement-dependent cytotoxicity, Fc receptor binding, antibody-dependent cell-mediated cytotoxicity (ADCC), phagocytosis, down-regulation of cell surface receptors (e.g., B cell receptors), and B cell activation.

The term "monoclonal antibody" refers to a population of substantially homogeneous antibodies, that is, the amino acid sequences of the antibody molecules comprised in the population are identical, except for a small number of natural mutations that may exist. In contrast, a polyclonal antibody generally comprises a plurality of different antibodies having different amino acid sequences in their variable domains, which are generally specific for different epitopes. "Monoclonal" should not be construed as requiring the production of the antibody by any particular method. In some embodiments, the antibody provided by the present disclosure is a monoclonal antibody.

The term "antigen" refers to a molecule or a portion of a molecule that can be selectively bound, for example, by an antigen-binding protein (including, for example, an antibody). An antigen may have one or more epitopes capable of interacting with different antigen-binding proteins (e.g., antibodies).

The term "epitope" refers to an area or region on an antigen that is capable of specifically binding to an antibody or an antigen-binding fragment thereof. Epitopes can be formed from contiguous strings of amino acids (linear epitope) or comprise noncontiguous amino acids (conformational epitope), e.g., coming in spatial proximity due to the folding of the antigen. The difference between the conformational epitope and the linear epitope is that in the presence of denaturing solvents, the binding of the antibody to the conformational epitope is lost. An epitope comprises at least 3, at least 4, at least 5, at least 6, at least 7, or 8-10 amino acids in a unique spatial conformation. Screening for antibodies that bind to particular epitopes (i.e., those that bind to identical epitopes) can be performed using routine methods in the art, including, for example, but not limited to, alanine scanning, peptide blotting, peptide cleavage analysis, epitope excision, epitope extraction, chemical modification of the antigen (Prot. Sci. 9 (2000) 487-496), and cross-blocking.

The term "capable of specifically binding", "specifically binding", or "binding" means that an antibody is capable of binding to a certain antigen or an epitope thereof with higher affinity than to other antigens or epitopes. Generally, an antibody binds to an antigen or an epitope thereof with an equilibrium dissociation constant (KD) of about $1 \times 10^{-7}$ M or less (e.g., about $1 \times 10^{-8}$ M, $1 \times 10^{-9}$ M, $1 \times 10^{-10}$ M, $1 \times 10^{-11}$ M, or less). In some embodiments, the KD for the binding of an antibody to an antigen is 10% or less (e.g., 1%) of the KD for the binding of the antibody to a non-specific antigen (e.g., BSA or casein). KD may be determined using known methods, for example, by a BIACORE® surface plasmon resonance assay. However, an antibody that specifically binds to an antigen or an epitope thereof may have cross-reactivity to other related antigens, e.g., to corresponding antigens from other species (homologous), such as humans or monkeys, e.g., Macaca fascicularis (cynomolgus, cyno), Pan troglodytes (chimpanzee, chimp), or Callithrix jacchus (commonmarmoset, marmoset).

The terms "anti-ILT4 antibody" and "antibody that binds to ILT4" refer to an antibody that is capable of binding to ILT4 or an epitope thereof with sufficient affinity.

The term "antibody-dependent cellular cytotoxicity", "antibody-dependent cell-mediated cytotoxicity", or "ADCC" is a mechanism for inducing cell death, which depends on the interaction of antibody-coated target cells with effector cells with lytic activity, such as natural killer cells (NK), monocytes, macrophages, and neutrophils, via an Fcγ receptor (FcγR) expressed on the effector cells. For example, NK cells express FcγRIIIa, while monocytes express FcγRI, FcγRII, and FcγRIIIa. The ADCC activity of the antibodies provided herein can be assessed by *in vitro* assays, using cells expressing the antigen as target cells and NK cells as effector cells. Cell lysis is detected based on the release of a label (e.g., radioactive substrate, fluorescent dye, or natural intracellular protein) from the lysed cells.

The term "antibody-dependent cellular phagocytosis (ADCP)" refers to a mechanism by which antibody-coated target cells are eliminated by internalization of phagocytic cells (such as macrophages or dendritic cells).

The term "complement-dependent cytotoxicity" or "CDC" refers to a mechanism for inducing cell death in which the Fc effector domain of a target-binding antibody binds to and activates a complement component C1q, and C1q then activates the complement cascade, resulting in the death of the target cell. Activation of a complement may also result in the deposition of complement components on the surface of target cells that promote CDC by binding to complement receptors on leukocytes (e.g., CR3).

The term "nucleic acid" is used interchangeably herein with the term "polynucleotide" and refers to deoxyribonucleotide or ribonucleotide and a polymer thereof in either single-stranded or double-stranded form. The term encompasses nucleic acids comprising known nucleotide analogs or modified backbone residues or linkages, which are synthetic, naturally occurring, and non-naturally occurring, have similar binding properties to the reference nucleic acid, and are metabolized in a manner similar to the reference nucleotide. Examples of such analogs include, but are not limited to, phosphorothioate, phosphoramidate, methylphosphonate, chiral-methylphosphonate, 2-O-methyl ribonucleotide, and peptide-nucleic acid (PNA).

"Isolated" nucleic acid refers to a nucleic acid molecule that has been separated from components of its natural environment. An isolated nucleic acid encoding a polypeptide refers to one or more nucleic acid molecules encoding the polypeptide, including such one or more nucleic acid molecules in a single vector or separate vectors, and such one or more nucleic acid molecules present at one or more positions in a host cell. Unless otherwise stated, a particular nucleic acid sequence also implicitly encompasses conservatively modified variants thereof (e.g., degenerate codon substitutions) and complementary sequences, as well as the sequence explicitly indicated. Specifically, as detailed below, degenerate codon substitutions may be obtained by generating sequences in which the third position of one or more selected (or all) codons is substituted with mixed bases and/or deoxyinosine residues.

The terms "polypeptide" and "protein" are used interchangeably herein and refer to a polymer of amino acid residues. The terms apply to amino acid polymers in which one or more amino acid residues are artificial chemical mimics of corresponding naturally occurring amino acids, as well as to naturally occurring amino acid polymers and non-naturally occurring amino acid polymers. Unless otherwise stated, a particular polypeptide sequence also implicitly encompasses conservatively modified variants thereof.

The term sequence "identity" refers to the degree (percentage) to which the amino acids/nucleic acids of two sequences are identical at equivalent positions when the two sequences are optimally aligned; gaps are introduced, when necessary, to achieve the maximum percent sequence identity, and any conservative substitution is not seen as part of the sequence identity. To determine percent sequence identity, alignments can be accomplished by techniques known to those skilled in the art, for example, using publicly available computer software, such as BLAST, BLAST-2, ALIGN, ALIGN-2, or Megalign (DNASTAR) software. Those skilled in the art can determine parameters suitable for measuring alignment, including any algorithms required to achieve maximum alignment of the full length of the aligned sequences.

The term "vector" means a polynucleotide molecule capable of transporting another polynucleotide linked thereto. One type of vector is a "plasmid", which refers to a circular double-stranded DNA loop into which additional DNA segments can be ligated. Another type of vector is a viral vector, such as an adeno-associated viral vector (AAV or AAV2), wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome. The term "expression vector" or "expression construct" refers to a vector that can be transformed into a host cell and comprises a nucleic acid sequence that directs and/or controls (along with the host cell) the expression of one or more heterologous coding regions operably linked thereto. Expression constructs may include, but are not limited to, sequences that affect or control transcription and translation and affect RNA splicing of a coding region operably linked thereto in the presence of an intron.

The terms "host cell", "host cell line", and "host cell culture" are used interchangeably and refer to cells into which exogenous nucleic acids have been introduced, including progenies of such cells. Host cells include "transformants" and "transformed cells", which include primary transformed cells and progeny derived therefrom, regardless of the number of passages. Progeny may not be completely identical to parent cells in terms of nucleic acid content and may contain mutations. Mutant progenies that have the same function or biological activity as the cells screened or selected from the initially transformed cells are included herein. Host cells include prokaryotic and eukaryotic host cells, wherein the eukaryotic host cells include, but are not limited to, mammalian cells, insect cell lines, plant cells, and fungal cells. Mammalian host cells include human, mouse, rat, canine, monkey, porcine, goat, bovine, equine, and hamster cells, including but not limited to, Chinese hamster ovary (CHO) cells, NSO, SP2 cells, HeLa cells, baby hamster kidney (BHK) cells, monkey kidney cells (COS), human hepatocellular carcinoma cells (e.g., Hep G2), A549 cells, 3T3 cells, and HEK-293 cells. Fungal cells include yeast and filamentous fungal cells, including, for example, *Pichiapastoris, Pichia finlandica, Pichia trehalophila, Pichia koclamae, Pichia membranaefaciens, Pichia minuta (Ogataea minuta, Pichia lindneri), Pichiaopuntiae, Pichia thermotolerans, Pichia salictaria, Pichia guercuum, Pichia pijperi, Pichia stiptis, Pichia methanolica, Pichia, Saccharomycescerevisiae, Saccharomyces, Hansenula polymorpha, Kluyveromyces, Kluyveromyces lactis, Candida albicans, Aspergillus nidulans, Aspergillus niger, Aspergillus oryzae, Trichoderma reesei, Chrysosporium lucknowense, Fusarium sp., Fusarium gramineum, Fusarium venenatum, Physcomitrella patens,* and *Neurospora crassa. Pichia,* any *Saccharomyces, Hansenula polymorpha,* any *Kluyveromyces, Candida albicans,* any *Aspergillus, Trichoderma reesei, Chrysosporium lucknowense,* any *Fusarium, Yarrowia lipolytica,* and *Neurospora crassa.*

"Optional" or "optionally" means that the event or circumstance subsequently described may, but does not necessarily, occur, and this description includes instances where the event or circumstance occurs or does not occur.

The term "pharmaceutical composition" refers to a mixture comprising one or more of the anti-ILT4 antibodies described herein and other chemical components; the other components are, for example, physiological/pharmaceutically acceptable carriers and excipients.

The term "pharmaceutically acceptable carriers, diluents, or excipients" refers to ingredients in a pharmaceutical formulation that are different from the active ingredient and are not toxic to the subject. Pharmaceutically acceptable carriers, diluents, or excipients include, but are not limited to, buffers, excipients, stabilizers, or preservatives.

The term "subject" or "individual" includes humans and non-human animals. Non-human animals include all vertebrates (e.g., mammals and non-mammals) such as non-human primates, sheep, dogs, cows, chickens, amphibians, and reptiles. Unless indicated, the terms "patient" and "subject" are used interchangeably herein. In certain embodiments, the individual or subject is a human.

"Administrating" or "giving", when applied to animals, humans, experimental subjects, cells, tissue, organs, or biological fluids, refers to contact of an exogenous drug, a therapeutic agent, a diagnostic agent, or a composition with the animals, humans, subjects, cells, tissue, organs, or biological fluids.

The term "sample" refers to a collection isolated from a subject (such as fluids, cells, or tissues), as well as fluids, cells, or tissues present in a subject. Exemplary samples are biological fluids (such as blood; serum; serosal fluids; plasma; lymph; urine; saliva; cystic fluids; tears; excretions; sputum; mucosal secretions of secretory tissue or organs; vaginal secretions; ascites; fluids in the pleura; pericardium; peritoneum; fluids from abdominal cavity and other body cavities; fluids collected from bronchial lavage; synovial fluids; liquid solutions in contact with a subject or biological source, e.g., culture media (including conditioned media); and lavage fluids), tissue biopsy samples, fine needle punctures, surgically excised tissues, organ cultures, or cell cultures.

"Treatment" or "treat" (and grammatical variations thereof) refers to clinical intervention applied to the treated individual, and the clinical intervention may be performed either for prophylaxis or during the course of clinical pathology. Desirable effects of the treatment include, but are not limited to, preventing the occurrence or recurrence of a disease, alleviating symptoms, alleviating/reducing any direct or indirect pathological consequences of the disease, preventing metastasis, decreasing the rate of disease progression, ameliorating or alleviating the disease state, and regressing or improving prognosis. In some embodiments, the antibody of the present disclosure is used to delay the development of or slow the progression of a disease.

"Effective amount" is generally an amount sufficient to reduce the severity and/or frequency of symptoms, eliminate symptoms and/or underlying causes, prevent the appearance of symptoms and/or their underlying causes, and/or alleviate or ameliorate damage caused by or associated with a disease state (e.g., a lung disease). In some examples, the effective amount is a therapeutically effective amount or a prophylactically effective amount.

"Therapeutically effective amount" is an amount sufficient to treat a disease state or symptom, particularly a state or symptom associated with the disease state, or to otherwise prevent, hinder, delay, or reverse the progression of the disease state or any other undesirable symptoms associated with the disease in any way. "Prophylactically effective amount" is an amount that, when administered to a subject, will have a predetermined prophylactic effect, e.g., preventing or delaying the onset (or recurrence) of the disease state, or reducing the likelihood of the onset (or recurrence) of the disease state or associated symptoms. Complete treatment or prevention does not necessarily occur after administration of one dose and may occur after administration of a series of doses. Thus, a therapeutically or prophylactically effective amount may be administered in one or more doses. "Therapeutically effective amount" and "prophylactically effective amount" may vary depending on a variety of factors such as the disease state, age, sex, and weight of the individual, and the ability of a therapeutic agent or combination of therapeutic agents to elicit a desired response in the individual. Exemplary indicators of an effective therapeutic agent or combination of therapeutic agents include, for example, improved health of a patient.

The term "immune checkpoint" refers to a set of molecules on the cell surface of CD4 T cells and CD8 T cells. These molecules can effectively act as a "brake" to downregulate or inhibit the anti-tumor immune response. Immune checkpoint molecules include, but are not limited to, programmed death-1 (PD-1), cytotoxic T lymphocyte antigen 4 (CTLA-4), B7H1, B7H4, OX-40, CD137, CD40, and LAG-3, which directly inhibit immune cells.

Immune checkpoint inhibitors for use in the present disclosure are substances that inhibit the functions of immune checkpoint molecules. There is no particular limitation on immune checkpoint inhibitors as long as the inhibitors are substances that can inhibit the functions (signals) of immune checkpoint molecules. Inhibitors that can be used as immune checkpoint inhibitors in the methods of the present disclosure include, but are not limited to, inhibitors of PD-1, PD-L2, CTLA-4, TIM-3, LAG-3, VISTA, BTLA, TIGIT, LAIR1, CD160, 2B4, CEACAM (e.g., CEACAM-1, CEACAM-3, and/or CEACAM-5), and/or TGFRβ. The inhibition of an inhibitory molecule may be performed by inhibition of a DNA, RNA, or protein level. In an embodiment, an inhibitory nucleic acid (e.g., dsRNA, siRNA, or shRNA) may be used to inhibit the expression of an inhibitory molecule. In other embodiments, an inhibitor of an inhibitory signal is a polypeptide that binds to an inhibitory molecule, e.g., a soluble ligand or antibody. Examples of the immune checkpoint inhibitors used in the present disclosure may include, but are not particularly limited to, anti-PD-1 antibodies, anti-PD-L1 antibodies, and anti-CTLA-4 antibodies, and anti-PD-1 antibodies may be preferred.

**Target Molecule**

"ILT4" should be understood in the broadest sense and is intended to encompass various forms of molecules of ILT4 in various stages in mammals, such as, but not limited to, molecules produced by the ILT4 gene during amplification, replication, transcription, splicing, processing, translation, and modification, e.g., precursor ILT4, mature ILT4, ILT4 expressed on the membrane, ILT4 splice variants, modified ILT4, or fragments thereof; the term also encompasses ILT4 artificially prepared or expressed in vitro.

**Disclosed Anti-ILT4 Antibodies**

The present disclosure provides anti-ILT4 antibodies having a number of advantageous properties, such as good *in vitro* killing activity, therapeutic activity, safety, pharmacokinetic properties, and druggability (e.g., yield, purity, and stability).

**Exemplary Anti-ILT4 Antibodies**

The examples of the present disclosure disclose the antibody series P1-B4, P1-B9, P1-D10, P2-E8, P2-C10, P2-F4, P2-B3, P2-E7, P2-G4, P2-C4, P2-E11, P1-F7, P2-E3, P2-F10, P2-G9, P1-C4, P1-G7, P2-C7, P2-C6, P1-D7, P1-E11, P2-B9, P2-C2, and P2-E10. Below, the antibodies P2-G9 and P2-C6 are used as examples to describe the antibodies of the present disclosure.

Illustratively, provided is an anti-ILT4 antibody of the present disclosure, wherein the anti-ILT4 antibody comprises a heavy chain variable region and a light chain variable region, wherein:

a. the HCDR1, the HCDR2, and the HCDR3 of the heavy chain variable region comprise amino acid sequences of a HCDR1, a HCDR2, and a HCDR3, respectively, in SEQ ID NO: 184 or 185, and the LCDR1, the LCDR2, and the LCDR3 of the light chain variable region comprise amino acid sequences of a LCDR1, a LCDR2, and a LCDR3, respectively, in SEQ ID NO: 187 or 186; or the HCDR1, the HCDR2, and the HCDR3 of the heavy chain variable region comprise amino acid sequences of a HCDR1, a HCDR2, and a HCDR3, respectively, in SEQ ID NO: 32, and the LCDR1, the LCDR2, and the LCDR3 of the light chain variable region comprise amino acid sequences of a LCDR1, a LCDR2, and a LCDR3, respectively, in SEQ ID NO: 33; or

b. the HCDR1, the HCDR2, and the HCDR3 of the heavy chain variable region comprise amino acid sequences of a HCDR1, a HCDR2, and a HCDR3, respectively, in any one of SEQ ID NO: 211, 205, 206, 207, 208, 209, or 210, and the LCDR1, the LCDR2, and the LCDR3 of the light chain variable region comprise amino acid sequences of a LCDR1, a LCDR2, and a LCDR3, respectively, in any one of SEQ ID NO: 212, 213, or 214; or the HCDR1, the HCDR2, and the HCDR3 of the heavy chain variable region comprise amino acid sequences of a HCDR1, a HCDR2, and a HCDR3, respectively, in SEQ ID NO: 40, and the LCDR1, the LCDR2, and the LCDR3 of the light chain variable region comprise amino acid sequences of a LCDR1, a LCDR2, and a LCDR3, respectively, in SEQ ID NO: 41.

Illustratively, provided is an anti-ILT4 antibody of the present disclosure, wherein the anti-ILT4 antibody comprises a heavy chain variable region and a light chain variable region, wherein:

a. the HCDR1, the HCDR2, and the HCDR3 of the heavy chain variable region comprise amino acid sequences of a HCDR1, a HCDR2, and a HCDR3, respectively, in SEQ ID NO: 184, and the LCDR1, the LCDR2, and the LCDR3 of the light chain variable region comprise amino acid sequences of a LCDR1, a LCDR2, and a LCDR3, respectively, in SEQ ID NO: 187; or
b. the HCDR1, the HCDR2, and the HCDR3 of the heavy chain variable region comprise amino acid sequences of a HCDR1, a HCDR2, and a HCDR3, respectively, in SEQ ID NO: 211, and the LCDR1, the LCDR2, and the LCDR3 of the light chain variable region comprise amino acid sequences of a LCDR1, a LCDR2, and a LCDR3, respectively, in SEQ ID NO: 212.

Illustratively, provided is an anti-ILT4 antibody of the present disclosure, wherein the HCDR1, the HCDR2, and the HCDR3 of the heavy chain variable region and the LCDR1, the LCDR2, and the LCDR3 of the light chain variable region are defined according to the same numbering scheme selected from the group consisting of Kabat, IMGT, Chothia, AbM, and Contact.
Illustratively, provided is an anti-ILT4 antibody of the present disclosure, wherein the anti-ILT4 antibody comprises a heavy chain variable region and a light chain variable region, wherein:
in the heavy chain variable region, the HCDR1 comprises an amino acid sequence of SEQ ID NO: 105, the HCDR2 comprises an amino acid sequence of SEQ ID NO: 120, and the HCDR3 comprises an amino acid sequence of SEQ ID NO: 121; and in the light chain variable region, the LCDR1 comprises an amino acid sequence of SEQ ID NO: 114, the LCDR2 comprises an amino acid sequence of SEQ ID NO: 109, and the LCDR3 comprises an amino acid sequence of SEQ ID NO: 119.
Illustratively, provided is an anti-ILT4 antibody of the present disclosure, wherein the anti-ILT4 antibody comprises a heavy chain variable region and a light chain variable region, wherein:

in the heavy chain variable region, the HCDR1 comprises an amino acid sequence of SEQ ID NO: 135, the HCDR2 comprises an amino acid sequence of SEQ ID NO: 203, 136, 201, 202, or 204, and the HCDR3 comprises an amino acid sequence of SEQ ID NO: 137; and in the light chain variable region, the LCDR1 comprises an amino acid sequence of SEQ ID NO: 138, the LCDR2 comprises an amino acid sequence of SEQ ID NO: 139, and the LCDR3 comprises an amino acid sequence of SEQ ID NO: 140;
preferably, in the heavy chain variable region, the HCDR1 comprises an amino acid sequence of SEQ ID NO: 135, the HCDR2 comprises an amino acid sequence of SEQ ID NO: 203, and the HCDR3 comprises an amino acid sequence of SEQ ID NO: 137; and in the light chain variable region, the LCDR1 comprises an amino acid sequence of SEQ ID NO: 138, the LCDR2 comprises an amino acid sequence of SEQ ID NO: 139, and the LCDR3 comprises an amino acid sequence of SEQ ID NO: 140.

Illustratively, provided is an anti-ILT4 antibody of the present disclosure, wherein the HCDR1, the HCDR2, and the HCDR3 of the heavy chain variable region and the LCDR1, the LCDR2, and the LCDR3 of the light chain variable region are defined according to the Kabat numbering scheme.
Illustratively, provided is an anti-ILT4 antibody of the present disclosure, wherein:

a. in the heavy chain variable region, the HCDR1 comprises an amino acid sequence of SEQ ID NO: 260, the HCDR2 comprises an amino acid sequence of SEQ ID NO: 261, and the HCDR3 comprises an amino acid sequence of SEQ ID NO: 262; and in the light chain variable region, the LCDR1 comprises an amino acid sequence of SEQ ID NO: 263, the LCDR2 comprises an amino acid sequence of SEQ ID NO: 264, and the LCDR3 comprises an amino acid sequence of SEQ ID NO: 119; or
b. in the heavy chain variable region, the HCDR1 comprises an amino acid sequence of SEQ ID NO: 267, the HCDR2 comprises an amino acid sequence of SEQ ID NO: 268, and the HCDR3 comprises an amino acid sequence of SEQ ID NO: 269; and in the light chain variable region, the LCDR1 comprises an amino acid sequence of SEQ ID NO: 270, the LCDR2 comprises an amino acid sequence of SEQ ID NO: 271, and the LCDR3 comprises an amino acid sequence of SEQ ID NO: 140;

wherein the CDRs are defined according to the IMGT numbering scheme.

Illustratively, provided is an anti-ILT4 antibody of the present disclosure, wherein:

in the heavy chain variable region, the HCDR1 comprises an amino acid sequence set forth in SEQ ID NO: 260, the HCDR2 comprises an amino acid sequence set forth in SEQ ID NO: 261, and the HCDR3 comprises an amino acid sequence set forth in SEQ ID NO: 262; and in the light chain variable region, the LCDR1 comprises an amino acid sequence set forth in SEQ ID NO: 263, the LCDR2 comprises an amino acid sequence set forth in SEQ ID NO: 264, and the LCDR3 comprises an amino acid sequence set forth in SEQ ID NO: 119;
wherein the CDRs are defined according to the IMGT numbering scheme.

Illustratively, provided is an anti-ILT4 antibody of the present disclosure, wherein:

a. in the heavy chain variable region, the HCDR1 comprises an amino acid sequence of SEQ ID NO: 265, the HCDR2 comprises an amino acid sequence of SEQ ID NO: 266, and the HCDR3 comprises an amino acid sequence of SEQ ID NO: 121; and in the light chain variable region, the LCDR1 comprises an amino acid sequence of SEQ ID NO: 114, the LCDR2 comprises an amino acid sequence of SEQ ID NO: 109, and the LCDR3 comprises an amino acid sequence of SEQ ID NO: 119; or
b. in the heavy chain variable region, the HCDR1 comprises an amino acid sequence of SEQ ID NO: 272, the HCDR2 comprises an amino acid sequence of SEQ ID NO: 273, and the HCDR3 comprises an amino acid sequence of SEQ ID NO: 137; and in the light chain variable region, the LCDR1 comprises an amino acid sequence of SEQ ID NO: 138, the LCDR2 comprises an amino acid sequence of SEQ ID NO: 139, and the LCDR3 comprises an amino acid sequence of SEQ ID NO: 140;

wherein the CDRs are defined according to the Chothia numbering scheme.

Illustratively, provided is an anti-ILT4 antibody of the present disclosure, wherein:

in the heavy chain variable region, the HCDR1 comprises an amino acid sequence set forth in SEQ ID NO: 265, the HCDR2 comprises an amino acid sequence set forth in SEQ ID NO: 266, and the HCDR3 comprises an amino acid sequence set forth in SEQ ID NO: 121; and in the light chain variable region, the LCDR1 comprises an amino acid sequence set forth in SEQ ID NO: 114, the LCDR2 comprises an amino acid sequence set forth in SEQ ID NO: 109, and the LCDR3 comprises an amino acid sequence set forth in SEQ ID NO: 119;
wherein the CDRs are defined according to the Chothia numbering scheme.

Illustratively, provided is an anti-ILT4 antibody of the present disclosure, wherein the anti-ILT4 antibody is a murine antibody, a chimeric antibody, a humanized antibody, or a fully human-derived antibody. In some embodiments, the antibody is a chimeric antibody. In some embodiments, the antibody is a fully human-derived antibody. In some embodiments, the antibody is a humanized antibody.

Illustratively, provided is an anti-ILT4 antibody of the present disclosure, wherein the anti-ILT4 antibody comprises a framework region (FR) of a human antibody.

Illustratively, provided is an anti-ILT4 antibody of the present disclosure, wherein:

a. the heavy chain variable region comprises an amino acid sequence having at least 70% (e.g., at least 70%, 80%, 85%, 90%, 95%, 98%, or 99%) sequence identity to SEQ ID NO: 184, and the light chain variable region comprises an amino acid sequence having at least 70% (e.g., at least 70%, 80%, 85%, 90%, 95%, 98%, or 99%) sequence identity to SEQ ID NO: 187; or
b. the heavy chain variable region comprises an amino acid sequence having at least 70% (e.g., at least 70%, 80%, 85%, 90%, 95%, 98%, or 99%) sequence identity to SEQ ID NO: 211, and the light chain variable region comprises an amino acid sequence having at least 70% (e.g., at least 70%, 80%, 85%, 90%, 95%, 98%, or 99%) sequence identity to SEQ ID NO: 212.

Illustratively, provided is an anti-ILT4 antibody of the present disclosure, wherein the heavy chain variable region comprises a FR1, a FR2, and a FR3 that are derived from IGHV4-31*01 and a FR4 derived from IGHJ1*01, and the FRs are unsubstituted or have one or more amino acid substitutions selected from the group consisting of 1E, 27S, 30T, 44K, 71R, and 76S; and/or the light chain variable region comprises a FR1, a FR2, and a FR3 that are derived from IGKV1-39*01 and a FR4 derived from IGKJ4*01, and the FRs are unsubstituted or have one or more amino acid substitutions selected from the group consisting of 43S, 48V, and 71Y. In some embodiments, provided is the anti-ILT4 antibody, wherein in the heavy chain variable region, the HCDR1 comprises an amino acid sequence of SEQ ID NO: 105, the HCDR2 comprises an amino acid sequence of SEQ ID NO: 120, and the HCDR3 comprises an amino acid sequence of SEQ ID NO: 121, and the FRs of the heavy chain variable region comprise one or more amino acid substitutions selected from the group consisting of 1E, 27S, 30T, 44K, 71R, and 76S; and in the light chain variable region, the LCDR1 comprises an amino acid sequence of SEQ ID NO: 114, the LCDR2 comprises an amino acid sequence of SEQ ID NO: 109, and the LCDR3 comprises an amino acid sequence of SEQ ID NO: 119, and the FRs of the light chain variable region comprise one or more amino acid substitutions selected from the group consisting of 43S, 48V, and 71Y. In some embodiments, the variable regions and CDRs described above are defined according to the Kabat numbering scheme.

Illustratively, provided is an anti-ILT4 antibody of the present disclosure, wherein the heavy chain variable region comprises a FR1, a FR2, and a FR3 that are derived from IGHV1-18*01 and a FR4 derived from IGHJ6*01, and the FRs are unsubstituted or have one or more amino acid substitutions selected from the group consisting of 1E, 43E, 69L, 71V, 73K, 76N, and 93T; and/or the light chain variable region comprises a FR1, a FR2, and a FR3 that are derived from IGKV4-1*01 or IGKV3-11*01 and a FR4 derived from IGKJ4*01, and the FRs are unsubstituted or have one or more amino acid substitutions selected from the group consisting of 4L, 43P, 45K, 58I, 68R, and 85T. In some embodiments, provided is the anti-ILT4 antibody, wherein in the heavy chain variable region, the HCDR1 comprises an amino acid sequence of SEQ ID NO: 135, the HCDR2 comprises an amino acid sequence of SEQ ID NO: 203, 136, 201, 202, or 204, and the HCDR3 comprises an amino acid sequence of SEQ ID NO: 137, and the FRs of the heavy chain variable region comprise one or more amino acid substitutions selected from the group consisting of 1E, 43E, 69L, 71V, 73K, 76N, and 93T; and in the light chain variable region, the LCDR1 comprises an amino acid sequence of SEQ ID NO: 138, the LCDR2 comprises an amino acid sequence of SEQ ID NO: 139, and the LCDR3 comprises an amino acid sequence of SEQ ID NO: 140, and the FRs of the light chain variable region comprise one or more amino acid substitutions selected from the group consisting of 4L, 43P, 45K, 58I, 68R, and 85T. In some embodiments, the variable regions and CDRs described above are defined according to the Kabat numbering scheme.

Illustratively, provided is an anti-ILT4 antibody of the present disclosure, wherein the anti-ILT4 antibody is an antibody fragment; in some embodiments, the antibody fragment is selected from the group consisting of Fab, Fab', F(ab')2, Fd, Fv, scFv, dsFv, and dAb.

Illustratively, provided is an anti-ILT4 antibody of the present disclosure, wherein the anti-ILT4 antibody comprises a heavy chain constant region and a light chain constant region. In some embodiments, the heavy chain constant region is a human IgG1 or IgG4 heavy chain constant region. In some embodiments, the heavy chain constant region comprises 234A and 235A mutations; the mutations are numbered according to the EU index. In some embodiments, the light chain constant region is a human κ light chain constant region. In some embodiments, the heavy chain constant region comprises an amino acid sequence of SEQ ID NO: 240 or 171, and the light chain constant region comprises an amino acid sequence of SEQ ID NO: 172.

Illustratively, provided is an anti-ILT4 antibody of the present disclosure, wherein the anti-ILT4 antibody comprises a heavy chain and a light chain, wherein:

a. the heavy chain comprises an amino acid sequence having at least 70% (e.g., at least 70%, 80%, 85%, 90%, 95%, 98%, or 99%) sequence identity to SEQ ID NO: 228, and the light chain comprises an amino acid sequence having at least 70% (e.g., at least 70%, 80%, 85%, 90%, 95%, 98%, or 99%) sequence identity to SEQ ID NO: 229; or
b. the heavy chain comprises an amino acid sequence having at least 70% (e.g., at least 70%, 80%, 85%, 90%, 95%, 98%, or 99%) sequence identity to SEQ ID NO: 234, and the light chain comprises an amino acid sequence having at least 70% (e.g., at least 70%, 80%, 85%, 90%, 95%, 98%, or 99%) sequence identity to SEQ ID NO: 235.

Illustratively, provided is an anti-ILT4 antibody of the present disclosure, wherein the anti-ILT4 antibody comprises a heavy chain and a light chain, wherein:

a. the heavy chain comprises an amino acid sequence of SEQ ID NO: 228, and the light chain comprises an amino acid sequence of SEQ ID NO: 229; or
b. the heavy chain comprises an amino acid sequence of SEQ ID NO: 234, and the light chain comprises an amino acid sequence of SEQ ID NO: 235.

Illustratively, the present disclosure further provides an isolated anti-ILT4 antibody that competes for binding to human ILT4 or an epitope thereof with the anti-ILT4 antibody according to any one of the foregoing.

Illustratively, the anti-ILT4 antibody provided by the present disclosure specifically binds to human ILT4 and does not bind to other LILR family members: ILT2, ILT3, LILRB5, LILRA1, LILRA2, LILRA3, LILRA4, LILRA5, and/or LILRA6.

Illustratively, the ability of the isolated anti-ILT4 antibody of the present disclosure to bind to ILT4 protein is higher than that of a control antibody. Specifically, the isolated anti-ILT4 antibody of the present disclosure binds to human ILT4 with a KD value of less than $1 \times 10^{-8}$ M (e.g., less than $8 \times 10^{-9}$ M, less than $5 \times 10^{-9}$ M, less than $2 \times 10^{-9}$ M, less than $1 \times 10^{-9}$ M, less than $8 \times 10^{-10}$ M, less than $5 \times 10^{-10}$ M, less than $2 \times 10^{-10}$ M, less than $1 \times 10^{-10}$ M, less than $8 \times 10^{-11}$ M, or less than $5 \times 10^{-11}$ M), as measured by surface plasmon resonance (Biacore).

Illustratively, a humanized anti-ILT4 antibody provided by the present disclosure is capable of effectively removing the inhibitory effect of ILT4 and promoting the secretion of higher levels of mIL-2 by 3A9 cells.

Illustratively, a humanized anti-ILT4 antibody provided by the present disclosure is capable of effectively promoting the transformation of macrophages to the M1 phenotype and the secretion of high levels of the proinflammatory cytokine TNF-$\alpha$.

Illustratively, a humanized anti-ILT4 antibody provided by the present disclosure (e.g., huP2-G9) is capable of inducing dendritic cells to secrete higher levels of proinflammatory cytokines or chemokines in an immunosuppressive environment with IL-10.

Illustratively, a humanized anti-ILT4 antibody provided by the present disclosure is capable of effectively blocking the binding of ILT4 to HLA-G.

Illustratively, a humanized anti-ILT4 antibody provided by the present disclosure is capable of effectively blocking the binding of ILT4 to HLA-G, and the blocking activity of the humanized anti-ILT4 antibody is stronger than that of the control antibodies MK and JTX.

## Antibody Structure

In certain embodiments, the antibody provided herein is a full-length antibody.

In certain embodiments, the antibody provided herein is an antibody fragment.

In one embodiment, the antibody fragment is a Fab, Fab', Fab'-SH, or F(ab')$_2$ fragment, particularly a Fab fragment. "Fab" is a monovalent fragment consisting of the VL, VH, CL, and CH1 domains. "Fab fragment" may be produced by papain cleavage of an antibody. "Fab'" comprises VL, CL, and VH and CH1, and further comprises the region between the CH1 and CH2 domains, so that an interchain disulfide bond can be formed between the two heavy chains of two Fab' fragments to form a F(ab')2 molecule. "Fab'-SH" is a Fab' fragment in which the cysteine residues of the constant regions have a free sulfhydryl group. "F(ab')$_2$" is a bivalent fragment comprising two Fab fragments linked by a disulfide bond at the hinge region.

In another embodiment, the antibody fragment is a diabody, a triabody, or a tetrabody. Diabodies are antibody fragments having two antigen-binding sites. The fragment comprises a VH and a VL linked in the same polypeptide chain (VH-VL). By using a linker that is too short to allow pairing between two domains on the same chain, the domains are forced to pair with the complementary domains of another chain, thereby resulting in two antigen-binding sites. The two antigens may be identical or different.

In another embodiment, the antibody fragment is a single-chain Fab fragment. "Single-chain Fab fragment" or "scFab" is a polypeptide consisting of VH, CH1, VL, CL, and a linker, wherein the antibody domains and the linker have one of the following orders in the N-terminus to C-terminus direction: a) VH-CH1-linker-VL-CL, b) VL-CL-linker-VH-CH1, c) VH-CL-linker-VL-CH1, or d) VL-CH1-linker-VH-CL. In one embodiment, the linker is a polypeptide having at least 30 amino acids. In another embodiment, the linker is a polypeptide having between 32 and 50 amino acids. The single-chain Fab fragment is stabilized via the natural disulfide bond between CL and CH1. In addition, these single-chain Fab molecules may be further stabilized by generating interchain disulfide bonds through insertion of cysteine residues (e.g., position 44 in the heavy chain variable region and position 100 in the light chain variable region, according to Kabat numbering).

In another embodiment, the antibody fragment is an Fv fragment consisting of the VH and VL domains of a single arm of the antibody.

In another embodiment, the antibody fragment is a single-chain variable fragment (scFv). "scFv" is a fusion protein comprising at least one antibody fragment comprising a light chain variable region and at least one antibody fragment comprising a heavy chain variable region, wherein the light and heavy chain variable regions are linked by a short flexible peptide linker and are capable of being expressed as a single-chain polypeptide, and wherein the scFv retains the specificity of the intact antibody from which it is derived. Unless specifically indicated, an scFv may have VL and VH variable regions in any order herein; for example, with respect to the N-terminus and C-terminus of the polypeptide, the scFv may comprise VL-linker-VH or may comprise VH-linker-VL.

In another embodiment, the antibody fragment is a dsFv obtained by linking polypeptides in which one amino acid residue of each of VH and VL is substituted with a cysteine residue by a disulfide bond between the cysteine residues. The amino acid residues substituted with cysteine residues can be selected according to known methods (Protein Engineering, 7:697 (1994)) based on prediction of the three-dimensional structure of the antibody.

In another embodiment, the antibody fragment is a single-domain antibody (dAb). Single-domain antibodies are antibody fragments comprising all or a portion of the heavy chain variable domain or all or a portion of the light chain variable domain of an antibody.

In certain embodiments, the antibody provided herein is a chimeric antibody. In one example, a chimeric antibody comprises a non-human variable region (e.g., a variable region derived from a mouse, rat, hamster, domestic rabbit, or non-human primate, such as a monkey) and a human constant region. In another example, a chimeric antibody is a "class switched" antibody in which the class or subclass has been changed from the class or subclass of the parent antibody.

In certain embodiments, the antibody is a humanized antibody. Typically, non-human antibodies are humanized to reduce immunogenicity to humans while retaining the specificity and affinity of the parent non-human antibody. Generally, the humanized antibody comprises one or more variable regions in which the CDRs or portions thereof are derived from a non-human antibody and the FRs or portions thereof are derived from a human antibody. Optionally, the humanized antibody can further comprise a portion of a human constant region. In some embodiments, some FR residues in a humanized antibody may be replaced with corresponding residues from a non-human antibody (e.g., an antibody that provides CDR sequences).

Humanized antibodies and methods for their production are reviewed, e.g., in Almagro and Fransson, Front. Biosci. 13:1619-1633 (2008), and further described, e.g., in Riechmann et al., Nature 332:323-329 (1988); Queen et al., Proc. Nat'l Acad. Sci. USA 86:10029-10033 (1989); U.S. Patent Nos. 5,821,337, 7,527,791, 6,982,321, and 7,087,409; Kashmiri et al., Methods 36:25-34 (2005) (describing specificity determining region (SDR) grafting); Padlan, Mol. Immunol. 28:489-498 (1991) (describing "resurfuacing"); Dall' Acqua et al., Methods 36:43-60 (2005) (describing "FR shuffling"); and Osbourn et al., Methods 36:61-68 (2005) and Klimka et al., Br. J. Cancer 83:252-260 (2000) (describing the "guided selection" method for FR shuffling).

Human framework regions that can be used for humanization include, but are not limited to: framework regions selected using the "best-fit" method (see, e.g., Sims et al., J. Immunol. 151:2296 (1993)); framework regions derived from the consensus sequence of human antibodies of a particular subgroup of light or heavy chain variable regions (see, e.g., Carter et al., Proc. Natl. Acad. Sci. USA, 89:4285 (1992); and Presta et al., J. Immunol., 151:2623 (1993)); human mature (somatically mutated) framework regions or human germline framework regions (see, e.g., Almagro and Fransson, Front. Biosci. 13:1619-1633 (2008)); and framework regions obtained by screening FR libraries (see, e.g., Baca et al., J. Biol. Chem. 272:10678-10684 (1997) and Rosok et al., J. Biol. Chem. 271:22611-22618 (1996)).

### Variants of Anti-ILT4 Antibody

In certain embodiments, amino acid sequence variants of the anti-ILT4 antibody provided herein are encompassed. For example, it may be desirable to improve the binding affinity and/or other biological properties of the antibody. Amino acid sequence variants of an antibody can be prepared by introducing appropriate modifications into the nucleotide sequence encoding the antibody, or by peptide synthesis. Such modifications include, for example, deletions from, and/or insertions into and/or substitutions of residues within the amino acid sequence of the anti-ILT4 antibody. Any combination of deletion, insertion, and substitution can be made to obtain the final construct, provided that the final construct possesses the desired characteristics, e.g., antigen binding.

### Substitution, Insertion, and Deletion Variants

In certain embodiments, antibody variants having one or more amino acid substitutions are provided. Positions of interest for substitutional mutagenesis include CDRs and FRs. Conservative substitutions are shown in Table 2 under the heading of "Preferred substitution". More substantial changes are provided in Table 2 under the heading of "exemplary substitution", and as further described below with reference to amino acid side chain classes. Amino acid substitutions can be introduced into an antibody of interest and the products are screened for a desired activity, e.g., retained/improved antigen binding, reduced immunogenicity, or improved ADCC or CDC.

Table 2. Amino acid substitutions

| Original residue | Exemplary substitution | Preferred substitution |
|---|---|---|
| Ala(A) | Val, Leu, Ile | Val |
| Arg(R) | Lys, Gln, Asn | Lys |
| Asn(N) | Gln, His, Asp,Lys, Arg | Gln |
| Asp(D) | Glu, Asn | Glu |
| Cys(C) | Ser, Ala | Ser |
| Gln(Q) | Asn, Glu | Asn |
| Glu(E) | Asp, Gln | Asp |

(continued)

| Original residue | Exemplary substitution | Preferred substitution |
|---|---|---|
| Gly(G) | Ala | Ala |
| His(H) | Asn, Gln, Lys, Arg | Arg |
| Ile(I) | Leu; Val; Met; Ala; Phe; norleucine | Leu |
| Leu(L) | Norleucine; Ile; Val; Met; Ala; Phe | Ile |
| Lys(K) | Arg, Gln, Asn | Arg |
| Met(M) | Leu, Phe, Ile | Leu |
| Phe(F) | Trp, Leu, Val, Ile, Ala, Tyr | Tyr |
| Pro(P) | Ala | Ala |
| Ser(S) | Thr | Thr |
| Thr(T) | Ser | Ser |
| Trp(W) | Tyr, Phe | Tyr |
| Tyr(Y) | Trp, Phe, Thr, Ser | Phe |
| Val(V) | Ile; Leu; Met; Phe; Ala; norleucine | Leu |

According to common side-chain properties, amino acids can be grouped as follows:

(1) hydrophobic: norleucine, Met, Ala, Val, Leu, Ile;
(2) neutral, hydrophilic: Cys, Ser, Thr, Asn, Gln;
(3) acidic: Asp, Glu;
(4) basic: His, Lys, Arg;
(5) residues affecting chain orientation: Gly, Pro;
(6) aromatic: Trp, Tyr, Phe.

Non-conservative substitutions will involve substituting a member of one of these classes for a member of another class.

One type of substitutional variant involves substituting one or more CDR residues of a parent antibody (e.g., a humanized or human antibody). Generally, the resulting variant selected for further study will have changes (e.g., improvements) in certain biological properties (e.g., increased affinity or reduced immunogenicity) relative to the parent antibody, and/or will have substantially retained certain biological properties of the parent antibody. An exemplary substitutional variant is an affinity-matured antibody, which can be conveniently produced, for example, using phage display-based affinity maturation techniques such as those described herein. Briefly, one or more CDR residues are mutated and the variant antibodies are displayed on phages and screened for a particular biological activity (e.g. binding affinity). Changes (e.g., substitutions) can be made in CDRs, for example, to improve antibody affinity. Such changes can be made in CDR "hotspots", i.e., residues encoded by codons that undergo mutation at high frequency during the somatic maturation process, and/or antigen-contacting residues, and meanwhile, the resulting variant VH or VL is tested for binding affinity.

In some embodiments of affinity maturation, diversity is introduced into the variable genes selected for maturation by any one of a variety of methods (e.g., error-prone PCR, chain shuffling, or oligonucleotide-directed mutagenesis). A secondary library is then created. The library is then screened to identify any antibody variants with the desired affinity. Another method for introducing diversity involves CDR-directed methods in which several CDR residues (e.g., 4-6 residues at a time) are randomized. CDR residues involved in antigen binding can be specifically identified, for example, using alanine scanning mutagenesis or modeling. Particularly, HCDR3 and LCDR3 are often targeted.

In certain embodiments, substitutions, insertions or deletions may occur within one or more CDRs, as long as such changes do not substantially reduce the ability of the antibody to bind to the antigen. For example, conservative changes (e.g., conservative substitutions as provided herein) that do not substantially reduce binding affinity may be made in CDRs. Such changes may be, for example, outside of the antigen-contacting residues in CDRs. In certain embodiments of the variant VH and VL sequences provided above, each CDR is unaltered or contains no more than 1, 2, or 3 amino acid substitutions.

A useful method for identifying residues or regions of an antibody that can be used as mutagenesis targets is called "alanine scanning mutagenesis". In this method, a residue or group of target residues (e.g., charged residues such as Arg, Asp, His, Lys, and Glu) are identified and replaced with a neutral or negatively charged amino acid (e.g., Ala or polyalanine) to determine whether the interaction of the antibody with the antigen is affected. Further substitutions may be introduced at amino acid locations that show functional sensitivity to the initial substitutions. In addition, a crystal structure of an antigen-antibody complex may be studied to identify contact points between the antibody and antigen. These contact residues and neighboring residues may be targeted or eliminated as substitution candidates. Variants may be screened to determine whether they contain the desired properties.

Amino acid sequence insertions include amino- and/or carboxyl-terminal fusions ranging in length from 1 residue to polypeptides containing 100 or more residues, and intrasequence insertions of single or multiple amino acid residues. Examples of terminal insertions include an antibody with an N-terminal methionyl residue. Other insertional variants of the antibody molecule include the fusion of the N- or C-terminus of the antibody to an enzyme or a polypeptide that increases the serum half-life of the antibody.

## Recombination Method

Anti-ILT4 antibodies can be produced using recombinant methods. For these methods, one or more isolated nucleic acids encoding the anti-ILT4 antibodies are provided.

In one embodiment, the present disclosure provides an isolated nucleic acid encoding the aforementioned anti-ILT4 antibody. Such nucleic acids may each independently encode any one of the aforementioned polypeptide chains. In another aspect, the present disclosure provides one or more vectors (e.g., expression vectors) comprising such nucleic acids. In another aspect, the present disclosure provides a host cell comprising such nucleic acids. In one embodiment, provided is a method for preparing a polypeptide, wherein the method comprises culturing a host cell comprising a nucleic acid encoding the polypeptide, as provided above, under conditions suitable for expression, and optionally recovering the anti-ILT4 antibody from the host cell (or a host cell medium).

For recombinant production of the anti-ILT4 antibody, the nucleic acid encoding the protein is isolated and inserted into one or more vectors for further cloning and/or expression in a host cell. Such nucleic acids can be readily isolated and sequenced using conventional procedures, or produced by recombination methods or obtained by chemical synthesis.

Suitable host cells for cloning or expressing a vector encoding the anti-ILT4 antibody include prokaryotic or eukaryotic cells described herein. For example, it can be produced in bacteria, particularly when glycosylation and Fc effector functions are not needed. After expression, it can be isolated from the bacterial cell paste in a soluble fraction and can be further purified.

In addition to prokaryotes, eukaryotic microorganisms such as filamentous fungi or yeast are suitable cloning or expression hosts for vectors encoding proteins, including fungal and yeast strains. Suitable host cells for protein expression may also be derived from multicellular organisms (invertebrates and vertebrates); examples of invertebrate cells include plant and insect cells. A number of baculovirus strains have been identified, which can be used in combination with insect cells, particularly for transfection of *Spodoptera frugiperda* cells; plant cell cultures may also be used as hosts, see, e.g., US5959177, US 6040498, US6420548, US 7125978, and US6417429; vertebrate cells can also be used as hosts, e.g., mammalian cell lines adapted for growth in a suspension. Other examples of suitable mammalian host cell lines include an SV40-transformed monkey kidney CVIline (COS-7); a human embryonic kidney line (293 or 293T cells); baby hamster kidney cells (BHK); mouse sertoli cells (TM4 cells); monkey kidney cells (CV1); African green monkey kidney cells (VERO-76); human cervical cancer cells (HELA); canine kidney cells (MDCK); buffalo rat liver cells (BRL3A); human lung cells (W138); human liver cells (Hep G2); mouse mammary tumor cells (MMT 060562); TRI cells; MRC 5 cells; and FS4 cells. Other suitable mammalian host cell lines include Chinese hamster ovary (CHO) cells, including DHFR-CHO cells; and myeloma cell lines such as Y0, NS0, and Sp2/0. For reviews of certain mammalian host cell lines suitable for antibody production see, e.g., Yazaki, P. and Wu, A.M., Methods in Molecular Biology, Vol. 248, Lo, B.K.C. (eds.), Humana Press, Totowa, NJ (2004), pp. 255-268.

Assays

The physical/chemical characteristics and/or biological activity of the anti-ILT4 antibody provided herein may be identified, screened, or characterized through a variety of assays known in the art. In one aspect, the activity of the anti-ILT4 antibody of the present disclosure is tested, for example, by known methods such as ELISA and western blot.

## Treatment Method and Route of Administration

Any of the anti-ILT4 antibodies provided by the present disclosure can be used in a treatment method. In yet another aspect, the present disclosure provides use of the anti-ILT4 antibody in the manufacture or preparation of a medicament. In some embodiments, the disease is an ILT4-associated disease or disorder. In some embodiments, the disease is selected from the group consisting of astrocytoma (e.g., anaplastic astrocytoma), glioblastoma, bladder cancer, bone cancer, brain cancer, breast cancer (e.g., breast cancer characterized by BRCA1 and/or BRCA2 mutations), carcinoid, cervical cancer, choroid plexus papilloma, colorectal cancer (e.g., colon cancer and rectal cancer), fallopian tube cancer, ependymoma, gallbladder cancer, gastric cancer, head and neck cancer, idiopathic myelofibrosis, renal cancer or renal cell carcinoma (e.g., rhabdoid tumor of the kidney and Wilms' tumor), renal pelvis tumor, leukemia, liver cancer (e.g., hepatocellular carcinoma), esophageal cancer (e.g., also known as "oesophageal cancer", esophageal squamous cell carcinoma), lung cancer (e.g., non-small cell lung cancer and small cell lung cancer), lymphoma, medulloblastoma, melanoma, meningioma, Merkel cell carcinoma, mesothelioma, multiple myeloma, neuroblastoma, oligodendroglioma, ovarian cancer, peritoneal tumor, pancreatic cancer, polycythemia vera, primary neuroectodermal tumor, prostate cancer, retinoblastoma, salivary gland cancer, sarcoma (e.g., chondrosarcoma, Ewing sarcoma, osteosarcoma, rhabdomyosarcoma, synovial sarcoma, and soft tissue sarcoma), small intestine cancer, colorectal cancer, squamous cell carcinoma (e.g., cutaneous squamous cell carcinoma), thyroid cancer, endometrial cancer, vestibular schwannoma, blastoma, teratoma, pituitary cancer, vulva cancer, thymoma, testicular cancer, bile duct cancer, pheochromocytoma, paraganglioma or adenoid cystic carcinoma, and thrombocytosis; in some embodiments, the disease is selected from the group consisting of ovarian cancer, lung cancer, esophageal squamous cell carcinoma, colorectal cancer, renal cell carcinoma, and melanoma.

In yet another aspect, provided is a pharmaceutical composition comprising the anti-ILT4 antibody, e.g., for use in any one of the above pharmaceutical uses or treatment methods. In one embodiment, the pharmaceutical composition comprises any of the anti-ILT4 antibodies provided herein and a pharmaceutically acceptable carrier. In another embodiment, the pharmaceutical composition further comprises at least one additional therapeutic agent.

The anti-ILT4 antibody of the present disclosure may be used alone or in combination with other agents for treatment. For example, the antibody of the present disclosure can be co-administered with at least one additional therapeutic agent.

The anti-ILT4 antibody of the present disclosure (and any additional therapeutic agent) may be administered by any suitable means, including parenteral, intrapulmonary, and intranasal administration, and, if required for local treatment, intralesional administration. Parenteral infusion includes intramuscular, intravenous, intra-arterial, intraperitoneal, or subcutaneous administration. Administration may be performed by any suitable route, e.g., by injection, such as intravenous or subcutaneous injection, depending in part on whether the administration is short-term or long-term. Various administration schedules are contemplated herein, including but not limited to, single administration or multiple administrations at multiple time points, bolus administration, and pulse infusion.

The anti-ILT4 antibody of the present disclosure will be formulated, administered, and applied in a manner consistent with Good Medical Practice. Factors considered in this context include the specific disorder being treated, the specific mammal being treated, the clinical state of the individual patient, the cause of the disorder, the site of delivery of the agent, the method of administration, the timing of administration, and other factors known to medical practitioners. The anti-ILT4 antibody may be formulated with or without one or more agents currently used for preventing or treating the disorder. The effective amount of such additional agents depends on the amount present in the pharmaceutical composition, the type of the disorder or treatment, and other factors. These are generally used in the same dosages and routes of administration as described herein, or in about 1% to 99% of the dosages described herein, or in other dosages, and by any route empirically/clinically determined to be appropriate.

For the prevention or treatment of disease, the appropriate dosage of the anti-ILT4 antibody of the present disclosure (when used alone or in combination with one or more other additional therapeutic agents) will depend on the type of the disease to be treated, the type of the therapeutic molecule, the severity and course of the disease, whether the therapeutic molecule is administered for preventive or therapeutic purposes, previous therapy, the patient's clinical history and response to the therapeutic molecule, and the discretion of the attending physician. The therapeutic molecule is suitably administered to a patient in one or a series of treatments.

**Product**

In another aspect of the present disclosure, provided is a product (as an example, the product is prepared in the form of a kit) comprising materials useful for the treatment, prevention, and/or diagnosis of the disorders described above. The product comprises a container and a label or package insert on or associated with the container. Suitable containers include, for example, bottles, vials, syringes, IV solution bags, and the like. The container may be formed from a variety of materials such as glass or plastic. The container contains a composition effective in the treatment, prevention, and/or diagnosis of a disease, either alone or in combination with another composition, and may have a sterile access port (e.g., the container may be an intravenous solution bag or vial with a stopper pierceable by a hypodermic injection needle). At least one active agent in the composition is the anti-ILT4 antibody of the present disclosure. The label or package insert indicates that the composition is used to treat the selected condition. In addition, the product may comprise: (a) a first container containing a composition, wherein the composition comprises the anti-ILT4 antibody of the present disclosure; and (b) a second container containing a composition, wherein the composition comprises an additional cytotoxic agent or a therapeutic agent of other aspects. The product in this embodiment of the present disclosure may further comprise a package insert indicating that the composition may be used to treat a particular condition. Alternatively or additionally, the product may further comprise a second (or third) container containing a pharmaceutically acceptable buffer. From a commercial and user standpoint, it may further contain other materials as required, including other buffers, diluents, filters, needles, and syringes.

## Examples

The present disclosure is further described below with reference to the following examples and test examples, which, however, do not limit the present disclosure. The experimental methods in the examples and test examples of the present disclosure in which specific conditions are not specified are generally performed under conventional conditions such as Antibodies: A Laboratory Manual and Molecular Cloning: A Laboratory Manual by Cold Spring Harbor Laboratory, or under conditions recommended by the manufacturers of the starting materials or commercial products. Reagents without specific origins indicated were commercially available conventional reagents.

## Example 1: Preparation of ILT4 Antigens

With UniProt LIRB2_HUMAN (human LILRB2 protein, Uniprot number: Q8N423) as a template of ILT4, the amino acid sequences of the antigens and assay proteins used in the present disclosure were designed; optionally, ILT4 protein was fused with different tags such as a His tag or an Fc. After cloning into a pTT5 vector (Biovector, CAT#102762), 293 cells were transiently transfected with the vector. After expression and purification, immunization and assay antigens of the present disclosure were obtained.

1. Human-IgG1-Fc-tagged ILT4 protein extracellular domain: ILT4-Fc, as an immunization antigen.

*QTGTIPKPTLWAEPDSVITQGSPVTLSCQGSLEAQEYRLYREKKSASWITRIRPELVK*
*NGQFHIPSITWEHTGRYGCQYYSRARWSELSDPLVLVMTGAYPKPTLSAQPSPVVTS*
*GGRVTLQCESQVAFGGFILCKEGEEEHPQCLNSQPHARGSSRAIFSVGPVSPNRR*
*WSHRCYGYDLNSPYVWSSPSDLLELLVPGVSKKPSLSVQPGPVVAPGESLTLQCVS*
*DVGYDRFVLYKEGERDLRQLPGRQPQAGLSQANFTLGPVSRSYGGQYRCYGAHN*
*LSSECSAPSDPLDILITGQIRGTPFISVQPGPTVASGENVTLLCQSWRQFHTFLLTKA*
*GAADAPLRLRSIHEYPKYQAEFPMSPVTSAHAGTYRCYGSLNSDPYLLSHPSEPLEL*
*VVSGPSMGSSPPPTGPISTPAGPEDQPLTPTGSDPQSGLGRHLGV*<u>EPKSSDKTHTC</u>
<u>PPCPAPELLGGPSVFLFPPKPKDTLMISRTPEVTCVVVDVSHEDPEVKFNWYV</u>
<u>DGVEVHNAKTKPREEQYNSTYRVVSVLTVLHQDWLNGKEYKCKVSNKALPA</u>
<u>PIEKTISKAKGQPREPQVYTLPPSRDELTKNQVSLTCLVKGFYPSDIAVEWESN</u>
<u>GQPENNYKTTPPVLDSDGSFFLYSKLTVDKSRWQQGNVFSCSVMHEALHNHY</u>
<u>TQKSLSLSPGK</u>

(SEQ ID NO: 1)

Note: The extracellular domain is italicized, and the human-IgG1-Fc is single underlined.

2. His-tagged ILT4 protein extracellular domain: ILT4-His, as an assay antigen.

*QTGTIPKPTLWAEPDSVITQGSPVTLSCQGSLEAQEYRLYREKKSASWITRIRPELVK*

*NGQFHIPSITWEHTGRYGCQYYSRARWSELSDPLVLVMTGAYPKPTLSAQPSPVVTS*

*GGRVTLQCESQVAFGGFILCKEGEEEHPQCLNSQPHARGSSRAIFSVGPVSPNRR*

*WSHRCYGYDLNSPYVWSSPSDLLELLVPGVSKKPSLSVQPGPVVAPGESLTLQCVS*

*DVGYDRFVLYKEGERDLRQLPGRQPQAGLSQANFTLGPVSRSYGGQYRCYGAHN*

*LSSECSAPSDPLDILITGQIRGTPFISVQPGPTVASGENVTLLCQSWRQFHTFLLTKA*

*GAADAPLRLRSIHEYPKYQAEFPMSPVTSAHAGTYRCYGSLNSDPYLLSHPSEPLEL*

*VVSGPSMGSSPPPTGPISTPAGPEDQPLTPTGSDPQSGLGRHLGV*<u>HHHHHH</u>

(SEQ ID NO: 2)

Note: The extracellular domain is italicized, and the His tag is single underlined. CHO-S cells (CHO-S/ILT4) overexpressing full-length ILT4 protein were used as assay cells.

<u>MTPIVTVLICLGLSLGPRTHV</u>*QTGTIPKPTLWAEPDSVITQGSPVTLSCQGSLEAQ*

*EYRLYREKKSASWITRIRPELVKNGQFHIPSITWEHTGRYGCQYYSRARWSELSDPL*

*VLVMTGAYPKPTLSAQPSPVVTSGGRVTLQCESQVAFGGFILCKEGEEEHPQCLNS*

*QPHARGSSRAIFSVGPVSPNRRWSHRCYGYDLNSPYVWSSPSDLLELLVPGVSKKPS*

*LSVQPGPVVAPGESLTLQCVSDVGYDRFVLYKEGERDLRQLPGRQPQAGLSQANF*

*TLGPVSRSYGGQYRCYGAHNLSSECSAPSDPLDILITGQIRGTPFISVQPGPTVASGE*

*NVTLLCQSWRQFHTFLLTKAGAADAPLRLRSIHEYPKYQAEFPMSPVTSAHAGTYR*

*CYGSLNSDPYLLSHPSEPLELVVSGPSMGSSPPPTGPISTPAGPEDQPLTPTGSDPQ*

*SGLGRHLGV*<u>VIGILVAVVLLLLLLLLLFLILR</u><u>HRRQGKHWTSTQRKADFQHPAG</u>

<u>AVGPEPTDRGLQWRSSPAADAQEENLYAAVKDTQPEDGVEMDTRAAASEAP</u>

<u>QDVTYAQLHSLTLRRKATEPPPSQEREPPAEPSIYATLAIH</u>

(SEQ ID NO: 3)

Note: The signal peptide is single underlined, the extracellular domain is italicized, the transmembrane domain is dotted underlined, and the intracellular domain is double underlined.

**Example 2: Purification of ILT4-Associated Recombinant Proteins**

1. Purification of His-tagged recombinant protein

The cell expression supernatant sample was centrifuged at high speed to remove impurities, the buffer was exchanged for PBS, and imidazole was added until the final concentration was 5 mM. A nickel column was equilibrated with a PBS solution containing 5 mM imidazole and washed with 2-5 column volumes. The cell supernatant sample after exchange was loaded on the Ni Sepharose excel column (GE, 17-3712-02). The column was washed with a PBS solution containing 5 mM imidazole until the $A_{280}$ reading dropped to the baseline. The chromatography column was then washed with PBS + 10 mM imidazole to remove non-specifically bound protein impurities, and the eluate was collected. The target protein was eluted with a PBS solution containing 300 mM imidazole, and the eluted peak was collected. The collected eluate was concentrated and further purified using a gel chromatography column Superdex200 (GE, 28-9893-35) with PBS as the mobile phase. The polymer peaks were removed, and the eluted peak was collected. The obtained protein was verified by electrophoresis, peptide mapping, and LC-MS and then aliquoted for later use. ILT4-His, which contains a His tag, was obtained for use as an assay antigen for the antibodies of the present disclosure.

2. Purification of Fc-tagged recombinant proteins or antibodies

The cell expression supernatant sample was centrifuged at high speed to remove impurities, and the supernatant was purified by MabSelect Sure (GE, 17-5438-01) affinity chromatography. The MabSelect Sure chromatography column was regenerated first with 0.2 M NaOH, then washed with purified water, and then equilibrated with PBS. After the supernatant was bound, the column was washed with PBS until the $A_{280}$ reading dropped to the baseline. The target protein was eluted with 0.1 M acetic acid buffer at pH 3.5 and neutralized with 1 M Tris-HCl. The eluted sample was appropriately concentrated and further purified using a gel chromatography column Superdex200 (GE, 28-9893-35) equilibrated with PBS, and the target protein was concentrated to an appropriate concentration in the receiver tube where it was collected. This method was used to purify ILT4-Fc fusion proteins, and it can also be used to purify the related humanized antibody proteins in the present disclosure.

## Example 3: Obtaining of Anti-Human ILT4 Antibodies

1. Mouse immunization

Anti-human ILT4 monoclonal antibodies were produced by immunization of mice. Laboratory SJL white mice, female, aged 6-8 weeks (Shanghai SLAC Laboratory Animal Co., Ltd., animal production license number: SCXK (Shanghai) 2017-0005). Housing environment: SPF. The purchased mice were housed in a laboratory environment for 1 week, with a 12/12-hour light/dark cycle, at a temperature of 20-25 °C with humidity at 40-60%. The acclimatized mice were immunized according to the following scheme.

The mice were immunized with the ILT4-Fc protein. Cross-immunization was performed using the adjuvants TiterMax® Gold Adjuvant (Sigma Cat No. T2684) and Thermo Imject® Alum (Thermo Cat No. 77161). The ratio of the antigen to the adjuvant TiterMax® Gold Adjuvant was 1:1, and the ratio of the antigen to the adjuvant Thermo Imject® Alum was 3:1. The dose for the first immunization was 50 μg/animal/immunization, and the dose for boost immunization was 25 μg/animal/immunization. The antigen was emulsified before inoculation. Immunization time points were 0, 14, and 34 days. Blood samples were collected on days 25 and 41, and the antibody titer in the mouse serum was determined. After the third immunization, mice in which the serum antibody titer was high and tended to plateau were selected for single B-cell screening. Boost immunization was performed 3 days before screening. Each animal was intraperitoneally (i.p.) injected with 25 μg of an antigen solution prepared in normal saline.

2. Single B-cell screening

Single B-cell screening primarily involved using forward and reverse screening, 10*genomics, and bioinformatics technologies to obtain a series of antibody variable region sequences. The obtained sequences were categorized based on their CDRs. Their murine variable region sequences were selected and linked with human antibody constant region sequences, and chimeric antibodies were expressed. These antibodies were screened through methods such as binding to ILT4 protein, cell binding, and ILT4/HLA-G blocking to obtain antibody sequences that specifically bind to ILT4. The heavy and light chain variable region sequences of the obtained antibodies are shown below:

> M P1-B4 HCVR (SEQ ID NO: 4)

QVQLKQSGPGLVQPSQSLSITCTVSSFSLTNYGVHWVRQSPGKGLEWLGVIW
TGGSTDYNAAFISRLSIIRDNSKSQVFFKMNSLQADDTAMYYCAITTVVTAMD
YWGQGTSVTVSS

> M P1-B4 LCVR (SEQ ID NO: 5)

DIVMTQSHKFMSTSVGDRVSITCKASQDVNIAVVWYQQKPGQSPKLLIYAASY
RYTGVPDRFTGSGSGTDFTFTISSVQAEDLAVYYCQQHYSSPYTFGGGTKLEI
K

> M P1-B9 HCVR (SEQ ID NO: 6)

QVQLKQSGPGLVQPSQSLSITCTVSGFSLTSYGVHWVRQSPGKGLEWLGVIWS
GGNTDYNAAFMSRLRINKDNSKSQVFFKMNSLQGSDTAIYYCATPYDVVPFA
YWGQGTLVTVSS

> M P1-B9 LCVR (SEQ ID NO: 7)

DIVMTQSHKFMSTSVGDRVSITCKASQDVNIAVAWYQQKPGLSPKLLIYSASY
RYTGVPGRFTGSGSGTDFTFTISSVKAEDLAVYYCQQHYSIPFTFGAGTKLEIK

> M P1-D10 HCVR (SEQ ID NO: 8)

QVQLKQSGPGLVQPSQSLSITCTVSGFSLTTYGVHWVRQSPGKGLEWLGVIW
SGGNTDSNAAFISRLSISKDNSKSQVFFKMNNLQADDTAIYYCARYSYAMDY
WGQGTSVTVSS

> M P1-D10 LCVR (SEQ ID NO: 9)

DIVMTQSHKFMSTSVGDRVSITCKASQDVSNGVAWYQQKPGQSPKVLIYAAS
YRYTGVPDRFTGSGSGTDFTFTIGSVQAEDLALYYCQQHYSTPFTFGSGTMLEI
K

> M P2-E8 HCVR (SEQ ID NO: 10)

EVQLLESGGGLVKPGGSLKLSCAASGFTFSDYGIHWVRQAPEKGLEWVAYISS
GSSIINYADTVKGRFTISRDNAKNTLLLQMTSLRSEDTAMYYCARGPNYYGSS
YLLVYWGQGTLVTVSS

> M P2-E8 LCVR (SEQ ID NO: 11)

DIVMTQSHKFMSTSVGDRVSITCKASQDVNIAVAWYQQRPGQSPKLLIYSASY
RYTGVPDRFTGSGSGTDFTFTISSVQAEDLAVYYCQQHYNIPWTFGGGTKLEI
K

> M P2-C10 HCVR (SEQ ID NO: 12)

QVQLQQPGAELVKPGASVKLSCKASGYTFTSYWMHWVKQRPGQGLEWIGVI
HPNSDTTNYNEKFKNKATLTVDKSSSTAYMQLSSLTSEDSAVYYCATRYDYAY
YAMDYWGQGTSVTVSS

> M P2-C10 LCVR (SEQ ID NO: 13)

DIVMTQSHKFMSTSVGDRVSITCKASQDVTTAVAWYQQKPGQSPKLLIYSASY
RYTGVPDRFTGSGSGTDFTFTISSVQAEDLAVYHCQQHYSTSPFTFGGGTMLEI
K

> M P2-F4 HCVR (SEQ ID NO: 14)

EVQLVESGGGLVKPGGSLKLSCAASGFTFSDYGMHWVRQAPEKGLEWVAYIS
SGSRIIYYADTVKGRFTISRDNAKNTLFLQMTSLRSEDTAMYSCARFYNYAHY
TMDYWGQGTSVTVSS

> M P2-F4 LCVR (SEQ ID NO: 15)

DIVMTQSHKFMSTSVGDRVSITCKASQDVSTAVAWYQQKPGQSPKLLIYSASY
RYTGVPDRFTGSGSGTDFTFTISSVQAEDLTLYYCQHHYSTPPTFGGGTKLEIK

> M P2-B3 HCVR (SEQ ID NO: 16)

QVQLKQSGPGLVQPSQSLSITCTVSGFSLTNYGVHWFRQSPGKGLEWLGMIW
SGGNTDYNAAFTSRLIITKDNSKSQVFFKMNSMQADDTAIYYCARGDYGSSS
AYWGQGTLVTVSS

> M P2-B3 LCVR (SEQ ID NO: 17)

DIVMTQSHKFMSTSVGDRVSITCKASQDVSIAVAWYQQKPGQSPKLLIYSASY
RYTGVPDRFTGSGSGTDFTFTISSVQAEDLAVYYCQQHYSNTLTFGAGTKLEI
K

> M P2-E7 HCVR (SEQ ID NO: 18)

QVQLQQPGTELVKPGASVKLSCKASGYTFTSYWMHWVKQSPGQGLEWIGNI
NPSNGGPNYNENFKTKATLTVDKSSSTAYMQLNSLTSEDSAVYYCAGGDYYG
GSYLFAYWGQGTLVTVSS

> M P2-E7 LCVR (SEQ ID NO: 19)

DTVMTQSHKFMSTSVGDRVSVTCKASQDVNIAVAWYQQKPGQSPKLLIYSSS
YRYTGVPDRFTGSGSETDFTFTISSVQAEDLAVYYCQQHYTFPYTFGGGTKLEI
K

> M P2-G4 HCVR (SEQ ID NO: 20)

EVQLQQSGPELVKPGASVKMSCKASGYTFTDYNMHWVKQSHGKSLEWIGYI
NPNNGGTTYNQKFKGKATLTVNKSSSTAYMELRSLTSEDSAVYYCASSEYYGS
TFLFAYWGQGTLVTVSS

> M P2-G4 LCVR (SEQ ID NO: 21)

DIVMTQSHKFMSTSVGDRVGITCKASQDVSIAVAWYQQKPGQSPKLLIYSASY
RYTGVPDRFTGSGSGTDFTFTISSVQAEDLAAYYCQQHYSTPWTFGGGTKLEI
K

> M P2-C4 HCVR (SEQ ID NO: 22)

QVQLKESGPGLVAPSQSLSITCTVSGFSLSTYAIGWVRQPPGKGLEWLGVIWT
GGGTSYNSALKSRLSISKDNSKSQVFLKMNSLQTDDTARYYCARGDYGPFAY
WGQGTLVTVSS

> M P2-C4 LCVR (SEQ ID NO: 23)

DIVMTQSHKFMSTSLGDRVSISCKASQDVSIAVAWYQQKAGQSPKLLIYSASY
RHTGVPDRFTGSGSGTDFTFTISSVQAEDLAVYYCQQHYSTPFTFGSGTKLEIK

> M P2-E11 HCVR (SEQ ID NO: 24)

QVQLQQSGPELVKPGASVKISCKASAYTFTDYYINWVKQRPGQGLEWIGWIF
PGSGSTYYNEKFKGKATLTVDKSSSTAYMLVSSLTSEDSAVYFCARSGTLVAPY
AMDYWGQGTSVTVSS

> M P2-E11 LCVR (SEQ ID NO: 25)

SIVMTQTPKFLLVSAGDRVTITCKASQSVSNDVAWYQQKPGQSPKLLIYYTSN
RYTGVPDRFTGSGYGTDFTFTISTVQAEDLAVYFCQQDYSFPLTFGAGTKLEIK

> M P1-F7 HCVR (SEQ ID NO: 26)

DVQLQESGPGLVKPSQSLSLTCSVTGYSITSGYYWNWIRQFPGNKLEWMGYIS
YDGNNYYNPSLKNRISVTRDTSKNQFFLKLNSVTSEDTATYYCAREDGSYSD
YDGFAYWGQGTLVTVSS

> M P1-F7 LCVR (SEQ ID NO: 27)

DIQMTQSPASLSVSVGETVTITCRANENIYSNLAWYQQKQGKSPQLLVYAATN
LADGVSSRFSGSGSGTQYSLRINSLQSEDFGSYYCQQFWGTPYTFGGGTKLEI
K

> M P2-E3 HCVR (SEQ ID NO: 28)

EVQLQQSGPELVKPGASVKISCKASGYTFTDYYMNWVKQSHGKSLEWIGDIN
PNNGGTSYNQKFKGKATLTVDKSSSTAYMELRSLTSEDSTVYYCARGGVTTV
VATYWYFDVWGTGTTVTVSS

> M P2-E3 LCVR (SEQ ID NO: 29)

DIQMTQSPASLSVSVGETVTITCRASENIYSNLAWYQQKQGKSPQLLVYAATN
VADGVPSRFSGGGSGTQYSLKINSLQSEDFGSYYCQQFWDTPFTFGSGTKLEI
K

> M P2-F10 HCVR (SEQ ID NO: 30)

DVQLQESGPGLVKPSQSLSLTCSVTGYSIASGYYWNWIRQFPGNKLEWMGYITYDGSNNYNPSLKNRISITRDTSKNQFFLKLNSVTTEDTATYYCAREYSYGSTLSWFAYWGQGTLVTVSS

> M P2-F10 LCVR (SEQ ID NO: 31)

DIQMTQSPASLSISVGETVTITCRASENIYSNLAWYQQKQGKSPQLLVYAATNLADGVPSRFSGSGSGTQYSLKINSLQSEDFGSYYCQHFWDTPYTFGGGTKLEIK

> M P2-G9 HCVR (SEQ ID NO: 32)

DVQLQESGPGLVKPSQSLSLACSVTGSSITSGYYWNWIRQFPGNKLEWMGYLSYDGSNNYNPSLKNRISITRDTSKSQFFLKLNSVTPEDTATYYCAREGAHYSGTLSWFAYWGQGTLVTVSS

> M P2-G9 LCVR (SEQ ID NO: 33)

DIQMTQSPASLSVSVGETVTITCRASENIYSNLAWYQQKQGKSPQLLVYAATNLADGVPSRFSGSGSGTQYSLKINSLQSEDFGSYYCQHFWDTPYTFGGGTKLEIK

> M P1-C4 HCVR (SEQ ID NO: 34)

QVQLKQSGAELVRPGASVKLSCKASGYSFTDYYINWVKQRPGQGLEWIARIYPGNDNTYYNEKFKDKATLTADSSSSTAYMQLSSLTSEDSAVYFCTRSQDGYYEENAMDYWGQGTSVTVSS

> M P1-C4 LCVR (SEQ ID NO: 35)

DIQMTQSPASLSVSVGETVTITCRASENIYSNLAWYQQRQGKSPQLLVYAATNLADGVPFRFSGSGSGTQYSLKINSLQSEDFGSYYCQQFWGSPHTFGGGTKLEIK

> M P1-G7 HCVR (SEQ ID NO: 36)

DVQLQESGPGLVKPSQSLSLTCSVTGYSITSGYYWNWIRQFPENKLEWMGYISYDGGNNYNPFLKNRISITRDTSKNQFFLNLNSVTIEDTATYYCAREGGSSWEYYGMDYWGQGTSVTVSS

> M P1-G7 LCVR (SEQ ID NO: 37)

DIQMTQSPASLSVSVGETVTITCRASENIYRNLAWYQQKQGHSPQLLLYAATNLADGVPSRFSGSGSGTQYSLKINSLQSEDFGSYYCQQFWGSPYTFGGGTKLEIK

> M P2-C7 HCVR (SEQ ID NO: 38)

EVQLQQSGPELVKPGTSVKMSCKASGYTFTDYSMHWVRQSHGKSLEWIGYF
NPNNGDTIYNQKFKGKATLTVNKSSSTAYMELRSLTSDDSAVYYCAREDSVST
NAWDGALDYWGQGTSVTVSS

> M P2-C7 LCVR (SEQ ID NO: 39)

DIQMTQSPASLSVSVGETVTITCRTSENIYSNLAWYQQKQGKSPQLLVYGATN
LADGVPSRFSGSGSGTQYSLKINSLQSEDFGSYYCQHFWDIPWTFGGGTTLEI
K

> M P2-C6 HCVR (SEQ ID NO: 40)

EVQLQQSGPELVKPGASVKMSCEASGYTFTDYYIHWVKQSHGESLEWIGYIY
PDNGYNGYNQKFKGKATLTVDKSSNTAYMELRSLTSEDSAVYFCTRRGDYSG
VHFDYWGQGTALTVSS

> M P2-C6 LCVR (SEQ ID NO: 41)

DIVLTQSPASLAVSLGQRATISCRASESVNNYGVGFLHWYQQKPGQPPKLLIYR
ASTLESGIPARFSGSGSRTDFTLTINPVETDDVATYYCQHSYKDPPWTFGGGTK
LEIK

> M P1-D7 HCVR (SEQ ID NO: 42)

QIQLVQSGPDLKKPGETVKISCKASGYTFTTYGMNWVKQAPGKALKWMGWI
NTYSGVPTYADDFKGRFAFSLETSATTAYLQINNLKNEDTATYFCARRTDYDG
YVMDCWGQGTSVTVSS

> M P1-D7 LCVR (SEQ ID NO: 43)

DIVMSQSPSSLAVSIGEKVTMSCKSSQSLFYSGNQKNYLAWYLQKPGQSPKLL
LYWASTRESGVPDRFTGSGSGTDFTLTISSVMAEDLAVYYCQQYFSYPPTFGG
GTKLEIK

> M P1-E11 HCVR (SEQ ID NO: 44)

EVQLQQSGPELVKPGASVKMSCQASGYTFTDYSIHWVKQSRGASLEWIGYIN
PNNGVTNYNQKFKGKATLTVNKSSSTAYMELRSLTSEDSAVYYCAREGDSVT
TIVVHWYFDVGGTGTTVTVSS

> M P1-E11 LCVR (SEQ ID NO: 45)

DIQMTQSPASLSVSLGETVTITCRASENIYSNLVWYQQKQGKFPQLLVYAAINL
VDGVPSRFSGSGSGTQYSLKINSLQSEDFGSYYCQQFWDTPLTFGAGTKLEIK

> M P2-B9 HCVR (SEQ ID NO: 46)

EVQLQQSGPELVKPGASVKISCKASGYTFTDYFMNWVKLSHGKSLEWIGDIN
PNNGGTSYNQRFKDKVTLTVDKSSSTAYMELRSLTSDSSAVYFCARNGYYDS
NYLSYFDVWGTGTTVTVSS

> M P2-B9 LCVR (SEQ ID NO: 47)

DIQMTQSPASLSVSVGETVTITCRASENIYSNLAWYQQKQGKSPQLLVYGATN
LADGVPSRFSGSGSGTQYSLKINSLQSEDFGSYYCQHFWDTPYTFGGGTKLEI
K

> M P2-C2 HCVR (SEQ ID NO: 48)

QVQLKQSGPGLVQPSQSLSITCTVSGFSLTSYGVHWVRQSPGKGLEWLGVIWS
GGHTDLNAAFISRLSINKDNSKSQVFFKMNSLQGDDTAIYYCARNGYGGCLD
YWGQGTTLTVSS

> M P2-C2 LCVR (SEQ ID NO: 49)

DIVMTQSHKFMSTSVGDRVSITCKAGQDVSIAVAWYQQKPGQSPQLLIYSASY
RYTGVPDRFTGSGSGTDFTFAISSVQAEDLAVYYCQQYYSIPPSFGAGTKLEIK

> M P2-E10 HCVR (SEQ ID NO: 50)

DVQLQESGPGLVKPSQSLSLTCSVTGYSITSGYYWNWIRQFPGNKLEWMGYM
SYEGTNNSNPSLKNRISITRDTSKNQFFLKLNSVTTEDTARYYCARERAYYYG
TLGAMDYWGQGTSVTVSS

> M P2-E10 LCVR (SEQ ID NO: 51)

DIQMTQSPASLSVSVGETVTITCRASENIYSNLAWYQQKQGKSPQLLVYAATN
LADGVPSRFSGSGSGTQYSLKINSMQSEDFGSYYCQHFWDTPFTFGSGTKLEI
K

Note: The variable regions are single underlined, and the CDRs are double underlined; the CDRs are defined according to the Kabat numbering scheme.

The heavy and light chain CDR sequences of the murine antibodies are shown in Table 3 below.

Table 3. The heavy and light chain CDR sequences of the antibodies

| Antibody | Heavy chain | | Light chain | | |
|---|---|---|---|---|---|
| M P1-B4 | HCDR1 | NYGVH (SEQ ID NO: 52) | LCDR1 | KASQDVNIAVV (SEQ ID NO: 55) |
| | HCDR2 | VIWTGGSTDYNAAFIS (SEQ ID NO: 53) | LCDR2 | AASYRYT (SEQ ID NO: 56) |
| | HCDR3 | TTVVTAMDY (SEQ ID NO: 54) | LCDR3 | QQHYSSPYT (SEQ ID NO: 57) |

(continued)

| Antibody | Heavy chain | | Light chain | |
|---|---|---|---|---|
| M P1-B9 | HCDR1 | SYGVH (SEQ ID NO: 58) | LCDR1 | KASQDVNIAVA (SEQ ID NO: 61) |
| | HCDR2 | VIWSGGNTDYNAAFMS (SEQ ID NO: 59) | LCDR2 | SASYRYT (SEQ ID NO: 62) |
| | HCDR3 | PYDVVPFAY (SEQ ID NO: 60) | LCDR3 | QQHYSIPFT (SEQ ID NO: 63) |
| MP1-D10 | HCDR1 | TYGVH (SEQ ID NO: 64) | LCDR1 | KASQDVSNGVA (SEQ ID NO: 67) |
| | HCDR2 | VIWSGGNTDSNAAFIS (SEQ ID NO: 65) | LCDR2 | AASYRYT (SEQ ID NO:56) |
| | HCDR3 | YSYAMDY (SEQ ID NO: 66) | LCDR3 | QQHYSTPFT (SEQ ID NO: 68) |
| M P2-E8 | HCDR1 | DYGIH (SEQ ID NO: 69) | LCDR1 | KASQDVNIAVA (SEQ ID NO:61) |
| | HCDR2 | YISSGSSIINYADTVKG (SEQ ID NO: 70) | LCDR2 | SASYRYT (SEQ ID NO:62) |
| | HCDR3 | GPNYYGSSYLLVY (SEQ ID NO: 71) | LCDR3 | QQHYNIPWT (SEQ ID NO: 72) |
| M P2-C10 | HCDR1 | SYWMH (SEQ ID NO: 73) | LCDR1 | KASQDVTTAVA (SEQ ID NO: 76) |
| | HCDR2 | VIHPNSDTTNYNEKFKN (SEQ ID NO: 74) | LCDR2 | SASYRYT (SEQ ID NO:62) |
| | HCDR3 | RYDYAYYAMDY (SEQ ID NO: 75) | LCDR3 | QQHYSTSPT (SEQ ID NO: 77) |
| M P2-F4 | HCDR1 | DYGMH (SEQ ID NO: 78) | LCDR1 | KASQDVSTAVA (SEQ ID NO: 81) |
| | HCDR2 | YISSGSRIIYYADTVKG (SEQ ID NO: 79) | LCDR2 | SASYRYT (SEQ ID NO:62) |
| | HCDR3 | FYNYAHYTMDY (SEQ ID NO: 80) | LCDR3 | QHHYSTPPT (SEQ ID NO: 82) |
| M P2-B3 | HCDR1 | NYGVH (SEQ ID NO: 52) | LCDR1 | KASQDVSIAVA (SEQ ID NO: 85) |
| | HCDR2 | MIWSGGNTDYNAAFTS (SEQ ID NO: 83) | LCDR2 | SASYRYT (SEQ ID NO:62) |
| | HCDR3 | GDYGSSSAY (SEQ ID NO: 84) | LCDR3 | QQHYSNTLT (SEQ ID NO: 86) |
| M P2-E7 | HCDR1 | SYWMH (SEQ ID NO: 73) | LCDR1 | KASQDVNIAVA (SEQ ID NO: 61) |
| | HCDR2 | NINPSNGGPNYNENFKT (SEQ ID NO: 87) | LCDR2 | SSSYRYT (SEQ ID NO: 89) |
| | HCDR3 | GDYYGGSYLFAY (SEQ ID NO: 88) | LCDR3 | QQHYTFPYT (SEQ ID NO: 90) |
| M P2-G4 | HCDR1 | DYNMH (SEQ ID NO: 91) | LCDR1 | KASQDVSIAVA (SEQ ID NO: 85) |
| | HCDR2 | YINPNNGGTTYNQKFKG (SEQ ID NO: 92) | LCDR2 | SASYRYT (SEQ ID NO:62) |
| | HCDR3 | SEYYGSTFLFAY (SEQ ID NO: 93) | LCDR3 | QQHYSTPWT (SEQ ID NO: 94) |
| M P2-C4 | HCDR1 | TYAIG (SEQ ID NO: 95) | LCDR1 | KASQDVSIAVA (SEQ ID NO: 85) |
| | HCDR2 | VIWTGGGTSYNSALKS (SEQ ID NO: 96) | LCDR2 | SASYRHT (SEQ ID NO: 98) |
| | HCDR3 | GDYGPFAY (SEQ ID NO: 97) | LCDR3 | QQHYSTPFT (SEQ ID NO:68) |

(continued)

| Antibody | Heavy chain | | Light chain | |
|---|---|---|---|---|
| M P2-E11 | HCDR1 | DYYIN (SEQ ID NO: 99) | LCDR1 | KASQSVSNDVA (SEQ ID NO: 102) |
| | HCDR2 | WIFPGSGSTYYNEKFKG (SEQ ID NO: 100) | LCDR2 | YTSNRYT (SEQ ID NO: 103) |
| | HCDR3 | SGTLVAPYAMDY (SEQ ID NO: 101) | LCDR3 | QQDYSFPLT (SEQ ID NO: 104) |
| M P1-F7 | HCDR1 | SGYYWN (SEQ ID NO: 105) | LCDR1 | RANENIYSNLA (SEQ ID NO: 108) |
| | HCDR2 | YISYDGNNYYNPSLKN (SEQ ID NO: 106) | LCDR2 | AATNLAD (SEQ ID NO: 109) |
| | HCDR3 | EDGSYSDYDGFAY (SEQ ID NO: 107) | LCDR3 | QQFWGTPYT (SEQ ID NO: 110) |
| M P2-E3 | HCDR1 | DYYMN (SEQ ID NO: 111) | LCDR1 | RASENIYSNLA (SEQ ID NO: 114) |
| | HCDR2 | DINPNNGGTSYNQKFKG (SEQ ID NO: 112) | LCDR2 | AATNVAD (SEQ ID NO: 115) |
| | HCDR3 | GGVTTVVATYWYFDV (SEQ ID NO: 113) | LCDR3 | QQFWDTPFT (SEQ ID NO: 116) |
| M P2-F10 | HCDR1 | SGYYWN (SEQ ID NO: 105) | LCDR1 | RASENIYSNLA (SEQ ID NO: 114) |
| | HCDR2 | YITYDGSNNYNPSLKN (SEQ ID NO: 117) | LCDR2 | AATNLAD (SEQ ID NO: 109) |
| | HCDR3 | EYSYGSTLSWFAY (SEQ ID NO: 118) | LCDR3 | QHFWDTPYT (SEQ ID NO: 119) |
| M P2-G9 | HCDR1 | SGYYWN (SEQ ID NO: 105) | LCDR1 | RASENIYSNLA (SEQ ID NO: 114) |
| | HCDR2 | YLSYDGSNNYNPSLKN (SEQ ID NO: 120) | LCDR2 | AATNLAD (SEQ ID NO: 109) |
| | HCDR3 | EGAHYSGTLSWFAY (SEQ ID NO: 121) | LCDR3 | QHFWDTPYT (SEQ ID NO: 119) |
| M P1-C4 | HCDR1 | DYYIN (SEQ ID NO: 99) | LCDR1 | RASENIYSNLA (SEQ ID NO: 114) |
| | HCDR2 | RIYPGNDNTYYNEKFKD (SEQ ID NO: 122) | LCDR2 | AATNLAD (SEQ ID NO: 109) |
| | HCDR3 | SQDGYYEENAMDY (SEQ ID NO: 123) | LCDR3 | QQFWGSPHT (SEQ ID NO: 124) |
| M P1-G7 | HCDR1 | SGYYWN (SEQ ID NO: 105) | LCDR1 | RASENIYRNLA (SEQ ID NO: 127) |
| | HCDR2 | YISYDGGNNYNPFLKN (SEQ ID NO: 125) | LCDR2 | AATNLAD (SEQ ID NO: 109) |
| | HCDR3 | EGGSSWEYYGMDY (SEQ ID NO: 126) | LCDR3 | QQFWGSPYT (SEQ ID NO: 128) |
| M P2-C7 | HCDR1 | DYSMH (SEQ ID NO: 129) | LCDR1 | RTSENIYSNLA (SEQ ID NO: 132) |
| | HCDR2 | YFNPNNGDTIYNQKFKG (SEQ ID NO: 130) | LCDR2 | GATNLAD (SEQ ID NO: 133) |
| | HCDR3 | EDSVSTNAWDGALDY (SEQ ID NO: 131) | LCDR3 | QHFWDIPWT (SEQ ID NO: 134) |

(continued)

| Antibody | Heavy chain | | Light chain | | |
|---|---|---|---|---|---|
| M P2-C6 | HCDR1 | DYYIH (SEQ ID NO: 135) | LCDR1 | RASESVNNYGVGFLH (SEQ ID NO: 138) | |
| | HCDR2 | YIYPDNGYNGYNQKFKG (SEQ ID NO: 136) | LCDR2 | RASTLES (SEQ ID NO: 139) | |
| | HCDR3 | RGDYSGVHFDY (SEQ ID NO: 137) | LCDR3 | QHSYKDPPWT (SEQ ID NO: 140) | |
| M P1-D7 | HCDR1 | TYGMN (SEQ ID NO: 141) | LCDR1 | KSSQSLFYSGNQKNYLA (SEQ ID NO: 144) | |
| | HCDR2 | WINTYSGVPTYADDFKG (SEQ ID NO: 142) | LCDR2 | WASTRES (SEQ ID NO: 145) | |
| | HCDR3 | RTDYDGYVMDC (SEQ ID NO: 143) | LCDR3 | QQYFSYPPT (SEQ ID NO: 146) | |
| M P1-E11 | HCDR1 | DYSIH (SEQ ID NO: 147) | LCDR1 | RASENIYSNLV (SEQ ID NO: 150) | |
| | HCDR2 | YINPNNGVTNYNQKFKG (SEQ ID NO: 148) | LCDR2 | AAINLVD (SEQ ID NO: 151) | |
| | HCDR3 | EGDSVTTIVVHWYFDV (SEQ ID NO: 149) | LCDR3 | QQFWDTPLT (SEQ ID NO: 152) | |
| M P2-B9 | HCDR1 | DYFMN (SEQ ID NO: 153) | LCDR1 | RASENIYSNLA (SEQ ID NO: 114) | |
| | HCDR2 | DINPNNGGTSYNQRFKD (SEQ ID NO: 154) | LCDR2 | GATNLAD (SEQ ID NO: 133) | |
| | HCDR3 | NGYYDSNYLSYFDV (SEQ ID NO: 155) | LCDR3 | QHFWDTPYT (SEQ ID NO: 119) | |
| M P2-C2 | HCDR1 | SYGVH (SEQ ID NO: 58) | LCDR1 | KAGQDVSIAVA (SEQ ID NO: 158) | |
| | HCDR2 | VIWSGGHTDLNAAFIS (SEQ ID NO: 156) | LCDR2 | SASYRYT (SEQ ID NO:62) | |
| | HCDR3 | NGYGGCLDY (SEQ ID NO: 157) | LCDR3 | QQYYSIPPS (SEQ ID NO: 159) | |
| M P2-E10 | HCDR1 | SGYYWN (SEQ ID NO: 105) | LCDR1 | RASENIYSNLA (SEQ ID NO: 114) | |
| | HCDR2 | YMSYEGTNNSNPSLKN (SEQ ID NO: 160) | LCDR2 | AATNLAD (SEQ ID NO: 109) | |
| | HCDR3 | ERAYYYGTLGAMDY (SEQ ID NO: 161) | LCDR3 | QHFWDTPFT (SEQ ID NO: 162) | |

3. Phage library screening

Since the screening was aimed at obtaining antibody sequences that bind only to ILT4, reverse screening and forward screening were combined: phages that bind to biotinylated human ILT2 were removed first from a phage library (reverse screening), and phages that bind to biotinylated human ILT4 were then selected from the remaining phages (forward screening). Specifically, the phage library ($5 \times 10^{12}$/pfu) was suspended in 1 mL of PBS containing skim milk powder (BD, 232100), 2 $\mu$g/mL biotinylated human ILT2-His protein was added, and incubation was performed in an inverted position. Subsequently, 100 $\mu$L of Dynabeads® M-280 Streptavidin (Thermofisher, 11206D) was added, and incubation was performed. The tube was placed on a magnetic rack for 30 s, and the supernatant was taken out. 2 $\mu$g/mL biotinylated human ILT4-His protein was added, and incubation was performed in an inverted position. Subsequently, 100 $\mu$L of Dynabeads was added, and incubation was performed. The tube was placed on a magnetic rack for 30 s, and the supernatant was removed. The Dynabeads were washed several times with 1 mL of PBS containing Tween-20, and 0.5 mL of trypsin (Sigma, T1426-250MG) was then added for elution. The eluted phages were used to infect E. coli SS320. The titer was determined, and expansion and concentration were performed. The resulting phages were used in the next round of panning. After three rounds of screening based on this method, the obtained single clones were inoculated into a 96-well plate (Sangon Biotech, F600582-0001) for phage packaging. Phages that bind to human ILT4 protein and do not bind to human ILT2 protein were identified by ELISA, and the corresponding single clones were sequenced. The variable region sequences of the finally obtained antibody are shown below:

> P2 HCVR (SEQ ID NO: 163)

QVQLVQSGAEVKKPGSSVKVSCKASGYTFTSYDISWVRQAPGQGLEWMGGII
PIFGTANYAQKFQGRVTITADESTSTAYMELSSLRSEDTAVYYCTAGIFLATAYW
GQGTLVTVSS

> P2 LCVR (SEQ ID NO: 164)

DIQMTQSPSSMSASVGDRVTISCQASQDINNYLNWYQQRPGQAPKLLIYDAST
LETGVPSRFSGSGSGTYFTLTITSLQPADVATYYCQQYENVPITFGQGTRLEIK

Note: The variable regions are single underlined, and the CDRs are double underlined; the CDRs are defined according to the Kabat numbering scheme.

The heavy and light chain CDR sequences of the fully human-derived antibody are shown in Table 4 below.

Table 4. The heavy and light chain CDR sequences of the antibody

| Antibody | Heavy chain | | Light chain | |
|---|---|---|---|---|
| P2 | HCDR1 | SYDIS (SEQ ID NO: 165) | LCDR1 | QASQDINNYLN (SEQ ID NO: 168) |
| | HCDR2 | GIIPIFGTANYAQ KFQG (SEQ ID NO: 166) | LCDR2 | DASTLET (SEQ ID NO: 169) |
| | HCDR3 | GIFLATAY (SEQ ID NO: 167) | LCDR3 | QQYENVPIT (SEQ ID NO: 170) |

4. Construction of chimeric antibodies

Primers were designed, and VH/VK gene fragments of the murine antibodies were obtained by PCR. The fragments were then homologously recombined with an expression vector pTT5 (with a signal peptide and a constant region gene (CH1-FC/CL) fragment, constructed in the laboratory) to construct an antibody full-length expression vector VH-CH1-FC-pTT5/VK-CL-pTT5, and anti-human ILT4 chimeric antibodies in IgG4(LALA) format were expressed. The variable region sequences of the murine anti-ILT4 antibodies ChP1-C4, ChP1-B4, ChP1-D10, ChP2-C7, ChP1-G7, ChP1-B9, ChP2-C6, ChP1-F7, ChP2-E8, ChP2-E3, ChP2-C10, ChP2-F4, ChP2-F10, ChP2-C4, ChP2-G9, ChP2-B3, ChP2-E7, ChP2-G4, ChP2-E11, ChP1-D7, ChP1-E11, ChP2-B9, ChP2-C2, and ChP2-E10 were combined with the constant regions set forth in SEQ ID NO: 171 and SEQ ID NO: 172 to obtain chimeric antibodies. Illustratively, ChP1-C4 represents a chimeric antibody containing the M P1-C4 murine heavy and light chain variable regions and the constant regions. The heavy and light chain constant region sequences of the chimeric antibodies are shown below:

> hIgG4(LALA) (SEQ ID NO: 171)

ASTKGPSVFPLAPCSRSTSESTAALGCLVKDYFPEPVTVSWNSGALTSGVHTFP

AVLQSSGLYSLSSVVTVPSSSLGTKTYTCNVDHKPSNTKVDKRVESKYGPPCP
PCPAPEAAGGPSVFLFPPKPKDTLMISRTPEVTCVVVDVSQEDPEVQFNWYVD
GVEVHNAKTKPREEQFNSTYRVVSVLTVLHQDWLNGKEYKCKVSNKGLPSSI
EKTISKAKGQPREPQVYTLPPSQEEMTKNQVSLTCLVKGFYPSDIAVEWESNG
QPENNYKTTPPVLDSDGSFFLYSRLTVDKSRWQEGNVFSCSVMHEALHNHYT
QKSLSLSLGK

Note: The CH1 region is single underlined, the hinge region is dotted underlined, and the Fc region is double underlined.
> CL (SEQ ID NO: 172)

RTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGE
C.

**Example 4: Humanization of Anti-Human ILT4 Murine Antibodies**

By comparing the IMGT human antibody heavy and light chain variable region germline gene database and MOE software, heavy and light chain variable region germline genes that are highly homologous with M P2-E3, M P2-G9, M P2-E8, M P1-G7, M P2-C6, and M P1-D7 were selected as templates, and the CDRs of the six murine antibodies were grafted into corresponding humanization templates to form variable region sequences with the order FR1-CDR1-FR2-CDR2-FR3-CDR3-FR4. The amino acid residues were determined using the Kabat numbering scheme and annotated.

1. Humanization engineering of M P2-E3

For humanized antibodies of the M P2-E3 antibody, IGKV1-39*01 was selected as a light chain framework region template, and IGHV1-3*01 was selected as a heavy chain framework region template. The CDRs of the murine antibodies were grafted onto the selected humanization templates, and embedded residues, residues directly interacting with the CDRs, and sites with high-risk hotspots and immunogenicity risks were identified using MOE software and back-mutated and point-mutated to design different light and heavy chain variable regions for humanized antibodies.

Table 5. The specific framework replacements and amino acid substitutions for P2-E3 humanized antibodies

| VL | | VH | |
|---|---|---|---|
| L1 | Graft(IGKV1-39*01) + I48V | H1 | Graft(IGHV1-3*01) + Q1E, R71V, T73K |
| L2 | Graft(IGKV1-39*01) + A43S, I48V, F71Y | H2 | Graft(IGHV1-3*01) + Q1E, R44S, I69L, R71V, T73K |
| | | H3 | Graft(IGHV1-3*01) + Q1E, R44S, I69L, R71V, T73K + N54S |
| | | H4 | Graft(IGHV1-3*01) + Q1E, R44S, I69L, R71V, T73K + N54T |
| | | H5 | Graft(IGHV1-3*01) + Q1E, R44S, I69L, R71V, T73K + G55V |
| | | H6 | Graft(IGHV1-3*01) + Q1E, R38K, R71V, T73K, A75G+N54S |
| FR4 | IGKJ2*01 | FR4 | IGHJ6*01 |
| Note: The amino acids are numbered according to the Kabat numbering scheme. I48V indicates that the I at position 48, according to the Kabat numbering scheme, is mutated into V, and so on for other mutations. | | | |

Table 6. The CDRs of P2-E3 humanized antibodies

| Variable region | CDR | Sequence | SEQ ID NO |
|---|---|---|---|
| huP2-E3 VH3 | HCDR2 | DINPNSGGTSYNQKFKG | 173 |
| huP2-E3 VH4 | HCDR2 | DINPNTGGTSYNQKFKG | 174 |
| huP2-E3 VH5 | HCDR2 | DINPNNVGTSYNQKFKG | 175 |
| huP2-E3 VH6 | HCDR2 | DINPNSGGTSYNQKFKG | 173 |

The variable region sequences of P2-E3 humanized antibodies are shown below:

> huP2-E3 VH1 (SEQ ID NO: 176)

EVQLVQSGAEVKKPGASVKVSCKASGYTFTDYYMNWVRQAPGQRLEWMG
DINPNNGGTSYNQKFKGRVTITVDKSASTAYMELSSLRSEDTAVYYCARGGVT
TVVATYWYFDVWGQGTTVTVSS

> huP2-E3 VH2 (SEQ ID NO: 177)

EVQLVQSGAEVKKPGASVKVSCKASGYTFTDYYMNWVRQAPGQSLEWMGD
INPNNGGTSYNQKFKGRVTLTVDKSASTAYMELSSLRSEDTAVYYCARGGVTT
VVATYWYFDVWGQGTTVTVSS

> huP2-E3 VH3 (SEQ ID NO: 178)

EVQLVQSGAEVKKPGASVKVSCKASGYTFTDYYMNWVRQAPGQSLEWMGD
INPNSGGTSYNQKFKGRVTLTVDKSASTAYMELSSLRSEDTAVYYCARGGVTT
VVATYWYFDVWGQGTTVTVSS

> huP2-E3 VH4 (SEQ ID NO: 179)

EVQLVQSGAEVKKPGASVKVSCKASGYTFTDYYMNWVRQAPGQSLEWMGD

INPNTGGTSYNQKFKGRVTLTVDKSASTAYMELSSLRSEDTAVYYCARGGVTT
VVATYWYFDVWGQGTTVTVSS

> huP2-E3 VH5 (SEQ ID NO: 180)

EVQLVQSGAEVKKPGASVKVSCKASGYTFTDYYMNWVRQAPGQSLEWMGD
INPNNVGTSYNQKFKGRVTLTVDKSASTAYMELSSLRSEDTAVYYCARGGVTT
VVATYWYFDVWGQGTTVTVSS

> huP2-E3 VH6 (SEQ ID NO: 181)

EVQLVQSGAEVKKPGASVKVSCKASGYTFTDYYMNWVKQAPGQRLEWMG
DINPNSGGTSYNQKFKGRVTITVDKSGSTAYMELSSLRSEDTAVYYCARGGVT
TVVATYWYFDVWGQGTTVTVSS

> huP2-E3 VL1 (SEQ ID NO: 182)

DIQMTQSPSSLSASVGDRVTITCRASENIYSNLAWYQQKPGKAPKLLVYAATN
VADGVPSRFSGSGSGTDFTLTISSLQPEDFATYYCQQFWDTPFTFGQGTKLEIK

> huP2-E3 VL2 (SEQ ID NO: 183)

DIQMTQSPSSLSASVGDRVTITCRASENIYSNLAWYQQKPGKSPKLLVYAATN
VADGVPSRFSGSGSGTDYTLTISSLQPEDFATYYCQQFWDTPFTFGQGTKLEIK

Note: The variable regions are single underlined, and the CDRs are double underlined; the CDRs are defined according to the Kabat numbering scheme.

Table 7. Humanized antibodies of the P2-E3 series

|  | huP2-E3 VH1 | huP2-E3 VH2 | huP2-E3 VH3 | huP2-E3 VH4 | huP2-E3 VH5 | huP2-E3 VH6 |
|---|---|---|---|---|---|---|
| huP2-E3 VL1 | P2-E3H1L1 | P2-E3H2L1 | P2-E3H3L1 | P2-E3H4L1 | P2-E3H5L1 | P2-E3H6L1 |
| huP2-E3 VL2 | P2-E3H1L2 | P2-E3H2L2 | P2-E3H3L2 | P2-E3H4L2 | P2-E3H5L2 | P2-E3H6L2 (also known as huP2-E3) |

2. Humanization engineering of M P2-G9

For humanized antibodies of the M P2-G9 antibody, IGKV1-39*01 was selected as a light chain framework region template, and IGHV4-31*01 was selected as a heavy chain framework region template. The CDRs of the murine antibodies were grafted onto the selected humanization templates, and embedded residues, residues directly interacting with the CDRs, and sites with high-risk hotspots and immunogenicity risks were identified using MOE software and back-mutated and point-mutated to design different light and heavy chain variable regions for humanized antibodies.

Table 8. The specific framework replacements and amino acid substitutions for P2-G9 humanized antibodies

| VL |  | VH |  |
|---|---|---|---|
| L1 | Graft(IGKV1-39*01) + I48V | H1 | Graft(IGHV4-31*01) + G27S, S30T, V71R + Q1E |
| L2 | Graft(IGKV1-39*01) + A43S, I48V, F71Y | H2 | Graft(IGHV4-31*01) + G27S, S30T, G44K, V71R, N76S + Q1E |
| FR4 | IGKJ4*01 | FR4 | IGHJ1*01 |
| Note: The amino acids are numbered according to the Kabat numbering scheme. | | | |

The variable region sequences of P2-G9 humanized antibodies are shown below:

> huP2-G9 VH1 (SEQ ID NO: 184)

EVQLQESGPGLVKPSQTLSLTCTVSGSSITSGYYWNWIRQHPGKGLEWIGYLS
YDGSNNYNPSLKNLVTISRDTSKNQFSLKLSSVTAADTAVYYCAREGAHYSGT
LSWFAYWGQGTLVTVSS

> huP2-G9 VH2 (SEQ ID NO: 185)

EVQLQESGPGLVKPSQTLSLTCTVSGSSITSGYYWNWIRQHPGKKLEWIGYLS YDGSNNYNPSLKNLVTISRDTSKSQFSLKLSSVTAADTAVYYCAREGAHYSGT LSWFAYWGQGTLVTVSS

> huP2-G9 VL1 (SEQ ID NO: 186)

DIQMTQSPSSLSASVGDRVTITCRASENIYSNLAWYQQKPGKAPKLLVYAATN LADGVPSRFSGSGSGTDFTLTISSLQPEDFATYYCQHFWDTPYTFGGGTKVEIK

> huP2-G9 VL2 (SEQ ID NO: 187)

DIQMTQSPSSLSASVGDRVTITCRASENIYSNLAWYQQKPGKSPKLLVYAATN LADGVPSRFSGSGSGTDYTLTISSLQPEDFATYYCQHFWDTPYTFGGGTKVEIK

Note: The variable regions are single underlined, and the CDRs are double underlined; the CDRs are defined according to the Kabat numbering scheme.

Table 9-1. Humanized antibodies of the P2-G9 series

|  | huP2-G9 VH1 | huP2-G9 VH2 |
|---|---|---|
| huP2-G9 VL1 | P2-G9H1L1 | P2-G9H2L1 |
| huP2-G9 VL2 | P2-G9H1L2 (also known as huP2-G9) | P2-G9H2L2 |

Table 9-2. The CDR sequences of the huP2-G9 antibodies (according to the Kabat numbering scheme)

| Antibody | Name | Sequence | No. |
|---|---|---|---|
| huP2-G9 | HCDR1 | SGYYWN | SEQ ID NO: 105 |
|  | HCDR2 | YLSYDGSNNYNPSLKN | SEQ ID NO: 120 |
|  | HCDR3 | EGAHYSGTLSWFAY | SEQ ID NO: 121 |
|  | LCDR1 | RASENIYSNLA | SEQ ID NO: 114 |
|  | LCDR2 | AATNLAD | SEQ ID NO: 109 |
|  | LCDR3 | QHFWDTPYT | SEQ ID NO: 119 |

Table 9-3. The CDR sequences of the huP2-G9 antibodies (according to the IMGT numbering scheme)

| Antibody | Name | Sequence | No. |
|---|---|---|---|
| huP2-G9 | HCDR1 | GSSITSGYY | SEQ ID NO: 260 |
|  | HCDR2 | LSYDGSN | SEQ ID NO: 261 |
|  | HCDR3 | AREGAHYSGTLSWFAY | SEQ ID NO: 262 |
|  | LCDR1 | ENIYSN | SEQ ID NO: 263 |
|  | LCDR2 | AAT | SEQ ID NO: 264 |
|  | LCDR3 | QHFWDTPYT | SEQ ID NO: 119 |

Table 9-4. The CDR sequences of the huP2-G9 antibodies (according to the Chothia numbering scheme)

| Antibody | Name | Sequence | No. |
|---|---|---|---|
| huP2-G9 | HCDR1 | GSSITSGY | SEQ ID NO: 265 |
| | HCDR2 | SYDGS | SEQ ID NO: 266 |
| | HCDR3 | EGAHYSGTLSWFAY | SEQ ID NO: 121 |
| | LCDR1 | RASENIYSNLA | SEQ ID NO: 114 |
| | LCDR2 | AATNLAD | SEQ ID NO: 109 |
| | LCDR3 | QHFWDTPYT | SEQ ID NO: 119 |

3. Humanization engineering of M P2-E8

For humanized antibodies of the M P2-E8 antibody, IGKV1-33*01 was selected as a light chain framework region template, and IGHV3-21*01 was selected as a heavy chain framework region template. The CDRs of the murine antibodies were grafted onto the selected humanization templates, and embedded residues, residues directly interacting with the CDRs, and sites with high-risk hotspots and immunogenicity risks were identified using MOE software and back-mutated and point-mutated to design different light and heavy chain variable regions for humanized antibodies.

Table 10. The specific framework replacements and amino acid substitutions for P2-E8 humanized antibodies

| VL | | VH | |
|---|---|---|---|
| L1 | Graft(IGKV1-33*01) | H1 | Graft(IGHV3-21*01) |
| L2 | Graft(IGKV1-33*01) + A43S, T85V | H2 | Graft(IGHV3-21*01) + S49A |
| L3 | Graft(IGKV1-33*01) + K39R, K42Q, A43S, T85V | H3 | Graft(IGHV3-21*01) + S49A + S55N |
| | | H4 | Graft(IGHV3-21*01) + S49A + I57H |
| FR4 | IGKJ4*01 | FR4 | IGHJ1*01 |
| Note: The amino acids are numbered according to the Kabat numbering scheme. | | | |

Table 11. The CDRs of P2-E8 humanized antibodies

| Variable region | CDR | Sequence | SEQ ID NO |
|---|---|---|---|
| huP2-E8 VH3 | HCDR2 | YISSGSNIINYADTVKG | 188 |
| huP2-E8 VH4 | HCDR2 | YISSGSSIHNYADTVKG | 189 |

The variable region sequences of P2-E8 humanized antibodies are shown below:

> huP2-E8 VH1 (SEQ ID NO: 190)

EVQLVESGGGLVKPGGSLRLSCAASGFTFSDYGIHWVRQAPGKGLEWVSYISS
GSSIINYADTVKGRFTISRDNAKNSLYLQMNSLRAEDTAVYYCARGPNYYGSS
YLLVYWGQGTLVTVSS

> huP2-E8 VH2 (SEQ ID NO: 191)

EVQLVESGGGLVKPGGSLRLSCAASGFTFSDYGIHWVRQAPGKGLEWVAYISS
GSSIINYADTVKGRFTISRDNAKNSLYLQMNSLRAEDTAVYYCARGPNYYGSS
YLLVYWGQGTLVTVSS

> huP2-E8 VH3 (SEQ ID NO: 192)

EVQLVESGGGLVKPGGSLRLSCAASGFTFSDYGIHWVRQAPGKGLEWVAYISSGSNIINYADTVKGRFTISRDNAKNSLYLQMNSLRAEDTAVYYCARGPNYYGSSYLLVYWGQGTLVTVSS

> huP2-E8 VH4 (SEQ ID NO: 193)

EVQLVESGGGLVKPGGSLRLSCAASGFTFSDYGIHWVRQAPGKGLEWVAYISSGSSIHNYADTVKGRFTISRDNAKNSLYLQMNSLRAEDTAVYYCARGPNYYGSYLLVYWGQGTLVTVSS

> huP2-E8 VL1 (SEQ ID NO: 194)

DIQMTQSPSSLSASVGDRVTITCKASQDVNIAVAWYQQKPGKAPKLLIYSASYRYTGVPSRFSGSGSGTDFTFTISSLQPEDIATYYCQQHYNIPWTFGGGTKVEIK

> huP2-E8 VL2 (SEQ ID NO: 195)

DIQMTQSPSSLSASVGDRVTITCKASQDVNIAVAWYQQKPGKSPKLLIYSASYRYTGVPSRFSGSGSGTDFTFTISSLQPEDIAVYYCQQHYNIPWTFGGGTKVEIK

> huP2-E8 VL3 (SEQ ID NO: 196)

DIQMTQSPSSLSASVGDRVTITCKASQDVNIAVAWYQQRPGQSPKLLIYSASYRYTGVPSRFSGSGSGTDFTFTISSLQPEDIAVYYCQQHYNIPWTFGGGTKVEIK

Note: The variable regions are single underlined, and the CDRs are double underlined; the CDRs are defined according to the Kabat numbering scheme.

Table 12. Humanized antibodies of the P2-E8 series

|  | huP2-E8 VH1 | huP2-E8 VH2 | huP2-E8 VH3 | huP2-E8 VH4 |
|---|---|---|---|---|
| huP2-E8 VL1 | P2-E8H1L1 | P2-E8H2L1 | P2-E8H3L1 | P2-E8H4L1 (also known as huP2-E8) |
| huP2-E8 VL2 | P2-E8H1L2 | P2-E8H2L2 | P2-E8H3L2 | P2-E8H4L2 |
| huP2-E8 VL3 | P2-E8H1L3 | P2-E8H2L3 | P2-E8H3L3 | P2-E8H4L3 |

4. Humanization engineering of M P1-G7

For humanized antibodies of the M P1-G7 antibody, IGKV1-39*01 was selected as a light chain framework region template, and IGHV4-31*02 was selected as a heavy chain framework region template. The CDRs of the murine antibodies were grafted onto the selected humanization templates, and embedded residues, residues directly interacting with the CDRs, and sites with high-risk hotspots and immunogenicity risks were identified using MOE software and back-mutated and point-mutated to design different light and heavy chain variable regions for humanized antibodies.

Table 13. The specific framework replacements and amino acid substitutions for P1-G7 humanized antibodies

| VL | | | VH | | |
|---|---|---|---|---|---|
| L1 | Graft(IGKV1-39*01) + I48L | | H1 | Graft(IGHV4-31*02) + G27Y, S30T, V71R + Q1E | |
| L2 | Graft(IGKV1-39*01) + A43S, I48L, F71Y | | H2 | Graft(IGHV4-31*02) + G27Y, S30T, G42E, G44K, V71R + Q1E | |

(continued)

| VL | | VH | |
|---|---|---|---|
| FR4 | IGKJ4*01 | FR4 | IGHJ6*01 |
| Note: The amino acids are numbered according to the Kabat numbering scheme. | | | |

The variable region sequences of P1-G7 humanized antibodies are shown below:

> huP1-G7 VH1 (SEQ ID NO: 197)

EVQLQESGPGLVKPSQTLSLTCTVSGYSITSGYYWNWIRQHPGKGLEWIGYIS
YDGGNNYNPFLKNRVTISRDTSKNQFSLKLSSVTAADTAVYYCAREGGSSWE
YYGMDYWGQGTTVTVSS

> huP1-G7 VH2 (SEQ ID NO: 198)

EVQLQESGPGLVKPSQTLSLTCTVSGYSITSGYYWNWIRQHPEKKLEWIGYIS
YDGGNNYNPFLKNRVTISRDTSKNQFSLKLSSVTAADTAVYYCAREGGSSWE
YYGMDYWGQGTTVTVSS

> huP1-G7 VL1 (SEQ ID NO: 199)

DIQMTQSPSSLSASVGDRVTITCRASENIYRNLAWYQQKPGKAPKLLLYAATN
LADGVPSRFSGSGSGTDFTLTISSLQPEDFATYYCQQFWGSPYTFGGGTKVEIK

> huP1-G7 VL2 (SEQ ID NO: 200)

DIQMTQSPSSLSASVGDRVTITCRASENIYRNLAWYQQKPGKSPKLLLYAATNL
ADGVPSRFSGSGSGTDYTLTISSLQPEDFATYYCQQFWGSPYTFGGGTKVEIK

Note: The variable regions are single underlined, and the CDRs are double underlined; the CDRs are defined according to the Kabat numbering scheme.

Table 14. Humanized antibodies of the P1-G7 series

| | huP1-G7 VH1 | huP1-G7 VH2 |
|---|---|---|
| huP1-G7 VL1 | P1-G7H1L1 (also known as huP1-G7) | P1-G7H2L1 |
| huP1-G7 VL2 | P1-G7H1L2 | P1-G7H2L2 |

5. Humanization engineering of M P2-C6

For humanized antibodies of the P2-C6 antibody, IGKV4-1*01 and IGKV3-11*01 were selected as light chain framework region templates, and IGHV1-18*01 was selected as a heavy chain framework region template. The CDRs of the murine antibodies were grafted onto the selected humanization templates, and embedded residues, residues directly interacting with the CDRs, and sites with high-risk hotspots and immunogenicity risks were identified using MOE software and back-mutated and point-mutated to design different light and heavy chain variable regions for humanized antibodies.

Table 15. The specific framework replacements and amino acid substitutions for P2-C6 humanized antibodies

| VL | | VH | |
|---|---|---|---|
| L1 | Graft(IGKV4-1*01) + M4L, V58I | H1 | Graft(IGHV1-18*01) + Q1E, T71V, T73K, A93T |

(continued)

| VL | | VH | |
|---|---|---|---|
| L2 | Graft(IGKV4-1*01) + M4L, V58I, G68R, V85T | H2 | Graft(IGHV1-18*01) + Q1E, Q43E, M69L, T71V, T73K, S76N, A93T |
| L3 | Graft(IGKV3-11*01) + A43P, R45K, G68R | H3 | Graft(IGHV1-18*01) + Q1E, Q43E, M69L, T71V, T73K, S76N, A93T+ N57S |
| | | H4 | Graft(IGHV1-18*01) + Q1E, Q43E, M69L, T71V, T73K, S76N, A93T + N57T |
| | | H5 | Graft(IGHV1-18*01) + Q1E, Q43E, M69L, T71V, T73K, S76N, A93T + N54T, N57S |
| | | H6 | Graft(IGHV1-18*01) + Q1E, Q43E, M69L, T71V, T73K, S76N, A93T + N54S |
| | | H7 | Graft(IGHV1-18*01) + Q1E, T71V, T73K, A93T + N54T, N57S |
| FR4 | IGKJ4*01 | FR4 | IGHJ6*01 |
| Note: The amino acids are numbered according to the Kabat numbering scheme. | | | |

Table 16. The CDRs of P2-C6 humanized antibodies

| Variable region | CDR | Sequence | SEQ ID NO |
|---|---|---|---|
| huP2-C6 VH3 | HCDR2 | YIYPDNGYSGYNQKFKG | 201 |
| huP2-C6 VH4 | HCDR2 | YIYPDNGYTGYNQKFKG | 202 |
| huP2-C6 VH5 | HCDR2 | YIYPDTGYSGYNQKFKG | 203 |
| huP2-C6 VH6 | HCDR2 | YIYPDSGYNGYNQKFKG | 204 |
| huP2-C6 VH7 | HCDR2 | YIYPDTGYSGYNQKFKG | 203 |

The variable region sequences of P2-C6 humanized antibodies are shown below:

> huP2-C6 VH1 (SEQ ID NO: 205)

EVQLVQSGAEVKKPGASVKVSCKASGYTFTDYYIHWVRQAPGQGLEWMGYI
YPDNGYNGYNQKFKGRVTMTVDKSTSTAYMELRSLRSDDTAVYYCTRRGDY
SGVHFDYWGQGTTVTVSS

> huP2-C6 VH2 (SEQ ID NO: 206)

EVQLVQSGAEVKKPGASVKVSCKASGYTFTDYYIHWVRQAPGEGLEWMGYI
YPDNGYNGYNQKFKGRVTLTVDKSTNTAYMELRSLRSDDTAVYYCTRRGDY
SGVHFDYWGQGTTVTVSS

> huP2-C6 VH3 (SEQ ID NO: 207)

EVQLVQSGAEVKKPGASVKVSCKASGYTFTDYYIHWVRQAPGEGLEWMGYI
YPDNGYSGYNQKFKGRVTLTVDKSTNTAYMELRSLRSDDTAVYYCTRRGDYS
GVHFDYWGQGTTVTVSS

> huP2-C6 VH4 (SEQ ID NO: 208)

EVQLVQSGAEVKKPGASVKVSCKASGYTFTDYYIHWVRQAPGEGLEWMGYIYPDNGYTGYNQKFKGRVTLTVDKSTNTAYMELRSLRSDDTAVYYCTRRGDYSGVHFDYWGQGTTVTVSS

> huP2-C6 VH5 (SEQ ID NO: 209)

EVQLVQSGAEVKKPGASVKVSCKASGYTFTDYYIHWVRQAPGEGLEWMGYIYPDTGYSGYNQKFKGRVTLTVDKSTNTAYMELRSLRSDDTAVYYCTRRGDYSGVHFDYWGQGTTVTVSS

> huP2-C6 VH6 (SEQ ID NO: 210)

EVQLVQSGAEVKKPGASVKVSCKASGYTFTDYYIHWVRQAPGEGLEWMGYIYPDSGYNGYNQKFKGRVTLTVDKSTNTAYMELRSLRSDDTAVYYCTRRGDYSGVHFDYWGQGTTVTVSS

> huP2-C6 VH7 (SEQ ID NO: 211)

EVQLVQSGAEVKKPGASVKVSCKASGYTFTDYYIHWVRQAPGQGLEWMGYIYPDTGYSGYNQKFKGRVTMTVDKSTSTAYMELRSLRSDDTAVYYCTRRGDYSGVHFDYWGQGTTVTVSS

> huP2-C6 VL1 (SEQ ID NO: 212)

DIVLTQSPDSLAVSLGERATINCRASESVNNYGVGFLHWYQQKPGQPPKLLIYRASTLESGIPDRFSGSGSGTDFTLTISSLQAEDVAVYYCQHSYKDPPWTFGGGTKVEIK

> huP2-C6 VL2 (SEQ ID NO: 213)

DIVLTQSPDSLAVSLGERATINCRASESVNNYGVGFLHWYQQKPGQPPKLLIYRASTLESGIPDRFSGSGSRTDFTLTISSLQAEDVATYYCQHSYKDPPWTFGGGTKVEIK

> huP2-C6 VL3 (SEQ ID NO: 214)

EIVLTQSPATLSLSPGERATLSCRASESVNNYGVGFLHWYQQKPGQPPKLLIYRASTLESGIPARFSGSGSRTDFTLTISSLEPEDFAVYYCQHSYKDPPWTFGGGTKVEIK

Note: The variable regions are single underlined, and the CDRs are double underlined; the CDRs are defined according to the Kabat numbering scheme.

**EP 4 585 618 A1**

Table 17-1. Humanized antibodies of the P2-C6 series

| | huP2-C6 VH1 | huP2-C6 VH2 | huP2-C6 VH3 | huP2-C6 VH4 | huP2-C6 VH5 | huP2-C6 VH6 | huP2-C6 VH7 |
|---|---|---|---|---|---|---|---|
| huP2-C6 VL1 | P2-C6H1L1 | P2-C6H2L1 | P2-C6H3L1 | P2-C6H4L1 | P2-CoH5L1 | P2-C6H6L1 | P2-C6H7L1 (also known as huP2-C6) |
| huP2-C6 VL2 | P2-C6H1L2 | P2-C6H2L2 | P2-C6H3L2 | P2-C6H4L2 | P2-C6H5L2 | P2-C6H6L2 | P2-C6H7L2 |
| huP2-C6 VL3 | P2-C6H1L3 | P2-C6H2L3 | P2-C6H3L3 | P2-C6H4L3 | P2-C6H5L3 | P2-C6H6L3 | P2-C6H7L3 |

Table 17-2. The CDR sequences of the huP2-C6 antibodies (according to the Kabat numbering scheme)

| Antibody | Name | Sequence | No. |
|---|---|---|---|
| huP2-C6 | HCDR1 | DYYIH | SEQ ID NO: 135 |
| | HCDR2 | YIYPDTGYSGYNQKFKG | SEQ ID NO: 203 |
| | HCDR3 | RGDYSGVHFDY | SEQ ID NO: 137 |
| | LCDR1 | RASESVNNYGVGFLH | SEQ ID NO: 138 |
| | LCDR2 | RASTLES | SEQ ID NO: 139 |
| | LCDR3 | QHSYKDPPWT | SEQ ID NO: 140 |

Table 17-3. The CDR sequences of the huP2-C6 antibodies (according to the IMGT numbering scheme)

| Antibody | Name | Sequence | No. |
|---|---|---|---|
| huP2-C6 | HCDR1 | GYTFTDYY | SEQ ID NO: 267 |
| | HCDR2 | IYPDTGYS | SEQ ID NO: 268 |
| | HCDR3 | TRRGDYSGVHFDY | SEQ ID NO: 269 |
| | LCDR1 | ESVNNYGVGF | SEQ ID NO: 270 |
| | LCDR2 | RAS | SEQ ID NO: 271 |
| | LCDR3 | QHSYKDPPWT | SEQ ID NO: 140 |

Table 17-4. The CDR sequences of the huP2-C6 antibodies (according to the Chothia numbering scheme)

| Antibody | Name | Sequence | No. |
|---|---|---|---|
| huP2-C6 | HCDR1 | GYTFTDY | SEQ ID NO: 272 |
| | HCDR2 | YPDTGY | SEQ ID NO: 273 |
| | HCDR3 | RGDYSGVHFDY | SEQ ID NO: 137 |
| | LCDR1 | RASESVNNYGVGFLH | SEQ ID NO: 138 |
| | LCDR2 | RASTLES | SEQ ID NO: 139 |
| | LCDR3 | QHSYKDPPWT | SEQ ID NO: 140 |

6. Humanization engineering of M P1-D7

For humanized antibodies of the M P1-D7 antibody, IGKV4-1*01 and IGKV2-28*01 were selected as light chain framework region templates, and IGHV1-18*01 was selected as a heavy chain framework region template. The CDRs of the murine antibodies were grafted onto the selected humanization templates, and embedded residues, residues directly interacting with the CDRs, and sites with high-risk hotspots and immunogenicity risks were identified using MOE software and back-mutated and point-mutated to design different light and heavy chain variable regions for humanized antibodies.

Table 18. The specific framework replacements and amino acid substitutions for P1-D7 humanized antibodies

| VL | | VH | |
|---|---|---|---|
| L1 | Graft(IGKV4-1*01) + P43S, I48L | H1 | Graft(IGHV1-18*01) + Q1E, D61E, C102S |
| L2 | Graft(IGKV2-28*01) + I48L | H2 | Graft(IGHV1-18*01) + V2I, S76T + Q1E, D61E, C102S |
| | | H3 | Graft(IGHV1-18*01) + V2I, G44A, E46K, S76T + Q1E, D61E, C102S |
| | | H4 | Graft(IGHV1-18*01) + V2I, G44A, E46K, S76T + Q1E, C102S |
| | | H5 | Graft(IGHV1-18*01) + V2I, G44A, E46K, S76T + Q1E |
| FR4 | IGKJ4*01 | FR4 | IGHJ6*01 |
| Note: The amino acids are numbered according to the Kabat numbering scheme. | | | |

Table 19. The CDRs of P1-D7 humanized antibodies

| Variable region | CDR | Sequence | SEQ ID NO |
|---|---|---|---|
| huP1-D7 VH1 | HCDR2 | WINTYSGVPTYAEDFKG | 215 |
| huP1-D7 VH2 | HCDR2 | WINTYSGVPTYAEDFKG | 215 |
| huP1-D7 VH3 | HCDR2 | WINTYSGVPTYAEDFKG | 215 |
| huP1-D7 VH1 | HCDR3 | RTDYDGYVMDS | 216 |
| huP1-D7 VH2 | HCDR3 | RTDYDGYVMDS | 216 |
| huP1-D7 VH3 | HCDR3 | RTDYDGYVMDS | 216 |
| huP1-D7 VH4 | HCDR3 | RTDYDGYVMDS | 216 |

The variable region sequences of P1-D7 humanized antibodies are shown below:

> huP1-D7 VH1 (SEQ ID NO: 217)

EVQLVQSGAEVKKPGASVKVSCKASGYTFTTYGMNWVRQAPGQGLEWMG
WINTYSGVPTYAEDFKGRVTMTTDTSTSTAYMELRSLRSDDTAVYYCARRTD
YDGYVMDSWGQGTTVTVSS

> huP1-D7 VH2 (SEQ ID NO: 218

EIQLVQSGAEVKKPGASVKVSCKASGYTFTTYGMNWVRQAPGQGLEWMGW
INTYSGVPTYAEDFKGRVTMTTDTSTTAYMELRSLRSDDTAVYYCARRTDYD
GYVMDSWGQGTTVTVSS

> huP1-D7 VH3 (SEQ ID NO: 219)

EIQLVQSGAEVKKPGASVKVSCKASGYTFTTYGMNWVRQAPGQALKWMGW
INTYSGVPTYAEDFKGRVTMTTDTSTTAYMELRSLRSDDTAVYYCARRTDYD
GYVMDSWGQGTTVTVSS

> huP1-D7 VH4 (SEQ ID NO: 220)

EIQLVQSGAEVKKPGASVKVSCKASGYTFTTYGMNWVRQAPGQALKWMGW
INTYSGVPTYADDFKGRVTMTTDTSTTTAYMELRSLRSDDTAVYYCARRTDY
DGYVMDSWGQGTTVTVSS

> huP1-D7 VH5 (SEQ ID NO: 221)

EIQLVQSGAEVKKPGASVKVSCKASGYTFTTYGMNWVRQAPGQALKWMGW
INTYSGVPTYADDFKGRVTMTTDTSTTTAYMELRSLRSDDTAVYYCARRTDY
DGYVMDCWGQGTTVTVSS

> huP1-D7 VL1 (SEQ ID NO: 222)

DIVMTQSPDSLAVSLGERATINCKSSQSLFYSGNQKNYLAWYQQKPGQSPKLL
LYWASTRESGVPDRFSGSGSGTDFTLTISSLQAEDVAVYYCQQYFSYPPTFGGG
TKVEIK

> huP1-D7 VL2 (SEQ ID NO: 223)

DIVMTQSPLSLPVTPGEPASISCKSSQSLFYSGNQKNYLAWYLQKPGQSPQLLL
YWASTRESGVPDRFSGSGSGTDFTLKISRVEAEDVGVYYCQQYFSYPPTFGGG
TKVEIK

Note: The variable regions are single underlined, and the CDRs are double underlined; the CDRs are defined according to the Kabat numbering scheme.

Table 20. Humanized antibodies of the P1-D7 series

|  | huPI-D7 VH1 | huP1-D7 VH2 | huP1-D7 VH3 | huP1-D7 VH4 | huP1-D7 VH5 |
|---|---|---|---|---|---|
| huP1-D7 VL1 | P1-D7H1L1 (also known as huP1-D7) | P1-D7H2L1 | P1-D7H3L1 | P1-D7H4L1 | P1-D7HSL1 |
| huP1-D7 VL2 | P1-D7HIL2 | P1-D7H2L2 | P1-D7H3L2 | P1-D7H4L2 | P1-D7H5L2 |

7. Engineering of P2 fully human-derived antibody

According to a high-risk hotspot, the point mutation N30Q was added to the VL. According to sites with a risk of immunogenicity, the point mutations T76S and A81E were added to the VL.

Table 21. The CDRs of the P2 fully human-derived antibody

| Variable region | CDR | Sequence | SEQ ID NO |
|---|---|---|---|
| VL1 | LCDR1 | QASQDIQNYLN | 224 |

> P2m HCVR (SEQ ID NO: 163)

QVQLVQSGAEVKKPGSSVKVSCKASGYTFTSYDISWVRQAPGQGLEWMGGII
PIFGTANYAQKFQGRVTITADESTSTAYMELSSLRSEDTAVYYCTAGIFLATAYW
GQGTLVTVSS

> P2m LCVR (SEQ ID NO: 225)

DIQMTQSPSSMSASVGDRVTISCQASQDIQNYLNWYQQRPGQAPKLLIYDAST
LETGVPSRFSGSGSGTYFTLTISSLQPEDVATYYCQQYENVPITFGQGTRLEIK

Note: The variable regions are single underlined, and the CDRs are double underlined; the CDRs are defined according to the Kabat numbering scheme.

8. Construction and expression of complete forms of anti-human ILT4 humanized antibodies

Primers were designed, and VH/VK gene fragments of the humanized antibodies were constructed by PCR. The fragments were then homologously recombined with an expression vector pTT5 (with a signal peptide and a constant region gene (CH1-FC/CL) fragment, constructed in the laboratory) to construct an antibody full-length expression vector VH-CH1-Fc-pTT5/VK-CL-pTT5. Through assays using SPR and FACS methods of binding to ILT4 and the like, the heavy and light chain sequences of the humanized antibodies huP2-E3, huP2-G9, huP2-E8, huP1-G7, huP2-C6, and huP1-D7 and the fully human-derived antibody P2m were finally determined as follows:
The humanized antibody huP2-E3:

The heavy chain (SEQ ID NO: 226):

EVQLVQSGAEVKKPGASVKVSCKASGYTFTDYYMNWVKQAPGQRLEWMG
DINPNSGGTSYNQKFKGRVTITVDKSGSTAYMELSSLRSEDTAVYYCARGGVT

TVVATYWYFDVWGQGTTVTVSSASTKGPSVFPLAPCSRSTSESTAALGCLVK
DYFPEPVTVSWNSGALTSGVHTFPAVLQSSGLYSLSSVVTVPSSSLGTKTYTCN
VDHKPSNTKVDKRVESKYGPPCPPCPAPEAAGGPSVFLFPPKPKDTLMISRTPE
VTCVVVDVSQEDPEVQFNWYVDGVEVHNAKTKPREEQFNSTYRVVSVLTVL
HQDWLNGKEYKCKVSNKGLPSSIEKTISKAKGQPREPQVYTLPPSQEEMTKN
QVSLTCLVKGFYPSDIAVEWESNGQPENNYKTTPPVLDSDGSFFLYSRLTVDKS
RWQEGNVFSCSVMHEALHNHYTQKSLSLSLGK

The light chain (SEQ ID NO: 227):

DIQMTQSPSSLSASVGDRVTITCRASENIYSNLAWYQQKPGKSPKLLVYAATN
VADGVPSRFSGSGSGTDYTLTISSLQPEDFATYYCQQFWDTPFTFGQGTKLEIK
RTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGE
C.

The humanized antibody huP2-G9:

The heavy chain (SEQ ID NO: 228):

EVQLQESGPGLVKPSQTLSLTCTVSGSSITSGYYWNWIRQHPGKGLEWIGYLS
YDGSNNYNPSLKNLVTISRDTSKNQFSLKLSSVTAADTAVYYCAREGAHYSGT
LSWFAYWGQGTLVTVSSASTKGPSVFPLAPSSKSTSGGTAALGCLVKDYFPEP
VTVSWNSGALTSGVHTFPAVLQSSGLYSLSSVVTVPSSSLGTQTYICNVNHKPS
NTKVDKKVEPKSCDKTHTCPPCPAPEAAGGPSVFLFPPKPKDTLMISRTPEVTC
VVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRW
QQGNVFSCSVMHEALHNHYTQKSLSLSPGK

The light chain (SEQ ID NO: 229):

DIQMTQSPSSLSASVGDRVTITCRASENIYSNLAWYQQKPGKSPKLLVYAATN
LADGVPSRFSGSGSGTDYTLTISSLQPEDFATYYCQHFWDTPYTFGGGTKVEIK
RTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGE
C.

The humanized antibody huP2-E8:

The heavy chain (SEQ ID NO: 230):

EVQLVESGGGLVKPGGSLRLSCAASGFTFSDYGIHWVRQAPGKGLEWVAYISS
GSSIHNYADTVKGRFTISRDNAKNSLYLQMNSLRAEDTAVYYCARGPNYYGS
SYLLVYWGQGTLVTVSSASTKGPSVFPLAPCSRSTSESTAALGCLVKDYFPEPV

TVSWNSGALTSGVHTFPAVLQSSGLYSLSSVVTVPSSSLGTKTYTCNVDHKPS
NTKVDKRVESKYGPPCPPCPAPEAAGGPSVFLFPPKPKDTLMISRTPEVTCVVV
DVSQEDPEVQFNWYVDGVEVHNAKTKPREEQFNSTYRVVSVLTVLHQDWLN
GKEYKCKVSNKGLPSSIEKTISKAKGQPREPQVYTLPPSQEEMTKNQVSLTCL
VKGFYPSDIAVEWESNGQPENNYKTTPPVLDSDGSFFLYSRLTVDKSRWQEGN
VFSCSVMHEALHNHYTQKSLSLSLGK

The light chain (SEQ ID NO: 231):

DIQMTQSPSSLSASVGDRVTITCKASQDVNIAVAWYQQKPGKAPKLLIYSASY
RYTGVPSRFSGSGSGTDFTFTISSLQPEDIATYYCQQHYNIPWTFGGGTKVEIK
RTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGE
C.

The humanized antibody huP1-G7:

The heavy chain (SEQ ID NO: 232):

EVQLQESGPGLVKPSQTLSLTCTVSGYSITSGYYWNWIRQHPGKGLEWIGYIS
YDGGNNYNPFLKNRVTISRDTSKNQFSLKLSSVTAADTAVYYCAREGGSSWE
YYGMDYWGQGTTVTVSSASTKGPSVFPLAPCSRSTSESTAALGCLVKDYFPEP
VTVSWNSGALTSGVHTFPAVLQSSGLYSLSSVVTVPSSSLGTKTYTCNVDHKP
SNTKVDKRVESKYGPPCPPCPAPEAAGGPSVFLFPPKPKDTLMISRTPEVTCVV
VDVSQEDPEVQFNWYVDGVEVHNAKTKPREEQFNSTYRVVSVLTVLHQDWL
NGKEYKCKVSNKGLPSSIEKTISKAKGQPREPQVYTLPPSQEEMTKNQVSLTC
LVKGFYPSDIAVEWESNGQPENNYKTTPPVLDSDGSFFLYSRLTVDKSRWQEG
NVFSCSVMHEALHNHYTQKSLSLSLGK

The light chain (SEQ ID NO: 233):

DIQMTQSPSSLSASVGDRVTITCRASENIYRNLAWYQQKPGKAPKLLLYAATN
LADGVPSRFSGSGSGTDFTLTISSLQPEDFATYYCQQFWGSPYTFGGGTKVEIK
RTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGE
C.

The humanized antibody huP2-C6:

The heavy chain (SEQ ID NO: 234):

EVQLVQSGAEVKKPGASVKVSCKASGYTFTDYYIHWVRQAPGQGLEWMGYI
YPDTGYSGYNQKFKGRVTMTVDKSTSTAYMELRSLRSDDTAVYYCTRRGDY
SGVHFDYWGQGTTVTVSSASTKGPSVFPLAPSSKSTSGGTAALGCLVKDYFPE
PVTVSWNSGALTSGVHTFPAVLQSSGLYSLSSVVTVPSSSLGTQTYICNVNHKP

SNTKVDKKVEPKSCDKTHTCPPCPAPEAAGGPSVFLFPPKPKDTLMISRTPEVT
CVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRW
QQGNVFSCSVMHEALHNHYTQKSLSLSPGK

The light chain (SEQ ID NO: 235):

DIVLTQSPDSLAVSLGERATINCRASESVNNYGVGFLHWYQQKPGQPPKLLIY RASTLESGIPDRFSGSGSGTDFTLTISSLQAEDVAVYYCQHSYKDPPWTFGGGT KVEIKRTVAAPSVFIFPPSDEQLKSGTASVVCLLNNFYPREAKVQWKVDNALQ SGNSQESVTEQDSKDSTYSLSSTLTLSKADYEKHKVYACEVTHQGLSSPVTKS FNRGEC.

The humanized antibody huP1-D7:

The heavy chain (SEQ ID NO: 236):

EVQLVQSGAEVKKPGASVKVSCKASGYTFTTYGMNWVRQAPGQGLEWMG WINTYSGVPTYAEDFKGRVTMTTDTSTSTAYMELRSLRSDDTAVYYCARRTD YDGYVMDSWGQGTTVTVSSASTKGPSVFPLAPCSRSTSESTAALGCLVKDYF PEPVTVSWNSGALTSGVHTFPAVLQSSGLYSLSSVVTVPSSSLGTKTYTCNVD HKPSNTKVDKRVESKYGPPCPPCPAPEAAGGPSVFLFPPKPKDTLMISRTPEVT CVVVDVSQEDPEVQFNWYVDGVEVHNAKTKPREEQFNSTYRVVSVLTVLHQ DWLNGKEYKCKVSNKGLPSSIEKTISKAKGQPREPQVYTLPPSQEEMTKNQV SLTCLVKGFYPSDIAVEWESNGQPENNYKTTPPVLDSDGSFFLYSRLTVDKSR WQEGNVFSCSVMHEALHNHYTQKSLSLSLGK

The light chain (SEQ ID NO: 237):

DIVMTQSPDSLAVSLGERATINCKSSQSLFYSGNQKNYLAWYQQKPGQSPKLL LYWASTRESGVPDRFSGSGSGTDFTLTISSLQAEDVAVYYCQQYFSYPPTFGGG TKVEIKRTVAAPSVFIFPPSDEQLKSGTASVVCLLNNFYPREAKVQWKVDNAL QSGNSQESVTEQDSKDSTYSLSSTLTLSKADYEKHKVYACEVTHQGLSSPVTK SFNRGEC.

The fully human-derived antibody P2m:

The heavy chain (SEQ ID NO: 238):

QVQLVQSGAEVKKPGSSVKVSCKASGYTFTSYDISWVRQAPGQGLEWMGGII PIFGTANYAQKFQGRVTITADESTSTAYMELSSLRSEDTAVYYCTAGIFLATAYW GQGTLVTVSSASTKGPSVFPLAPSSKSTSGGTAALGCLVKDYFPEPVTVSWNS GALTSGVHTFPAVLQSSGLYSLSSVVTVPSSSLGTQTYICNVNHKPSNTKVDKK VEPKSCDKTHTCPPCPAPEAAGGPSVFLFPPKPKDTLMISRTPEVTCVVVDVSH

EDPEVKFNWYVDGVEVHNAKTKPREEQYNSTYRVVSVLTVLHQDWLNGKE
YKCKVSNKALPAPIEKTISKAKGQPREPQVYTLPPSRDELTKNQVSLTCLVKGF
YPSDIAVEWESNGQPENNYKTTPPVLDSDGSFFLYSKLTVDKSRWQQGNVFSC
SVMHEALHNHYTQKSLSLSPGK

The light chain (SEQ ID NO: 239):

DIQMTQSPSSMSASVGDRVTISCQASQDIQNYLNWYQQRPGQAPKLLIYDAST
LETGVPSRFSGSGSGTYFTLTISSLQPEDVATYYCQQYENVPITFGQGTRLEIKR
TVAAPSVFIFPPSDEQLKSGTASVVCLLNNFYPREAKVQWKVDNALQSGNSQE
SVTEQDSKDSTYSLSSTLTLSKADYEKHKVYACEVTHQGLSSPVTKSFNRGEC

Note: The variable regions are single underlined, and the hIgG1(LALA), hIgG4(LALA), and CL constant regions are not underlined.

The heavy and light chain constant region sequences of the humanized antibodies are shown below:

> hIgG1(LALA) (SEQ ID NO: 240)

ASTKGPSVFPLAPSSKSTSGGTAALGCLVKDYFPEPVTVSWNSGALTSGVHTF
PAVLQSSGLYSLSSVVTVPSSSLGTQTYICNVNHKPSNTKVDKKVEPKSCDKT
HTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNK
ALPAPIEKTISKAKGQPREPQVYTLPPSRDELTKNQVSLTCLVKGFYPSDIAVE
WESNGQPENNYKTTPPVLDSDGSFFLYSKLTVDKSRWQQGNVFSCSVMHEAL
HNHYTQKSLSLSPGK

Note: The CH1 region is single underlined, the hinge region is dotted underlined, and the Fc region is double underlined.
> hIgG4(LALA) (SEQ ID NO: 171)

ASTKGPSVFPLAPCSRSTSESTAALGCLVKDYFPEPVTVSWNSGALTSGVHTFP
AVLQSSGLYSLSSVVTVPSSSLGTKTYTCNVDHKPSNTKVDKRVESKYGPPCP
PCPAPEAAGGPSVFLFPPKPKDTLMISRTPEVTCVVVDVSQEDPEVQFNWYVD
GVEVHNAKTKPREEQFNSTYRVVSVLTVLHQDWLNGKEYKCKVSNKGLPSSI
EKTISKAKGQPREPQVYTLPPSQEEMTKNQVSLTCLVKGFYPSDIAVEWESNG
QPENNYKTTPPVLDSDGSFFLYSRLTVDKSRWQEGNVFSCSVMHEALHNHYT
QKSLSLSLGK

Note: The CH1 region is single underlined, the hinge region is dotted underlined, and the Fc region is double underlined.
> CL (SEQ ID NO: 172)

RTVAAPSVFIFPPSDEQLKSGTASVVCLLNNFYPREAKVQWKVDNALQSGNSQ ESVTEQDSKDSTYSLSSTLTLSKADYEKHKVYACEVTHQGLSSPVTKSFNRGE C

The positive control molecules used in the present disclosure were MK and JTX.

The source of the sequences of MK: WO2018187518A1

The amino acid sequences of the theoretical light and heavy chains of MK are shown below (the variable regions are single underlined, the CDRs are double underlined, and the constant regions are dotted underlined):

> The heavy chain (SEQ ID NO: 241):

EVQLQQWGAGLLKPSETLSLTCAVYGGSFSGYYWSWIRQPPGKGLEWIGEIN HAGSTNYNPSLKSRVTISVDTSKNQFSLKLSSVTAADTAVYYCARLPTRWVTT RYFDLWGRGTLVTVSSASTKGPSVFPLAPCSRSTSESTAALGCLVKDYFPEPVT VSWNSGALTSGVHTFPAVLQSSGLYSLSSVVTVPSSSLGTKTYTCNVDHKPSN TKVDKRVESKYGPPCPPCPAPEFLGGPSVFLFPPKPKDTLMISRTPEVTCVVVD VSQEDPEVQFNWYVDGVEVHNAKTKPREEQFNSTYRVVSVLTVLHQDWLNG KEYKCKVSNKGLPSSIEKTISKAKGQPREPQVYTLPPSQEEMTKNQVSLTCLV KGFYPSDIAVEWESNGQPENNYKTTPPVLDSDGSFFLYSRLTVDKSRWQEGNV FSCSVMHEALHNHYTQKSLSLSLGK

> The light chain (SEQ ID NO: 242):

ESVLTQPPSVSGAPGQRVTISCTGSSSNIGAGYDVHWYQQLPGTAPKLLIYGDS NRPSGVPDRFSVSKSGASASLAITGLQAEDEADYYCQSFDNSLSAYVFGGGTQ LTVLGQPKANPTVTLFPPSSEELQANKATLVCLISDFYPGAVTVAWKADGSPV KAGVETTKPSKQSNNKYAASSYLSLTPEQWKSHRSYSCQVTHEGSTVEKTVA PTECS

The source of the sequences of JTX: WO2019126514A2

The amino acid sequences of the theoretical light and heavy chains of JTX are shown below (the variable regions are single underlined, the CDRs are double underlined, and the constant regions are dotted underlined):

> The heavy chain (SEQ ID NO: 243):

QITLKESGPTLVKPTQTLTLTCTFSGFSLNTYAMGVSWIRQPPGKALEWLASIW WNGNKYNNPSLKSRLTVTKDTSKNQVVLTMTNMDPVDTATYYCAHSRIIRFT DYVMDAWGQGTLVTVSSASTKGPSVFPLAPCSRSTSESTAALGCLVKDYFPEP VTVSWNSGALTSGVHTFPAVLQSSGLYSLSSVVTVPSSSLGTKTYTCNVDHKP

SNTKVDKRVESKYGPPCPPCPAPEFLGGPSVFLFPPKPKDTLMISRTPEVTCVV
VDVSQEDPEVQFNWYVDGVEVHNAKTKPREEQFNSTYRVVSVLTVLHQDWL
NGKEYKCKVSNKGLPSSIEKTISKAKGQPREPQVYTLPPSQEEMTKNQVSLTC
LVKGFYPSDIAVEWESNGQPENNYKTTPPVLDSDGSFFLYSRLTVDKSRWQEG
NVFSCSVMHEALHNHYTQKSLSLSLGK

> The light chain (SEQ ID NO: 244):

DIQMTQSPSSLSTSVGDRVTITCRASEDIYNDLAWYQQKPGKAPKLLIYNANS
LHTGVASRFSGSGSGTDFTFTISSLQPEDVATYFCQQYYDYPLTFGQGTKLEIK
RTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGE
C.

Cells overexpressing HLA-G

MVVMAPRTLFLLLSGALTLTETWA*GSHSMRYFSAAVSRPGRGEPRFIAMGYVDD*
*TQFVRFDSDSACPRMEPRAPWVEQEGPEYWEEETRNTKAHAQTDRMNLQTLRGY*
*YNQSEASSHTLQWMIGCDLGSDGRLLRGYEQYAYDGKDYLALNEDLRSWTAADTA*
*AQISKRKCEAANVAEQRRAYLEGTCVEWLHRYLENGKEMLQRADPPKTHVTHHPV*
*FDYEATLRCWALGFYPAEIILTWQRDGEDQTQDVELVETRPAGDGTFQKWAAVVV*
*PSGEEQRYTCHVQHEGLPEPLMLRWKQSSLPTIPI*MGIVAGLVVLAAVVTGAAV
AAVLWRKKSSD

(SEQ ID NO: 247)

Note: The signal peptide is single underlined, the extracellular domain is italicized, the transmembrane domain is dotted underlined, and the intracellular domain is double underlined.

**Example 5: ELISA Protein Binding Assays of Anti-ILT4 Antibodies**

Human ILT4-His protein was diluted to 1 $\mu$g/mL with PBS, and a 96-well plate (Corning, CLS3590-100EA) was coated with the diluted protein and incubated overnight at 4 °C. The plate was washed and then blocked with 1% BSA at 37 °C for 1 h. The plate was washed, and the anti-ILT4 antibodies, at different concentrations, were then added. The plate was incubated at 37 °C for 1 h. The plate was washed, and an HRP-goat anti-human F(ab')2 secondary antibody (Jackson, 109-036-097) was then added. The plate was incubated at 37 °C for 1 h. The plate was washed, and the TMB chromogenic substrate (KPL, 52-00-03) was then added. The plate was incubated at room temperature for 5-10 min, and 1 M $H_2SO_4$ was added to stop the reaction. The absorbance was measured at a wavelength of 450 nm using a VERSAmax microplate reader (Molecular Devices). The results are shown in Table 22.

Table 22. The abilities of the anti-ILT4 antibodies to bind to ILT4 protein

| Assay 1 | | |
|---|---|---|
| Antibody | $EC_{50}$ (ng/mL) | Top |
| ChP1-C4 | 64.26 | 1.983 |
| JTX | 82.63 | 1.697 |

(continued)

| Assay 2 | | |
|---|---|---|
| Antibody | EC$_{50}$ (ng/mL) | Top |
| ChP2-E7 | 72.90 | 2.004 |
| ChP2-G4 | 62.09 | 1.829 |
| ChP2-E11 | 42.80 | 1.984 |
| JTX | 82.63 | 1.697 |
| Assay 3 | | |
| Antibody | EC$_{50}$ (ng/mL) | Top |
| ChP1-D7 | 29.23 | 2.071 |
| JTX | 29.34 | 1.426 |
| Assay 4 | | |
| Antibody | EC$_{50}$ (ng/mL) | Top |
| ChP1-E11 | 40.96 | 1.722 |
| JTX | 82.97 | 1.225 |
| Assay 5 | | |
| Antibody | EC$_{50}$ (ng/mL) | Top |
| ChP2-B9 | 48.66 | 1.530 |
| ChP2-C2 | 49.28 | 1.157 |
| ChP2-E10 | 65.82 | 1.646 |
| JTX | 80.10 | 1.144 |

The experimental results show that all the anti-ILT4 chimeric antibodies exhibited higher abilities to bind to ILT4 protein than the control antibody JTX.

**Example 6: Biacore Protein Binding Assays of Anti-ILT4 Antibodies**

The affinities of the humanized antibodies of the present disclosure for ILT4-His protein were determined by Biacore. The antibodies were affinity-captured using a Protein A biosensor chip (Cytiva, 29127556), and ILT4-His was then allowed to flow through the surface of the chip. Reaction signals were detected in real time using an instrument to obtain binding and dissociation curves. After the dissociation in each experimental cycle was complete, the biosensor chip was washed with 10 mM Glycine-HCl pH 1.5 (Cytiva, BR-1003-54) for regeneration. Data fitting was performed using a 1:1 model. The binding and dissociation of each antibody are shown in Table 23.

Table 23. The binding affinities of the anti-ILT4 antibodies for ILT4 protein

| Antibody | ka (1/Ms) | kd (1/s) | KD (M) |
|---|---|---|---|
| huP2-E3 | 1.01E+06 | 1.27E-03 | 1.26E-09 |
| huP2-G9 | 9.99E+05 | 1.09E-04 | 1.09E-10 |
| huP2-E8 | 2.22E+05 | 1.86E-04 | 8.39E-10 |
| huP1-G7 | 4.57E+05 | 7.20E-05 | 1.58E-10 |
| huP2-C6 | 1.21E+06 | 6.58E-05 | 5.43E-11 |
| huP1-D7 | 2.59E+06 | 1.03E-02 | 3.98E-09 |
| P2m | 2.03E+06 | 8.78E-05 | 4.33E-11 |
| MK | 4.52E+05 | 8.63E-03 | 1.91E-08 |

The experimental results show that the humanized and fully human-derived antibodies to which the present disclosure relates exhibited higher abilities to bind to ILT4 protein than the control antibody.

**Example 7: FACS Cell Binding Assays of Anti-ILT4 Antibodies**

The affinities of the humanized antibodies of the present disclosure for CHO-S/ILT4 cells were determined by FACS. $1 \times 10^6$ cells/mL CHO-S/ILT4 cells were blocked with 1% BSA and then incubated with the anti-ILT4 antibodies, at different concentrations, at 4 °C for 1 h. The cells were washed twice with 1% BSA and then incubated with a F(ab')2-goat anti-human IgG Fc secondary antibody (PE, Invitrogen, H10104) at 4 °C for 0.5 h. The cells were washed twice with 1% BSA, and fluorescence signal values were then obtained using FACS. The results are shown in Table 24.

Table 24. The abilities of the anti-ILT4 antibodies to bind to the ILT4 stably transfected cell strain

| Assay 1 | | |
|---|---|---|
| Antibody | EC$_{50}$ (μg/mL) | Top |
| huP2-E3 | 0.03067 | 25986 |
| MK | 0.03103 | 25142 |
| Assay 2 | | |
| Antibody | EC$_{50}$ (ng/mL) | Top |
| huP2-G9 | 0.04500 | 27255 |
| MK | 0.04151 | 22456 |
| Assay 3 | | |
| Antibody | EC$_{50}$ (ng/mL) | Top |
| huP2-C6 | 0.03678 | 29886 |
| MK | 0.30103 | 25142 |
| Assay 4 | | |
| Antibody | EC$_{50}$ (ng/mL) | Top |
| P2m | 0.01633 | 20764 |
| MK | 0.01388 | 18465 |

The experimental results show that the humanized and fully human-derived antibodies to which the present disclosure relates exhibited comparable or higher abilities to bind to ILT4-positive cells than the control antibody.

**Example 8: Anti-ILT4 Antibodies Do Not Bind to Other LILR Family Members**

Human ILT2-His (prepared by the same method as the ILT4-His in Examples 1 and 2; whose amino acid sequence is set forth in SEQ ID NO: 245), ILT3-His (prepared by the same method as the ILT4-His in Examples 1 and 2; whose amino acid sequence is set forth in SEQ ID NO: 246), ILT4-His (prepared by the same method as in Examples 1 and 2), LILRB5-His (Acro, CDC-H5220), LILRA1-His (Acro, LI1-H82E8), LILRA2-His (Acro, LI2-H82E9), LILRA4-His (Sino-Biological, 16058-H08H), LILRA5-His (Acro, LI5-H82E1), LILRA6-His (Acro, LI6-H82E2), or LILRA3-His (Acro, LI3-H82E0) was diluted to 1 μg/mL with PBS, and a 96-well plate (Corning, CLS3590-100EA) was coated with the diluted protein and incubated overnight at 4 °C. The plate was washed and then blocked with 1% BSA at 37 °C for 1 h. The plate was washed, and the anti-ILT4 antibodies, at 10 μg/mL, were then added. The plate was then incubated at 37 °C for 1 h. The plate was washed, and an HRP-goat anti-human F(ab')2 secondary antibody (Jackson, 109-036-097) was then added. The plate was incubated at 37 °C for 1 h. The plate was washed, and the TMB chromogenic substrate (KPL, 52-00-03) was then added. The plate was incubated at room temperature for 5-10 min, and 1 M $H_2SO_4$ was added to stop the reaction. The absorbance was measured at a wavelength of 450 nm using a VERSAmax microplate reader (Molecular Devices). The results are shown in Table 25.

His-tagged ILT2 protein extracellular domain: ILT2-His

*GHLPKPTLWAEPGSVITQGSPVTLRCQGGQETQEYRLYREKKTALWITRIPQELVK*
*KGQFPIPSITWEHAGRYRCYYGSDTAGRSESSDPLELVVTGAYIKPTLSAQPSPVVNS*
*GGNVILQCDSQVAFDGFSLCKEGEDEHPQCLNSQPHARGSSRAIFSVGPVSPSRR*
*WWYRCYAYDSNSPYEWSLPSDLLELLVLGVSKKPSLSVQPGPIVAPEETLTLQCGSD*
*AGYNRFVLYKDGERDFLQLAGAQPQAGLSQANFTLGPVSRSYGGQYRCYGAHNLS*
*SEWSAPSDPLDILIAGQFYDRVSLSVQPGPTVASGENVTLLCQSQGWMQTFLLTKE*
*GAADDPWRLRSTYQSQKYQAEFPMGPVTSAHAGTYRCYGSQSSKPYLLTHPSDPL*
*ELVVSGPSGGPSSPTTGPTSTSGPEDQPLTPTGSDPQSGLGRHLGV*<u>HHHHHH</u>

(SEQ ID NO: 245)

Note: The extracellular domain is italicized, and the His tag is single underlined.

His-tagged ILT3 protein extracellular domain: ILT3-His

*QAGPLPKPTLWAEPGSVISWGNSVTIWCQGTLEAREYRLDKEESPAPWDRQNPLE*
*PKNKARFSIPSMTEDYAGRYRCYYRSPVGWSQPSDPLELVMTGAYSKPTLSALPSPL*
*VTSGKSVTLLCQSRSPMDTFLLIKERAAHPLLHLRSEHGAQQHQAEFPMSPVTSVH*
*GGTYRCFSSHGFSHYLLSHPSDPLELIVSGSLEDPRPSPTRSVSTAAGPEDQPLMPT*
*GSVPHSGLRRHWE*<u>HHHHHH</u>

(SEQ ID NO: 246)

Note: The extracellular domain is italicized, and the His tag is single underlined.

Table 25. The abilities of the anti-ILT4 antibodies to bind to LILR family member proteins

| Antibody | ILT2 | ILT3 | ILT4 | LILRB5 | LILRA 1 | LILRA2 | LILRA3 | LILRA4 | LILRA5 | LILRA6 |
|---|---|---|---|---|---|---|---|---|---|---|
| huP2-E3 | 0.0541 | 0.0699 | 1.0428 | 0.0523 | 0.0532 | 0.0550 | 0.0476 | 0.0605 | 0.0466 | 0.0470 |
| huP2-G9 | 0.0541 | 0.0564 | 1.7672 | 0.0533 | 0.0457 | 0.0759 | 0.0461 | 0.0674 | 0.0441 | 0.0449 |
| huP2-C6 | 0.0621 | 0.1495 | 2.6914 | 0.0563 | 0.0535 | 0.0565 | 0.0613 | 0.0605 | 0.0598 | 0.0606 |
| huP2-E8 | 0.0569 | 0.0630 | 0.9543 | 0.0489 | 0.0483 | 0.0516 | 0.0467 | 0.0504 | 0.0467 | 0.0461 |
| P2m | 0.0517 | 0.0671 | 1.8701 | 0.0663 | 0.0469 | 0.0448 | 0.0468 | 0.0543 | 0.0459 | 0.0453 |

The experimental results show that none of the humanized and fully human-derived antibodies to which the present disclosure relates bound to other LILR family members.

**Example 9: Blocking Effects of Anti-ILT4 Antibodies on Binding of ILT4 to HLA-**G

The cell-blocking activity of the anti-ILT4 antibodies was measured by flow cytometry. $1 \times 10^6$ cells/mL CHO-S/ILT4 cells were blocked with 1% BSA and then incubated with the anti-ILT4 antibodies, diluted with 1% BSA to different concentrations, and an HLA-G tetramer (novoprotein) labeled with CF647 (SIGMA, MX647S100-1KT) at 4 °C for 1 h. After 4 min of centrifugation at 500 g, the supernatant was discarded. The cells were washed twice with 1% BSA, and fluorescence signal values were then obtained using FACS.

Table 26. The abilities of the anti-ILT4 antibodies to block the binding of ILT4 to HLA-G

| Assay 1 | |
|---|---|
| Antibody | $IC_{50}$ ($\mu$g/mL) |
| huP2-E3 | 0.012 |
| JTX | 0.022 |
| MK | 0.031 |
| Assay 2 | |
| Antibody | $IC_{50}$ ($\mu$g/mL) |
| huP2-G9 | 0.085 |
| JTX | 0.120 |
| MK | 0.138 |
| Assay 3 | |
| Antibody | $IC_{50}$ ($\mu$g/mL) |
| huP2-C6 | 0.072 |
| JTX | 0.120 |
| MK | 0.138 |
| Assay 4 | |
| Antibody | $IC_{50}$ ($\mu$g/mL) |
| P2m | 0.033 |
| JTX | 0.061 |
| MK | 0.092 |

The experimental results show that the antibodies huP2-E3, huP2-G9, huP2-C6, and P2m, to which the present disclosure relates, all effectively blocked the binding of ILT4 to HLA-G, and their blocking activity was stronger than that of the control antibodies MK and JTX.

### Example 10: Blocking Effects of Anti-ILT4 Antibodies on ILT4 and Its Downstream Signaling Pathways

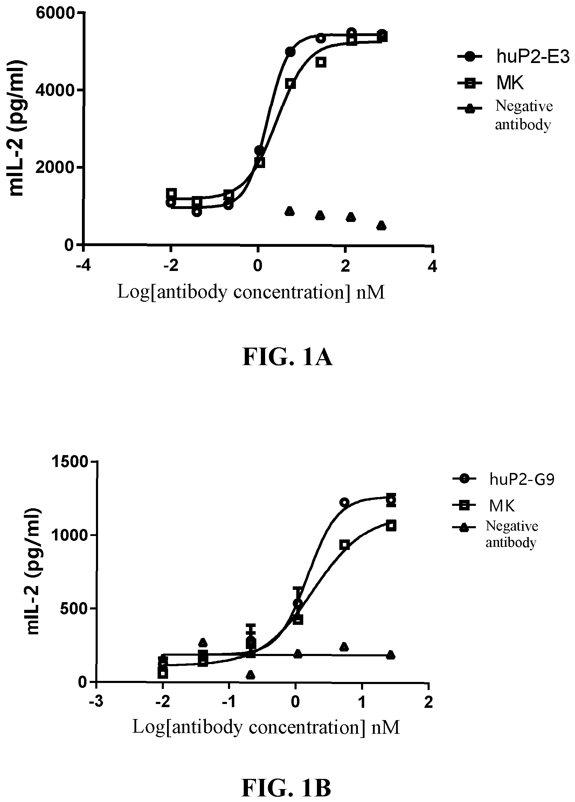

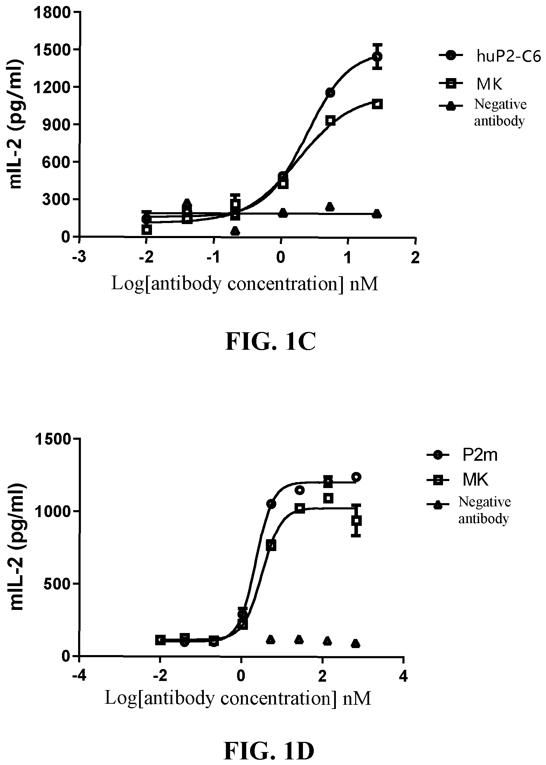

The abilities of the anti-ILT4 antibodies to block ILT4 were determined through a 3A9/ILT4 reporter system. 1 $\mu$g/mL mCD3 antibody (BioLegend, 100238) was inoculated into a 96-well plate (Corning, 3599). The plate was incubated at 37 °C for 2 h, and the supernatants were then discarded. 3A9 cells (ATCC, CRL-3293) overexpressing full-length ILT4 protein were collected and resuspended in RPMI 1640 (MeilunBio, PWL004) + 5% FBS (Gibco, 10099-141) + 0.05 mM 2-B mercaptoethanol (Sigma, M3148-25mL), and the density was adjusted to $8.3 \times 10^5$ cells/mL. The cell suspension was then added to the above 96-well plate. Meanwhile, the anti-ILT4 antibodies, at different concentrations, were added, and the plate was incubated at 37 °C with 5% $CO_2$ for 24 h. The cell supernatants were collected after 24 h. mIL-2 secretion was measured using an mIL-2 kit (NeoBioscience, EMC002.96.2). The results are shown in FIGs. 1A-1D.

The experimental results show that all the antibodies to which the present disclosure relates removed the inhibitory effect of ILT4 more effectively and promoted the secretion of higher levels of mIL-2 by 3A9 cells than the control antibody MK.

### Example 11: Effects of Anti-ILT4 Antibodies on Cytokine Secretion by Macrophages

CD14[+] monocytes were isolated from healthy human PBMCs using CD14 MicroBeads (Miltenyi Biotec, 130-050-201). The CD14[+] cells were resuspended in RPMI 1640 (MeilunBio, PWL004) + 10% FBS (Gibco, 10099-141) + 50 ng/mL M-CSF (Peprotech, 300-25), and the cell density was adjusted to $2 \times 10^6$ cells/mL. After 7 days of differentiation, macrophages were obtained. The macrophages were resuspended in RPMI 1640 + 10% FBS, and the density was adjusted to $1 \times 10^6$ cells/mL. The macrophages were then inoculated into a 96-well plate (Jet, 3788). Meanwhile, LPS (sigma, L3024-5MG) and the anti-ILT4 antibodies, at different concentrations, were added, and the plate was incubated at 37 °C with 5% $CO_2$ for 24 h. The cell supernatants were collected and assayed for TNF$\alpha$ using a HUMAN

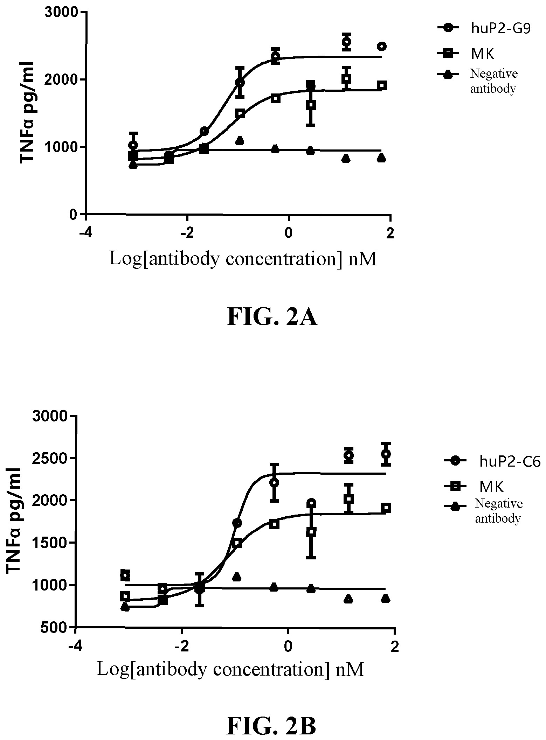

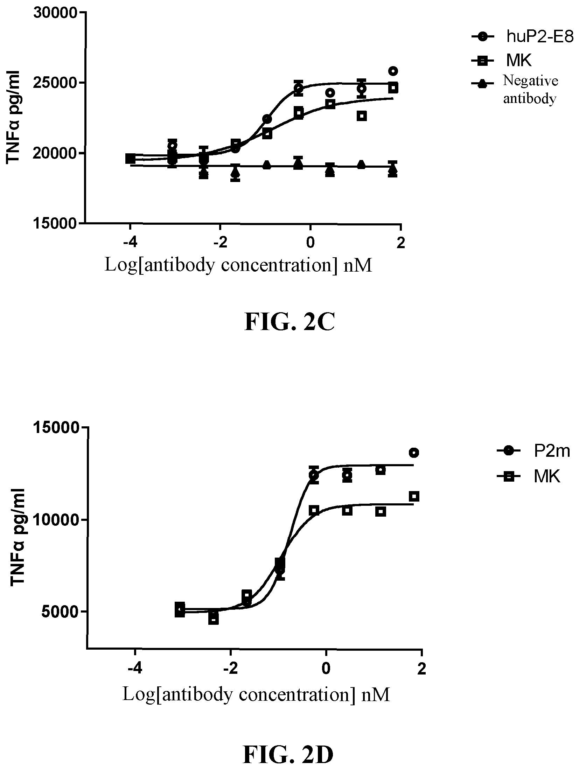

TNFα KIT (Univ, 62HTNFAPEG). The results are shown in FIGs. 2A-2D.

The experimental results show that all the antibodies to which the present disclosure relates more effectively promoted the transformation of macrophages to the M1 phenotype and the secretion of higher levels of the proinflammatory cytokine TNF-α than the control antibody MK.

## Example 12: Combined Effects of Anti-ILT4 Antibodies and Anti-PD-1 Antibody in Allogeneic MLR

CD14+ monocytes were isolated from healthy human PBMCs using CD14 MicroBeads (Miltenyi Biotec, 130-050-201). The CD14+ cells were resuspended in RPMI 1640 (MeilunBio, PWL004) + 10% FBS (Gibco, 10099-141) + 50 ng/mL M-CSF (Peprotech, 300-25), and the cell density was adjusted to $2 \times 10^6$ cells/mL. After 7 days of differentiation, macrophages were obtained. The macrophages were resuspended in RPMI 1640 + 10% FBS, and the density was adjusted to $2 \times 10^5$ cells/mL. The macrophages were then inoculated into a 96-well plate (Jet, 3788).

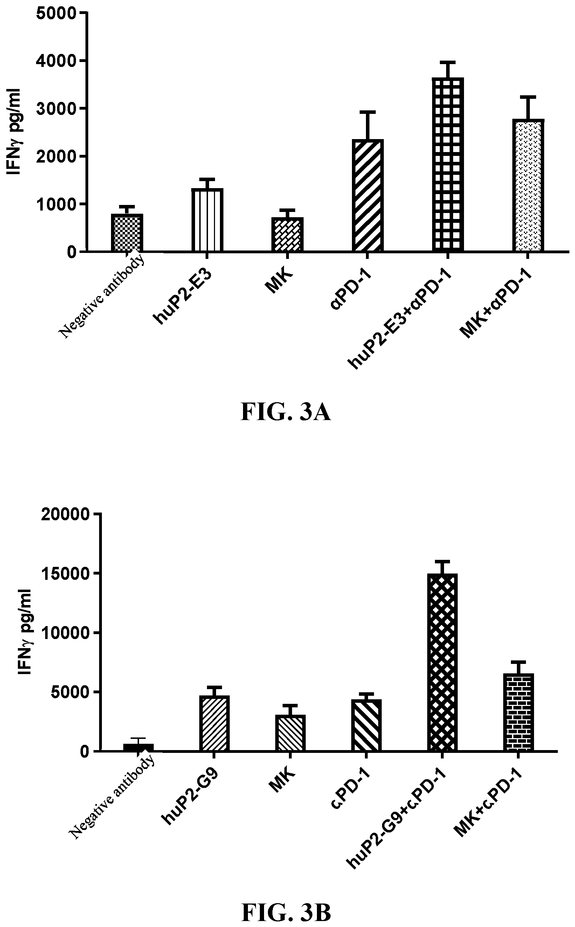

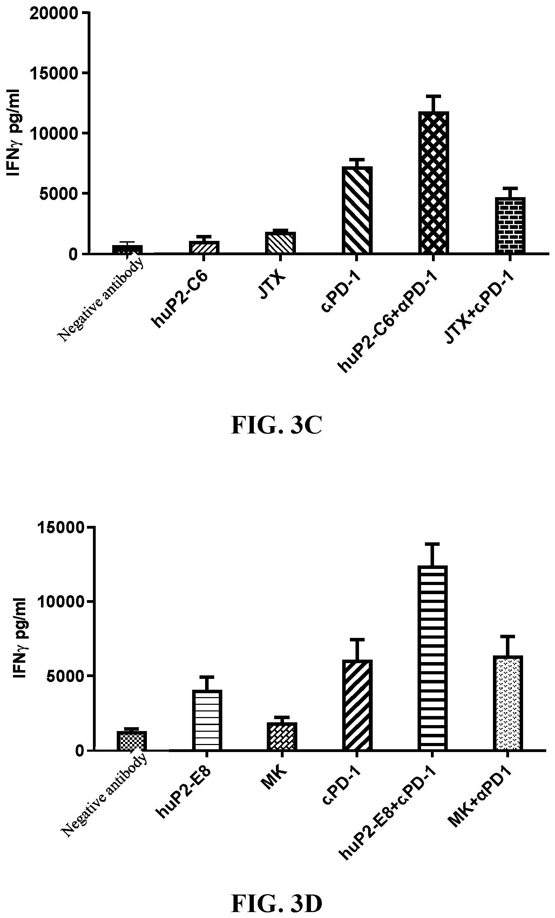

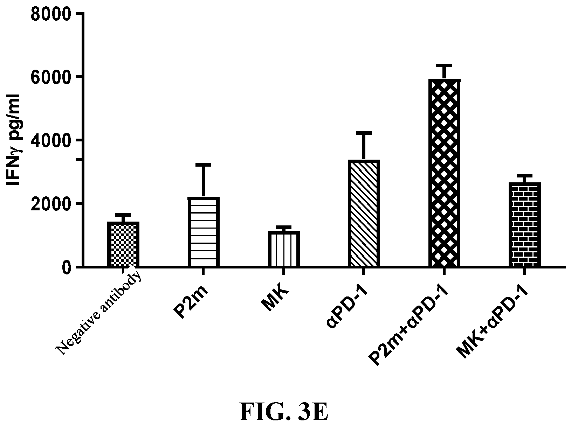

Pan T cells were isolated from PBMCs from another donor using a T cell sorting kit (Miltenyi, 130-096-535) and resuspended in RPMI 1640 + 10% FBS, and the density was adjusted to $2 \times 10^6$ cells/mL. The cell suspension was then added to the above 96-well plate. Meanwhile, the anti-ILT4 antibodies and/or the anti-PD-1 antibody Hu23-11.IgG4AA (prepared with reference to the patent WO2020156509 A1; its sequences are shown below) were added to a final concentration of 10 μg/mL, and the plate was incubated at 37 °C with 5% $CO_2$ for 6 d. The cell supernatants were collected and assayed for IFNγ using a HUMAN IFNγ KIT (Univ, 62HIFNGPEG). The results are shown in FIGs. 3A-3E.

The full-length sequence of the anti-PD-1 antibody Hu23-11.IgG4AA:

The heavy chain (SEQ ID NO: 248):

EVQLVQSGAEVKKPGSSVKVSCKASGGTFSDYEMHWVRQAPGQGLEWMGLI
DPETGGTVYNQKFKDRVTITADKSTSTAYMELSSLRSEDTAVYYCARERFSYY
GSTSDWYFDVWGQGTTVTVSSASTKGPSVFPLAPCSRSTSESTAALGCLVKD
YFPEPVTVSWNSGALTSGVHTFPAVLQSSGLYSLSSVVTVPSSSLGTKTYTCNV
DHKPSNTKVDKRVESKYGPPCPPCPAPEAAGGPSVFLFPPKPKDTLMISRTPEV
TCVVVDVSQEDPEVQFNWYVDGVEVHNAKTKPREEQFNSTYRVVSVLTVLH
QDWLNGKEYKCKVSNKGLPSSIEKTISKAKGQPREPQVYTLPPSQEEMTKNQ
VSLTCLVKGFYPSDIAVEWESNGQPENNYKTTPPVLDSDGSFFLYSRLTVDKSR
WQEGNVFSCSVMHEALHNHYTQKSLSLSLGK

The light chain (SEQ ID NO: 249):

DIVMTQTPLSLPVTPGEPASISCRSSQSLVHSTGNTYLEWYLQKPGQSPQLLIY
KVSNRFSGVPDRFSGSGSGTDFTLKISRVEAEDVGVYYCFQGSHVPYTFGGGT
KVEIKRTVAAPSVFIFPPSDEQLKSGTASVVCLLNNFYPREAKVQWKVDNALQ
SGNSQESVTEQDSKDSTYSLSSTLTLSKADYEKHKVYACEVTHQGLSSPVTKS
FNRGEC

Table 27. The CDRs of the anti-PD-1 antibody Hu23-11.IgG4AA

|  |  | Heavy chain |  | Light chain |
|---|---|---|---|---|
| PD-1 | HCDR1 | DYEMH (SEQ ID NO: 250) | LCDR1 | RSSQSLVHSTGNTYLE (SEQ ID NO: 253) |

(continued)

| | Heavy chain | | Light chain | |
|---|---|---|---|---|
| HCDR2 | LIDPETGGTVYNQKFKD (SEQ ID NO: 251) | LCDR2 | KVSNRFS (SEQ ID NO: 254) | |
| HCDR3 | ERFSYYGSTSDWYFDV (SEQ ID NO: 252) | LCDR3 | FQGSHVPYT (SEQ ID NO: 255) | |

The variable region sequences of the anti-PD-1 antibody Hu23-11.IgG4AA:

PD-1 VH (SEQ ID NO: 256):

EVQLVQSGAEVKKPGSSVKVSCKASGGTFSDYEMHWVRQAPGQGLEWMGLI
DPETGGTVYNQKFKDRVTITADKSTSTAYMELSSLRSEDTAVYYCARERFSYY
GSTSDWYFDVWGQGTTVTVSS

PD-1 VL (SEQ ID NO: 257):

DIVMTQTPLSLPVTPGEPASISCRSSQSLVHSTGNTYLEWYLQKPGQSPQLLIY
KVSNRFSGVPDRFSGSGSGTDFTLKISRVEAEDVGVYYCFQGSHVPYTFGGGT
KVEIK.

The experimental results show that in the MLR experiment, the combinations of the antibodies of the present disclosure and the anti-PD-1 antibody were significantly more effective in promoting the release of INFγ than the combination of the positive antibody MK or JTX and the anti-PD-1 antibody.

**Example 13: Roles of Anti-ILT4 Antibodies in Promoting Dendritic Cell Activation**

CD14$^+$ monocytes were isolated from healthy human PBMCs using CD14 MicroBeads (Miltenyi Biotec, 130-050-201). The CD14$^+$ cells were resuspended in RPMI 1640 (MeilunBio, PWL004) + 10% FBS (Gibco, 10099-141) + 100 ng/mL GM-CSF (Peprotech, 300-03-100 μg) + 100 ng/mL IL-4 (R&D, 217-IL-010). After 5-6 days of differentiation, dendritic cells were obtained. The dendritic cell density was adjusted to $1 \times 10^6$ cells/mL, and the dendritic cells were inoculated into a 96-well plate (Jet, 3788). Meanwhile, IL-10 (R&D, 217-IL-010) was added to a final concentration of 10 ng/mL, and the anti-ILT4 antibodies were added to a final concentration of 10 μg/mL. The plate was incubated at 37 °C with 5% CO$_2$ for 48 h. The cell supernatants were collected and assayed for CCL-2 (NeoBioscience, EHC.113.96.2) and IL-6 (Cisbio, 62HIL06PEG) using an ELISA kit. The upregulation of cytokines or chemokines is shown in Table 28.

Table 28. The abilities of the anti-ILT4 antibodies to induce dendritic cells to secrete proinflammatory cytokines and chemokines

| Antibody | CCL-2 (pg/mL) | IL-6 (pg/mL) |
|---|---|---|
| huP2-G9 | 3446.3863 | 438.9343 |
| MK | 2043.0930 | 322.2405 |
| Negative antibody | 429.1580 | 56.0929 |
| PBS | 593.5481 | 58.7046 |

The experimental results show that in the immunosuppressive environment with IL-10, the humanized antibody huP2-G9, to which the present disclosure relates, still induced dendritic cells to secrete higher levels of proinflammatory cytokines or chemokines.

**Example 14: Inhibitory Effects of Anti-ILT4 Antibodies Against Subcutaneously Grafted Tumors in Mice Bearing Human Breast Cancer MDA-MB-231/HLA-G**

HLA-G-overexpressing MDA-MB-231 cells (ATCC, HTB-26) in the logarithmic growth phase ($3 \times 10^6$), T cells ($5 \times 10^5$), and MØ cells ($5 \times 10^5$) were mixed in a ratio of 6:1:1, and 200 µL of the mixture (containing 100 µL of matrigel) was subcutaneously inoculated into the right flank of each mouse (Vital River). Each group contained 6-8 mice. The vehicle control group (the vehicle group) was not inoculated with MØ and T cells. The grouping day was defined as day 0 of the experiment. The stimulation of the remaining PBMCs was stopped, and the culture was continued. After 1 week, the cells were intraperitoneally injected into tumor-bearing mice at $5 \times 10^6$ cells/200 µL/mouse. The anti-ILT4 antibodies were intraperitoneally injected twice a week from day 0 at a dose of 20 mpk. The tumor volume was monitored twice a week, and data were recorded.

The tumor volume of each animal group was expressed as mean $\pm$ standard error of the mean (Mean $\pm$ SEM), and plotting was performed using Graphpad Prism 8 software. Statistical analysis was performed using two-way ANOVA.

The calculation formula for tumor volume (V) was: $V = 1/2 \times a \times b^2$, where a and b represent length and width, respectively.

Relative tumor proliferation rate T/C (%) = (T - T0)/(C - C0) $\times$ 100%, where T and C represent the tumor volumes of the treatment group and the control group at the end of the experiment; T0 and C0 represent the tumor volumes at the beginning of the experiment.

$$\text{Tumor growth inhibition rate TGI (\%)} = 1 - \text{T/C (\%)}.$$

The tumor growth inhibition rate of each group is shown in Table 29 below.

Table 29. The tumor growth inhibition rate of each group relative to the negative antibody group

| Group | Number of animals | Frequency of administration | Route of administration | 7-day tumor growth inhibition rate% | 10-day tumor growth inhibition rate% | 13-day tumor growth inhibition rate% |
|---|---|---|---|---|---|---|
| PBS vehicle control group | 8 | biw*3w | ip | - | - | - |
| T + MØ + negative antibody-20 mpk | 6 | biw*3w | ip | - | - | - |
| T+MØ +MK-20mpk | 8 | biw*3w | ip | 13.3 | 16.0 | 19.6 |
| T+MØ+huP2-G9-20mpk | 8 | biw*3w | ip | 15.3 | 11.8 | 22.2 |
| Note: biw stands for "twice a week", 3w stands for "three weeks", and ip stands for "intraperitoneal injection". | | | | | | |

The experimental results show that compared to the PBS vehicle control group (the vehicle group), the tumor growth inhibition rates of the T + MØ + negative antibody-20 mpk group, the MK-20 mpk group, and the huP2-G9-20 mpk group on day 13 were 33.0%, 46.2%, and 47.9%, respectively; compared to the control group, the T + MØ + negative antibody-20 mpk group, the tumor growth inhibition rates of the MK-20 mpk group and the huP2-G9-20 mpk group on day 13 were 19.6% and 22.2%, respectively; the huP2-G9 monotherapy group was comparable in efficacy to the positive MK monotherapy group and even exhibited a higher TGI value.

**Example 15: Inhibitory Effects of Anti-ILT4 Antibodies Against Subcutaneously Grafted Tumors in ILT4-ILT5 Double Transgenic Mice Bearing Mouse Colon Cancer MC38/HLA-G**

100 µL of a suspension of MC38 cells (Cobioer, CBP60825) overexpressing HLA-G (the same as in Example 9) ($5 \times 10^5$ cells) was subcutaneously inoculated into the right flanks of human ILT4-ILT5 double transgenic mice. Mice with a tumor volume of about 80-100 mm³ were selected and randomly divided into groups of 8. The anti-ILT4 antibodies and the anti-PD-1 antibody (made in-house; the amino acid sequences of the heavy and light chains of the anti-PD-1 antibody ($\alpha$PD-1) are set forth in SEQ ID NO: 258 and SEQ ID NO: 259, respectively) were intraperitoneally injected twice a week at doses of 30 mpk and 0.3 mpk, respectively. The following day after the grouping, administration was performed, and that day was defined as day 0. The tumor volume was monitored twice a week, and data were recorded.

The tumor volume of each animal group was expressed as mean $\pm$ standard error of the mean (Mean $\pm$ SEM), and plotting was performed using Graphpad Prism 8 software. Statistical analysis was performed using two-way ANOVA.

The calculation formula for tumor volume (V) was: $V = 1/2 \times a \times b^2$, where a and b represent length and width, respectively.

Relative tumor proliferation rate T/C (%) = (T - T0)/(C - C0) $\times$ 100%, where T and C represent the tumor volumes of the treatment group and the control group at the end of the experiment; T0 and C0 represent the tumor volumes at the beginning of the experiment.

$$\text{Tumor growth inhibition rate TGI (\%)} = 1 - \text{T/C (\%)}.$$

The tumor growth inhibition rate of each group is shown in Table 30 below.

The heavy chain (SEQ ID NO: 258):

EVRLLESGGGLVKPEGSLKLSCVASGFTFSDYFMSWVRQAPGKGLEWVAHIY
TKSYNYATYYSGSVKGRFTISRDDSRSMVYLQMNNLRTEDTATYYCTRDGSG
YPSLDFWGQGTQVTVSSASTKGPSVFPLAPCSRSTSESTAALGCLVKDYFPEP
VTVSWNSGALTSGVHTFPAVLQSSGLYSLSSVVTVPSSSLGTKTYTCNVDHKP
SNTKVDKRVESKYGPPCPPCPAPEFLGGPSVFLFPPKPKDTLMISRTPEVTCVV
VDVSQEDPEVQFNWYVDGVEVHNAKTKPREEQFNSTYRVVSVLTVLHQDWL
NGKEYKCKVSNKGLPSSIEKTISKAKGQPREPQVYTLPPSQEEMTKNQVSLTC
LVKGFYPSDIAVEWESNGQPENNYKTTPPVLDSDGSFFLYSRLTVDKSRWQEG
NVFSCSVMHEALHNHYTQKSLSLSLGK

The light chain (SEQ ID NO: 259):

YELTQPPSASVNVGETVKITCSGDQLPKYFADWFHQRSDQTILQVIYDDNKRP
SGIPERISGSSSGTTATLTIRDVRAEDEGDYYCFSGYVDSDSKLYVFGSGTQLTV
LRTVAAPSVFIFPPSDEQLKSGTASVVCLLNNFYPREAKVQWKVDNALQSGNS
QESVTEQDSKDSTYSLSSTLTLSKADYEKHKVYACEVTHQGLSSPVTKSFNRG
EC.

Table 30. The tumor growth inhibition rate of each group relative to the PBS group

| Group | Number of animals | Frequency of administration | Route of administration | 2-day tumor growth inhibition rate% | 4-day tumor growth inhibition rate% | 6-day tumor growth inhibition rate% | 9-day tumor growth inhibition rate% | 11-day tumor growth inhibition rate% | 13-day tumor growth inhibition rate% |
|---|---|---|---|---|---|---|---|---|---|
| PBS | 8 | biw*3w | ip | - | - | - | - | - | - |
| MK-30mpk | 8 | biw*3w | ip | 23.5 | 27.4 | 12.8 | -6.8 | -0.1 | 9.2 |
| huP2-G9-30mpk | 8 | biw*3w | ip | 50.3 | 38.8 | 27.7 | 14.0 | 30.2 | 31.3 |
| huP2-C6-30mpk | 8 | biw*3w | ip | 58.0 | 43.7 | 35.6 | 17.4 | 31.2 | 29.6 |
| αPD-1-0.3mpk | 8 | biw*3w | ip | 18.6 | 39.2 | 37.9 | 38.9 | 41.6 | 49.3 |
| huP2-G9-30mpk+αPD-1-0.3mpk | 8 | biw*3w | ip | 32.6 | 29.9 | 34.9 | 25.3 | 48.1 | 56.3 |
| huP2-C6-30mpk+αPD-1-0.3mpk | 8 | biw*3w | ip | 32.4 | 36.2 | 42.7 | 36.7 | 54.1 | *55.1* |
| Note: biw stands for "twice a week", 3w stands for "three weeks", and ip stands for "intraperitoneal injection". | | | | | | | | | |

The experimental results show that compared to the control antibody MK, the huP2-G9 monotherapy and the huP2-C6 monotherapy exhibited relatively good efficacy, and the anti-PD-1 antibody monotherapy exhibited relatively strong efficacy; in addition, huP2-G9 or huP2-C6's respective combination group exhibited slightly stronger efficacy than the corresponding monotherapy group.

Although the foregoing invention has been described in detail using drawings and examples for a clear understanding, the description and examples should not be construed as limiting the scope of the present disclosure. The disclosures of all the patents and scientific literature cited herein are clearly incorporated by reference in their entirety.

**Claims**

1. An anti-ILT4 antibody, comprising a heavy chain variable region and a light chain variable region, wherein the heavy chain variable region comprises a HCDR1, a HCDR2, and a HCDR3, and the light chain variable region comprises a LCDR1, a LCDR2, and a LCDR3, wherein:

    a. the HCDR1, the HCDR2, and the HCDR3 of the heavy chain variable region comprise amino acid sequences of a HCDR1, a HCDR2, and a HCDR3, respectively, in SEQ ID NO: 184 or 185, and the LCDR1, the LCDR2, and the LCDR3 of the light chain variable region comprise amino acid sequences of a LCDR1, a LCDR2, and a LCDR3, respectively, in SEQ ID NO: 187 or 186; or

    the HCDR1, the HCDR2, and the HCDR3 of the heavy chain variable region comprise amino acid sequences of a HCDR1, a HCDR2, and a HCDR3, respectively, in SEQ ID NO: 32, and the LCDR1, the LCDR2, and the LCDR3 of the light chain variable region comprise amino acid sequences of a LCDR1, a LCDR2, and a LCDR3, respectively, in SEQ ID NO: 33; or

    b. the HCDR1, the HCDR2, and the HCDR3 of the heavy chain variable region comprise amino acid sequences of a HCDR1, a HCDR2, and a HCDR3, respectively, in any one of SEQ ID NO: 211, 205, 206, 207, 208, 209, or 210, and the LCDR1, the LCDR2, and the LCDR3 of the light chain variable region comprise amino acid sequences of a LCDR1, a LCDR2, and a LCDR3, respectively, in any one of SEQ ID NO: 212, 213, or 214; or

    the HCDR1, the HCDR2, and the HCDR3 of the heavy chain variable region comprise amino acid sequences of a HCDR1, a HCDR2, and a HCDR3, respectively, in SEQ ID NO: 40, and the LCDR1, the LCDR2, and the LCDR3 of the light chain variable region comprise amino acid sequences of a LCDR1, a LCDR2, and a LCDR3, respectively, in SEQ ID NO: 41; or

    c. the HCDR1, the HCDR2, and the HCDR3 of the heavy chain variable region comprise amino acid sequences of a HCDR1, a HCDR2, and a HCDR3, respectively, in any one of SEQ ID NO: 181, 176, 177, 178, 179, or 180, and the LCDR1, the LCDR2, and the LCDR3 of the light chain variable region comprise amino acid sequences of a LCDR1, a LCDR2, and a LCDR3, respectively, in SEQ ID NO: 183 or 182; or

    the HCDR1, the HCDR2, and the HCDR3 of the heavy chain variable region comprise amino acid sequences of a HCDR1, a HCDR2, and a HCDR3, respectively, in SEQ ID NO: 28, and the LCDR1, the LCDR2, and the LCDR3 of the light chain variable region comprise amino acid sequences of a LCDR1, a LCDR2, and a LCDR3, respectively, in SEQ ID NO: 29; or

    d. the HCDR1, the HCDR2, and the HCDR3 of the heavy chain variable region comprise amino acid sequences of a HCDR1, a HCDR2, and a HCDR3, respectively, in any one of SEQ ID NO: 193, 190, 191, or 192, and the LCDR1, the LCDR2, and the LCDR3 of the light chain variable region comprise amino acid sequences of a LCDR1, a LCDR2, and a LCDR3, respectively, in any one of SEQ ID NO: 194, 195, or 196; or the HCDR1, the HCDR2, and the HCDR3 of the heavy chain variable region comprise amino acid sequences of a HCDR1, a HCDR2, and a HCDR3, respectively, in SEQ ID NO: 10, and the LCDR1, the LCDR2, and the LCDR3 of the light chain variable region comprise amino acid sequences of a LCDR1, a LCDR2, and a LCDR3, respectively, in SEQ ID NO: 11; or

    e. the HCDR1, the HCDR2, and the HCDR3 of the heavy chain variable region comprise amino acid sequences of a HCDR1, a HCDR2, and a HCDR3, respectively, in SEQ ID NO: 163, and the LCDR1, the LCDR2, and the LCDR3 of the light chain variable region comprise amino acid sequences of a LCDR1, a LCDR2, and a LCDR3, respectively, in SEQ ID NO: 225 or 164; or

    f. the HCDR1, the HCDR2, and the HCDR3 of the heavy chain variable region comprise amino acid sequences of a HCDR1, a HCDR2, and a HCDR3, respectively, in SEQ ID NO: 197 or 198, and the LCDR1, the LCDR2, and the LCDR3 of the light chain variable region comprise amino acid sequences of a LCDR1, a LCDR2, and a LCDR3, respectively, in SEQ ID NO: 199 or 200; or

    the HCDR1, the HCDR2, and the HCDR3 of the heavy chain variable region comprise amino acid sequences of a HCDR1, a HCDR2, and a HCDR3, respectively, in SEQ ID NO: 36, and the LCDR1, the LCDR2, and the LCDR3 of the light chain variable region comprise amino acid sequences of a LCDR1, a LCDR2, and a LCDR3, respectively, in SEQ ID NO: 37; or

g. the HCDR1, the HCDR2, and the HCDR3 of the heavy chain variable region comprise amino acid sequences of a HCDR1, a HCDR2, and a HCDR3, respectively, in any one of SEQ ID NO: 217, 218, 219, 220, or 221, and the LCDR1, the LCDR2, and the LCDR3 of the light chain variable region comprise amino acid sequences of a LCDR1, a LCDR2, and a LCDR3, respectively, in SEQ ID NO: 222 or 223; or

the HCDR1, the HCDR2, and the HCDR3 of the heavy chain variable region comprise amino acid sequences of a HCDR1, a HCDR2, and a HCDR3, respectively, in SEQ ID NO: 42, and the LCDR1, the LCDR2, and the LCDR3 of the light chain variable region comprise amino acid sequences of a LCDR1, a LCDR2, and a LCDR3, respectively, in SEQ ID NO: 43; or

h. the HCDR1, the HCDR2, and the HCDR3 of the heavy chain variable region comprise amino acid sequences of a HCDR1, a HCDR2, and a HCDR3, respectively, in SEQ ID NO: 34, and the LCDR1, the LCDR2, and the LCDR3 of the light chain variable region comprise amino acid sequences of a LCDR1, a LCDR2, and a LCDR3, respectively, in SEQ ID NO: 35; or

i. the HCDR1, the HCDR2, and the HCDR3 of the heavy chain variable region comprise amino acid sequences of a HCDR1, a HCDR2, and a HCDR3, respectively, in SEQ ID NO: 18, and the LCDR1, the LCDR2, and the LCDR3 of the light chain variable region comprise amino acid sequences of a LCDR1, a LCDR2, and a LCDR3, respectively, in SEQ ID NO: 19; or

j. the HCDR1, the HCDR2, and the HCDR3 of the heavy chain variable region comprise amino acid sequences of a HCDR1, a HCDR2, and a HCDR3, respectively, in SEQ ID NO: 20, and the LCDR1, the LCDR2, and the LCDR3 of the light chain variable region comprise amino acid sequences of a LCDR1, a LCDR2, and a LCDR3, respectively, in SEQ ID NO: 21; or

k. the HCDR1, the HCDR2, and the HCDR3 of the heavy chain variable region comprise amino acid sequences of a HCDR1, a HCDR2, and a HCDR3, respectively, in SEQ ID NO: 24, and the LCDR1, the LCDR2, and the LCDR3 of the light chain variable region comprise amino acid sequences of a LCDR1, a LCDR2, and a LCDR3, respectively, in SEQ ID NO: 25; or

l. the HCDR1, the HCDR2, and the HCDR3 of the heavy chain variable region comprise amino acid sequences of a HCDR1, a HCDR2, and a HCDR3, respectively, in SEQ ID NO: 44, and the LCDR1, the LCDR2, and the LCDR3 of the light chain variable region comprise amino acid sequences of a LCDR1, a LCDR2, and a LCDR3, respectively, in SEQ ID NO: 45; or

m. the HCDR1, the HCDR2, and the HCDR3 of the heavy chain variable region comprise amino acid sequences of a HCDR1, a HCDR2, and a HCDR3, respectively, in SEQ ID NO: 46, and the LCDR1, the LCDR2, and the LCDR3 of the light chain variable region comprise amino acid sequences of a LCDR1, a LCDR2, and a LCDR3, respectively, in SEQ ID NO: 47; or

n. the HCDR1, the HCDR2, and the HCDR3 of the heavy chain variable region comprise amino acid sequences of a HCDR1, a HCDR2, and a HCDR3, respectively, in SEQ ID NO: 48, and the LCDR1, the LCDR2, and the LCDR3 of the light chain variable region comprise amino acid sequences of a LCDR1, a LCDR2, and a LCDR3, respectively, in SEQ ID NO: 49; or

o. the HCDR1, the HCDR2, and the HCDR3 of the heavy chain variable region comprise amino acid sequences of a HCDR1, a HCDR2, and a HCDR3, respectively, in SEQ ID NO: 50, and the LCDR1, the LCDR2, and the LCDR3 of the light chain variable region comprise amino acid sequences of a LCDR1, a LCDR2, and a LCDR3, respectively, in SEQ ID NO: 51;

preferably,

a. the HCDR1, the HCDR2, and the HCDR3 of the heavy chain variable region comprise amino acid sequences of a HCDR1, a HCDR2, and a HCDR3, respectively, in SEQ ID NO: 184, and the LCDR1, the LCDR2, and the LCDR3 of the light chain variable region comprise amino acid sequences of a LCDR1, a LCDR2, and a LCDR3, respectively, in SEQ ID NO: 187; or

b. the HCDR1, the HCDR2, and the HCDR3 of the heavy chain variable region comprise amino acid sequences of a HCDR1, a HCDR2, and a HCDR3, respectively, in SEQ ID NO: 211, and the LCDR1, the LCDR2, and the LCDR3 of the light chain variable region comprise amino acid sequences of a LCDR1, a LCDR2, and a LCDR3, respectively, in SEQ ID NO: 212; or

c. the HCDR1, the HCDR2, and the HCDR3 of the heavy chain variable region comprise amino acid sequences of a HCDR1, a HCDR2, and a HCDR3, respectively, in SEQ ID NO: 181, and the LCDR1, the LCDR2, and the LCDR3 of the light chain variable region comprise amino acid sequences of a LCDR1, a LCDR2, and a LCDR3, respectively, in SEQ ID NO: 183; or

d. the HCDR1, the HCDR2, and the HCDR3 of the heavy chain variable region comprise amino acid sequences of a HCDR1, a HCDR2, and a HCDR3, respectively, in SEQ ID NO: 193, and the LCDR1, the LCDR2, and the LCDR3 of the light chain variable region comprise amino acid sequences of a LCDR1, a LCDR2, and a LCDR3, respectively, in SEQ ID NO: 194; or

e. the HCDR1, the HCDR2, and the HCDR3 of the heavy chain variable region comprise amino acid sequences of a HCDR1, a HCDR2, and a HCDR3, respectively, in SEQ ID NO: 163, and the LCDR1, the LCDR2, and the LCDR3 of the light chain variable region comprise amino acid sequences of a LCDR1, a LCDR2, and a LCDR3, respectively, in SEQ ID NO: 225; or

f. the HCDR1, the HCDR2, and the HCDR3 of the heavy chain variable region comprise amino acid sequences of a HCDR1, a HCDR2, and a HCDR3, respectively, in SEQ ID NO: 197, and the LCDR1, the LCDR2, and the LCDR3 of the light chain variable region comprise amino acid sequences of a LCDR1, a LCDR2, and a LCDR3, respectively, in SEQ ID NO: 199; or

g. the HCDR1, the HCDR2, and the HCDR3 of the heavy chain variable region comprise amino acid sequences of a HCDR1, a HCDR2, and a HCDR3, respectively, in SEQ ID NO: 217, and the LCDR1, the LCDR2, and the LCDR3 of the light chain variable region comprise amino acid sequences of a LCDR1, a LCDR2, and a LCDR3, respectively, in SEQ ID NO: 222;

more preferably,

a. the HCDR1, the HCDR2, and the HCDR3 of the heavy chain variable region comprise amino acid sequences of a HCDR1, a HCDR2, and a HCDR3, respectively, in SEQ ID NO: 184, and the LCDR1, the LCDR2, and the LCDR3 of the light chain variable region comprise amino acid sequences of a LCDR1, a LCDR2, and a LCDR3, respectively, in SEQ ID NO: 187; or

b. the HCDR1, the HCDR2, and the HCDR3 of the heavy chain variable region comprise amino acid sequences of a HCDR1, a HCDR2, and a HCDR3, respectively, in SEQ ID NO: 211, and the LCDR1, the LCDR2, and the LCDR3 of the light chain variable region comprise amino acid sequences of a LCDR1, a LCDR2, and a LCDR3, respectively, in SEQ ID NO: 212.

2. The anti-ILT4 antibody according to claim 1, wherein:

a. in the heavy chain variable region, the HCDR1 comprises an amino acid sequence of SEQ ID NO: 105, the HCDR2 comprises an amino acid sequence of SEQ ID NO: 120, and the HCDR3 comprises an amino acid sequence of SEQ ID NO: 121; and in the light chain variable region, the LCDR1 comprises an amino acid sequence of SEQ ID NO: 114, the LCDR2 comprises an amino acid sequence of SEQ ID NO: 109, and the LCDR3 comprises an amino acid sequence of SEQ ID NO: 119; or

b. in the heavy chain variable region, the HCDR1 comprises an amino acid sequence of SEQ ID NO: 135, the HCDR2 comprises an amino acid sequence of any one of SEQ ID NO: 203, 136, 201, 202, or 204, and the HCDR3 comprises an amino acid sequence of SEQ ID NO: 137; and in the light chain variable region, the LCDR1 comprises an amino acid sequence of SEQ ID NO: 138, the LCDR2 comprises an amino acid sequence of SEQ ID NO: 139, and the LCDR3 comprises an amino acid sequence of SEQ ID NO: 140; or

c. in the heavy chain variable region, the HCDR1 comprises an amino acid sequence of SEQ ID NO: 111, the HCDR2 comprises an amino acid sequence of any one of SEQ ID NO: 173, 112, 174, or 175, and the HCDR3 comprises an amino acid sequence of SEQ ID NO: 113; and in the light chain variable region, the LCDR1 comprises an amino acid sequence of SEQ ID NO: 114, the LCDR2 comprises an amino acid sequence of SEQ ID NO: 115, and the LCDR3 comprises an amino acid sequence of SEQ ID NO: 116; or

d. in the heavy chain variable region, the HCDR1 comprises an amino acid sequence of SEQ ID NO: 69, the HCDR2 comprises an amino acid sequence of any one of SEQ ID NO: 189, 70, or 188, and the HCDR3 comprises an amino acid sequence of SEQ ID NO: 71; and in the light chain variable region, the LCDR1 comprises an amino acid sequence of SEQ ID NO: 61, the LCDR2 comprises an amino acid sequence of SEQ ID NO: 62, and the LCDR3 comprises an amino acid sequence of SEQ ID NO: 72; or

e. in the heavy chain variable region, the HCDR1 comprises an amino acid sequence of SEQ ID NO: 165, the HCDR2 comprises an amino acid sequence of SEQ ID NO: 166, and the HCDR3 comprises an amino acid sequence of SEQ ID NO: 167; and in the light chain variable region, the LCDR1 comprises an amino acid sequence of SEQ ID NO: 224 or 168, the LCDR2 comprises an amino acid sequence of SEQ ID NO: 169, and the LCDR3 comprises an amino acid sequence of SEQ ID NO: 170; or

f. in the heavy chain variable region, the HCDR1 comprises an amino acid sequence of SEQ ID NO: 105, the HCDR2 comprises an amino acid sequence of SEQ ID NO: 125, and the HCDR3 comprises an amino acid sequence of SEQ ID NO: 126; and in the light chain variable region, the LCDR1 comprises an amino acid sequence of SEQ ID NO: 127, the LCDR2 comprises an amino acid sequence of SEQ ID NO: 109, and the LCDR3 comprises an amino acid sequence of SEQ ID NO: 128; or

g. in the heavy chain variable region, the HCDR1 comprises an amino acid sequence of SEQ ID NO: 141, the HCDR2 comprises an amino acid sequence of SEQ ID NO: 215 or 142, and the HCDR3 comprises an amino acid sequence of SEQ ID NO: 216 or 143; and in the light chain variable region, the LCDR1 comprises an amino acid sequence of SEQ ID NO: 144, the LCDR2 comprises an amino acid sequence of SEQ ID NO: 145, and the LCDR3 comprises an amino acid sequence of SEQ ID NO: 146; or

h. in the heavy chain variable region, the HCDR1 comprises an amino acid sequence of SEQ ID NO: 99, the HCDR2 comprises an amino acid sequence of SEQ ID NO: 122, and the HCDR3 comprises an amino acid sequence of SEQ ID NO: 123; and in the light chain variable region, the LCDR1 comprises an amino acid sequence of SEQ ID NO: 114, the LCDR2 comprises an amino acid sequence of SEQ ID NO: 109, and the LCDR3 comprises an amino acid sequence of SEQ ID NO: 124; or

i. in the heavy chain variable region, the HCDR1 comprises an amino acid sequence of SEQ ID NO: 73, the HCDR2 comprises an amino acid sequence of SEQ ID NO: 87, and the HCDR3 comprises an amino acid sequence of SEQ ID NO: 88; and in the light chain variable region, the LCDR1 comprises an amino acid sequence of SEQ ID NO: 61, the LCDR2 comprises an amino acid sequence of SEQ ID NO: 89, and the LCDR3 comprises an amino acid sequence of SEQ ID NO: 90; or

j. in the heavy chain variable region, the HCDR1 comprises an amino acid sequence of SEQ ID NO: 91, the HCDR2 comprises an amino acid sequence of SEQ ID NO: 92, and the HCDR3 comprises an amino acid sequence of SEQ ID NO: 93; and in the light chain variable region, the LCDR1 comprises an amino acid sequence of SEQ ID NO: 85, the LCDR2 comprises an amino acid sequence of SEQ ID NO: 62, and the LCDR3 comprises an amino acid sequence of SEQ ID NO: 94; or

k. in the heavy chain variable region, the HCDR1 comprises an amino acid sequence of SEQ ID NO: 99, the HCDR2 comprises an amino acid sequence of SEQ ID NO: 100, and the HCDR3 comprises an amino acid sequence of SEQ ID NO: 101; and in the light chain variable region, the LCDR1 comprises an amino acid sequence of SEQ ID NO: 102, the LCDR2 comprises an amino acid sequence of SEQ ID NO: 103, and the LCDR3 comprises an amino acid sequence of SEQ ID NO: 104; or

l. in the heavy chain variable region, the HCDR1 comprises an amino acid sequence of SEQ ID NO: 147, the HCDR2 comprises an amino acid sequence of SEQ ID NO: 148, and the HCDR3 comprises an amino acid sequence of SEQ ID NO: 149; and in the light chain variable region, the LCDR1 comprises an amino acid sequence of SEQ ID NO: 150, the LCDR2 comprises an amino acid sequence of SEQ ID NO: 151, and the LCDR3 comprises an amino acid sequence of SEQ ID NO: 152; or

m. in the heavy chain variable region, the HCDR1 comprises an amino acid sequence of SEQ ID NO: 153, the HCDR2 comprises an amino acid sequence of SEQ ID NO: 154, and the HCDR3 comprises an amino acid sequence of SEQ ID NO: 155; and in the light chain variable region, the LCDR1 comprises an amino acid sequence of SEQ ID NO: 114, the LCDR2 comprises an amino acid sequence of SEQ ID NO: 133, and the LCDR3 comprises an amino acid sequence of SEQ ID NO: 119; or

n. in the heavy chain variable region, the HCDR1 comprises an amino acid sequence of SEQ ID NO: 58, the HCDR2 comprises an amino acid sequence of SEQ ID NO: 156, and the HCDR3 comprises an amino acid sequence of SEQ ID NO: 157; and in the light chain variable region, the LCDR1 comprises an amino acid sequence of SEQ ID NO: 158, the LCDR2 comprises an amino acid sequence of SEQ ID NO: 62, and the LCDR3 comprises an amino acid sequence of SEQ ID NO: 159; or

o. in the heavy chain variable region, the HCDR1 comprises an amino acid sequence of SEQ ID NO: 105, the HCDR2 comprises an amino acid sequence of SEQ ID NO: 160, and the HCDR3 comprises an amino acid sequence of SEQ ID NO: 161; and in the light chain variable region, the LCDR1 comprises an amino acid sequence of SEQ ID NO: 114, the LCDR2 comprises an amino acid sequence of SEQ ID NO: 109, and the LCDR3 comprises an amino acid sequence of SEQ ID NO: 162;

preferably,

a. in the heavy chain variable region, the HCDR1 comprises an amino acid sequence of SEQ ID NO: 105, the HCDR2 comprises an amino acid sequence of SEQ ID NO: 120, and the HCDR3 comprises an amino acid sequence of SEQ ID NO: 121; and in the light chain variable region, the LCDR1 comprises an amino acid sequence of SEQ ID NO: 114, the LCDR2 comprises an amino acid sequence of SEQ ID NO: 109, and the LCDR3 comprises an amino acid sequence of SEQ ID NO: 119;

b. in the heavy chain variable region, the HCDR1 comprises an amino acid sequence of SEQ ID NO: 135, the HCDR2 comprises an amino acid sequence of SEQ ID NO: 203, and the HCDR3 comprises an amino acid sequence of SEQ ID NO: 137; and in the light chain variable region, the LCDR1 comprises an amino acid sequence of SEQ ID NO: 138, the LCDR2 comprises an amino acid sequence of SEQ ID NO: 139, and the LCDR3 comprises an amino acid sequence of SEQ ID NO: 140; or

c. in the heavy chain variable region, the HCDR1 comprises an amino acid sequence of SEQ ID NO: 111, the HCDR2 comprises an amino acid sequence of SEQ ID NO: 173, and the HCDR3 comprises an amino acid sequence of SEQ ID NO: 113; and in the light chain variable region, the LCDR1 comprises an amino acid sequence of SEQ ID NO: 114, the LCDR2 comprises an amino acid sequence of SEQ ID NO: 115, and the LCDR3 comprises an amino acid sequence of SEQ ID NO: 116; or

d. in the heavy chain variable region, the HCDR1 comprises an amino acid sequence of SEQ ID NO: 69, the HCDR2 comprises an amino acid sequence of SEQ ID NO: 189, and the HCDR3 comprises an amino acid sequence of SEQ ID NO: 71; and in the light chain variable region, the LCDR1 comprises an amino acid sequence of SEQ ID NO: 61, the LCDR2 comprises an amino acid sequence of SEQ ID NO: 62, and the LCDR3 comprises an amino acid sequence of SEQ ID NO: 72; or

e. in the heavy chain variable region, the HCDR1 comprises an amino acid sequence of SEQ ID NO: 165, the HCDR2 comprises an amino acid sequence of SEQ ID NO: 166, and the HCDR3 comprises an amino acid sequence of SEQ ID NO: 167; and in the light chain variable region, the LCDR1 comprises an amino acid sequence of SEQ ID NO: 224, the LCDR2 comprises an amino acid sequence of SEQ ID NO: 169, and the LCDR3 comprises an amino acid sequence of SEQ ID NO: 170; or

f. in the heavy chain variable region, the HCDR1 comprises an amino acid sequence of SEQ ID NO: 105, the HCDR2 comprises an amino acid sequence of SEQ ID NO: 125, and the HCDR3 comprises an amino acid sequence of SEQ ID NO: 126; and in the light chain variable region, the LCDR1 comprises an amino acid sequence of SEQ ID NO: 127, the LCDR2 comprises an amino acid sequence of SEQ ID NO: 109, and the LCDR3 comprises an amino acid sequence of SEQ ID NO: 128; or

g. in the heavy chain variable region, the HCDR1 comprises an amino acid sequence of SEQ ID NO: 141, the HCDR2 comprises an amino acid sequence of SEQ ID NO: 215, and the HCDR3 comprises an amino acid sequence of SEQ ID NO: 216; and in the light chain variable region, the LCDR1 comprises an amino acid sequence of SEQ ID NO: 144, the LCDR2 comprises an amino acid sequence of SEQ ID NO: 145, and the LCDR3 comprises an amino acid sequence of SEQ ID NO: 146;

more preferably,

a. in the heavy chain variable region, the HCDR1 comprises an amino acid sequence of SEQ ID NO: 105, the HCDR2 comprises an amino acid sequence of SEQ ID NO: 120, and the HCDR3 comprises an amino acid sequence of SEQ ID NO: 121; and in the light chain variable region, the LCDR1 comprises an amino acid sequence of SEQ ID NO: 114, the LCDR2 comprises an amino acid sequence of SEQ ID NO: 109, and the LCDR3 comprises an amino acid sequence of SEQ ID NO: 119;

b. in the heavy chain variable region, the HCDR1 comprises an amino acid sequence of SEQ ID NO: 135, the HCDR2 comprises an amino acid sequence of SEQ ID NO: 203, and the HCDR3 comprises an amino acid sequence of SEQ ID NO: 137; and in the light chain variable region, the LCDR1 comprises an amino acid sequence of SEQ ID NO: 138, the LCDR2 comprises an amino acid sequence of SEQ ID NO: 139, and the LCDR3 comprises an amino acid sequence of SEQ ID NO: 140.

**3.** The anti-ILT4 antibody according to claim 1 or 2, wherein the anti-ILT4 antibody is a murine antibody, a chimeric antibody, a humanized antibody, or a fully human-derived antibody, preferably a humanized antibody.

**4.** The anti-ILT4 antibody according to any one of claims 1 to 3, wherein:

a. the heavy chain variable region comprises an amino acid sequence having at least 70% sequence identity to SEQ ID NO: 184 or 185, and the light chain variable region comprises an amino acid sequence having at least 70% sequence identity to SEQ ID NO: 187 or 186; or
the heavy chain variable region comprises an amino acid sequence having at least 70% sequence identity to SEQ ID NO: 32, and the light chain variable region comprises an amino acid sequence having at least 70% sequence identity to SEQ ID NO: 33; or

b. the heavy chain variable region comprises an amino acid sequence having at least 70% sequence identity to SEQ ID NO: 211, 205, 206, 207, 208, 209, or 210, and the light chain variable region comprises an amino acid sequence having at least 70% sequence identity to SEQ ID NO: 212, 213, or 214; or
the heavy chain variable region comprises an amino acid sequence having at least 70% sequence identity to SEQ ID NO: 40, and the light chain variable region comprises an amino acid sequence having at least 70% sequence identity to SEQ ID NO: 41; or

c. the heavy chain variable region comprises an amino acid sequence having at least 70% sequence identity to SEQ ID NO: 181, 176, 177, 178, 179, or 180, and the light chain variable region comprises an amino acid sequence having at least 70% sequence identity to SEQ ID NO: 183 or 182; or

the heavy chain variable region comprises an amino acid sequence having at least 70% sequence identity to SEQ ID NO: 28, and the light chain variable region comprises an amino acid sequence having at least 70% sequence identity to SEQ ID NO: 29; or

d. the heavy chain variable region comprises an amino acid sequence having at least 70% sequence identity to SEQ ID NO: 193, 190, 191, or 192, and the light chain variable region comprises an amino acid sequence having at least 70% sequence identity to SEQ ID NO: 194, 195, or 196; or

the heavy chain variable region comprises an amino acid sequence having at least 70% sequence identity to SEQ ID NO: 10, and the light chain variable region comprises an amino acid sequence having at least 70% sequence identity to SEQ ID NO: 11; or

e. the heavy chain variable region comprises an amino acid sequence having at least 70% sequence identity to SEQ ID NO: 163, and the light chain variable region comprises an amino acid sequence having at least 70% sequence identity to SEQ ID NO: 225 or 164;

f. the heavy chain variable region comprises an amino acid sequence having at least 70% sequence identity to SEQ ID NO: 197 or 198, and the light chain variable region comprises an amino acid sequence having at least 70% sequence identity to SEQ ID NO: 199 or 200; or

the heavy chain variable region comprises an amino acid sequence having at least 70% sequence identity to SEQ ID NO: 36, and the light chain variable region comprises an amino acid sequence having at least 70% sequence identity to SEQ ID NO: 37; or

g. the heavy chain variable region comprises an amino acid sequence having at least 70% sequence identity to SEQ ID NO: 217, 218, 219, 220, or 221, and the light chain variable region comprises an amino acid sequence having at least 70% sequence identity to SEQ ID NO: 222 or 223; or

the heavy chain variable region comprises an amino acid sequence having at least 70% sequence identity to SEQ ID NO: 42, and the light chain variable region comprises an amino acid sequence having at least 70% sequence identity to SEQ ID NO: 43;

preferably,

a. the heavy chain variable region comprises an amino acid sequence of SEQ ID NO: 184 or 185, and the light chain variable region comprises an amino acid sequence of SEQ ID NO: 187 or 186; or

the heavy chain variable region comprises an amino acid sequence of SEQ ID NO: 32, and the light chain variable region comprises an amino acid sequence of SEQ ID NO: 33; or

b. the heavy chain variable region comprises an amino acid sequence of SEQ ID NO: 211, 205, 206, 207, 208, 209, or 210, and the light chain variable region comprises an amino acid sequence of SEQ ID NO: 212, 213, or 214; or

the heavy chain variable region comprises an amino acid sequence of SEQ ID NO: 40, and the light chain variable region comprises an amino acid sequence of SEQ ID NO: 41; or

c. the heavy chain variable region comprises an amino acid sequence of SEQ ID NO: 181, 176, 177, 178, 179, or 180, and the light chain variable region comprises an amino acid sequence of SEQ ID NO: 183 or 182; or

the heavy chain variable region comprises an amino acid sequence of SEQ ID NO: 28, and the light chain variable region comprises an amino acid sequence of SEQ ID NO: 29; or

d. the heavy chain variable region comprises an amino acid sequence of SEQ ID NO: 193, 190, 191, or 192, and the light chain variable region comprises an amino acid sequence of SEQ ID NO: 194, 195, or 196; or

the heavy chain variable region comprises an amino acid sequence of SEQ ID NO: 10, and the light chain variable region comprises an amino acid sequence of SEQ ID NO: 11; or

e. the heavy chain variable region comprises an amino acid sequence of SEQ ID NO: 163, and the light chain variable region comprises an amino acid sequence of SEQ ID NO: 225 or 164;

f. the heavy chain variable region comprises an amino acid sequence of SEQ ID NO: 197 or 198, and the light chain variable region comprises an amino acid sequence of SEQ ID NO: 199 or 200; or

the heavy chain variable region comprises an amino acid sequence of SEQ ID NO: 36, and the light chain variable region comprises an amino acid sequence of SEQ ID NO: 37; or

g. the heavy chain variable region comprises an amino acid sequence of SEQ ID NO: 217, 218, 219, 220, or 221, and the light chain variable region comprises an amino acid sequence of SEQ ID NO: 222 or 223; or

the heavy chain variable region comprises an amino acid sequence of SEQ ID NO: 42, and the light chain variable region comprises an amino acid sequence of SEQ ID NO: 43.

5. The anti-ILT4 antibody according to any one of claims 1 to 4, wherein the anti-ILT4 antibody is an antibody fragment; preferably, the antibody fragment is a Fab, Fab', F(ab')2, Fd, Fv, scFv, dsFv, or dAb.

6. The anti-ILT4 antibody according to any one of claims 1 to 4, wherein the anti-ILT4 antibody comprises a heavy chain constant region and a light chain constant region; preferably, the heavy chain constant region is a human IgG1 or IgG4 heavy chain constant region, and the light chain constant region is a human κ light chain constant region; more preferably, the heavy chain constant region comprises an amino acid sequence of SEQ ID NO: 240 or 171, and the light chain constant region comprises an amino acid sequence of SEQ ID NO: 172.

7. The anti-ILT4 antibody according to claim 6, wherein the anti-ILT4 antibody comprises a heavy chain and a light chain, wherein:

   a. the heavy chain comprises an amino acid sequence having at least 70% sequence identity to SEQ ID NO: 228, and the light chain comprises an amino acid sequence having at least 70% sequence identity to SEQ ID NO: 229; or
   b. the heavy chain comprises an amino acid sequence having at least 70% sequence identity to SEQ ID NO: 234, and the light chain comprises an amino acid sequence having at least 70% sequence identity to SEQ ID NO: 235; or
   c. the heavy chain comprises an amino acid sequence having at least 70% sequence identity to SEQ ID NO: 226, and the light chain comprises an amino acid sequence having at least 70% sequence identity to SEQ ID NO: 227; or
   d. the heavy chain comprises an amino acid sequence having at least 70% sequence identity to SEQ ID NO: 230, and the light chain comprises an amino acid sequence having at least 70% sequence identity to SEQ ID NO: 231; or
   e. the heavy chain comprises an amino acid sequence having at least 70% sequence identity to SEQ ID NO: 238, and the light chain comprises an amino acid sequence having at least 70% sequence identity to SEQ ID NO: 239; or
   f. the heavy chain comprises an amino acid sequence having at least 70% sequence identity to SEQ ID NO: 232, and the light chain comprises an amino acid sequence having at least 70% sequence identity to SEQ ID NO: 233; or
   g. the heavy chain comprises an amino acid sequence having at least 70% sequence identity to SEQ ID NO: 236, and the light chain comprises an amino acid sequence having at least 70% sequence identity to SEQ ID NO: 237;

   preferably,

   a. an amino acid sequence of the heavy chain is set forth in SEQ ID NO: 228, and an amino acid sequence of the light chain is set forth in SEQ ID NO: 229; or
   b. an amino acid sequence of the heavy chain is set forth in SEQ ID NO: 234, and an amino acid sequence of the light chain is set forth in SEQ ID NO: 235; or
   c. an amino acid sequence of the heavy chain is set forth in SEQ ID NO: 226, and an amino acid sequence of the light chain is set forth in SEQ ID NO: 227; or
   d. an amino acid sequence of the heavy chain is set forth in SEQ ID NO: 230, and an amino acid sequence of the light chain is set forth in SEQ ID NO: 231; or
   e. an amino acid sequence of the heavy chain is set forth in SEQ ID NO: 238, and an amino acid sequence of the light chain is set forth in SEQ ID NO: 239; or
   f. an amino acid sequence of the heavy chain is set forth in SEQ ID NO: 232, and an amino acid sequence of the light chain is set forth in SEQ ID NO: 233; or
   g. an amino acid sequence of the heavy chain is set forth in SEQ ID NO: 236, and an amino acid sequence of the light chain is set forth in SEQ ID NO: 237.

8. A pharmaceutical composition, comprising the anti-ILT4 antibody according to any one of claims 1 to 7 and one or more pharmaceutically acceptable carriers, diluents, or excipients, wherein preferably, the pharmaceutical composition further comprises at least one second therapeutic agent; more preferably, the second therapeutic agent is an anti-PD-1 antibody.

9. An isolated nucleic acid, wherein the isolated nucleic acid encodes the anti-ILT4 antibody according to any one of claims 1 to 7.

10. A host cell, comprising the isolated nucleic acid according to claim 9.

11. A method for blocking binding of ILT4 to HLA-G in a human subject in need thereof, comprising administering to the human subject an effective amount of the anti-ILT4 antibody according to any one of claims 1 to 7.

12. A method for detecting an ILT4 peptide or a fragment thereof in a sample, comprising contacting the sample with the anti-ILT4 antibody according to any one of claims 1 to 7 and detecting the presence of a complex between the anti-ILT4 antibody and the ILT4 peptide or the fragment thereof, wherein that the complex is detected indicates the presence of the ILT4 peptide or the fragment thereof.

13. A method for treating a disease, comprising administering to a subject a therapeutically effective amount of the anti-ILT4 antibody according to any one of claims 1 to 7 or the pharmaceutical composition according to claim 8, wherein:

preferably, the disease is a tumor; more preferably, the disease is selected from the group consisting of astrocytoma, glioblastoma, bladder cancer, bone cancer, brain cancer, breast cancer, carcinoid, cervical cancer, choroid plexus papilloma, colorectal cancer, fallopian tube cancer, ependymoma, gallbladder cancer, gastric cancer, head and neck cancer, idiopathic myelofibrosis, renal cancer, renal pelvis tumor, leukemia, liver cancer, esophageal cancer, lung cancer, lymphoma, medulloblastoma, melanoma, meningioma, Merkel cell carcinoma, mesothelioma, multiple myeloma, neuroblastoma, oligodendroglioma, ovarian cancer, peritoneal tumor, pancreatic cancer, polycythemia vera, primary neuroectodermal tumor, prostate cancer, retinoblastoma, salivary gland cancer, sarcoma, small intestine cancer, colorectal cancer, squamous cell carcinoma, thyroid cancer, endometrial cancer, vestibular schwannoma, blastoma, teratoma, pituitary cancer, vulva cancer, thymoma, testicular cancer, bile duct cancer, pheochromocytoma, paraganglioma, and adenoid cystic carcinoma; most preferably, the disease is selected from the group consisting of ovarian cancer, lung cancer, esophageal cancer, colorectal cancer, renal cell carcinoma, and melanoma.

14. The method for treating a disease according to claim 13, further comprising using a second therapeutic agent, wherein preferably, the second therapeutic agent comprises an immune checkpoint inhibitor; more preferably, the second therapeutic agent is an anti-PD-1 antibody.

15. The method for treating a disease according to claim 14, wherein the anti-PD-1 antibody comprises a heavy chain variable region PD-1-VH and a light chain variable region PD-1-VL, wherein:

the PD-1-VH comprises: a PD-1-HCDR1 comprising an amino acid sequence of SEQ ID NO: 250, a PD-1-HCDR2 comprising an amino acid sequence of SEQ ID NO: 251, and a PD-1-HCDR3 comprising an amino acid sequence of SEQ ID NO: 252, and the PD-1-VL comprises: a PD-1-LCDR1 comprising an amino acid sequence of SEQ ID NO: 253, a PD-1-LCDR2 comprising an amino acid sequence of SEQ ID NO: 254, and a PD-1-LCDR3 comprising an amino acid sequence of SEQ ID NO: 255; preferably, the PD-1-VH comprises an amino acid sequence of SEQ ID NO: 256, and the PD-1-VL comprises an amino acid sequence of SEQ ID NO: 257.

**FIG. 1A**

**FIG. 1B**

**FIG. 1C**

**FIG. 1D**

**FIG. 2A**

**FIG. 2B**

**FIG. 2C**

**FIG. 2D**

**FIG. 3A**

**FIG. 3B**

**FIG. 3C**

**FIG. 3D**

**FIG. 3E**

## INTERNATIONAL SEARCH REPORT

| International application No. |
|---|
| **PCT/CN2023/117653** |

### A. CLASSIFICATION OF SUBJECT MATTER

C07K 16/28(2006.01)i; A61K 39/395(2006.01)i; A61P 35/00(2006.01)i

According to International Patent Classification (IPC) or to both national classification and IPC

### B. FIELDS SEARCHED

Minimum documentation searched (classification system followed by classification symbols)

IPC:C07K,A61K,A61P

Documentation searched other than minimum documentation to the extent that such documents are included in the fields searched

Electronic data base consulted during the international search (name of data base and, where practicable, search terms used)

CNABS, WPABSC, CNTXT, ENTXTC, DWPI, WPABS, VEN, ENTXT, CNKI, 万方, WANFANG, PubMed, Genbank, 中国专利生物序列检索系统, China Patent Biological Sequence Search System, REGISTRY: 免疫球蛋白样转录子4, 免疫球蛋白样转录物4, 白细胞免疫球蛋白样受体B2, 抗体, 肿瘤, 癌, ILT4, hILT4, LILRB2, LIR2, MIR10, CD85d, antibody, antibodies, cancer, tumor, tumour, neoplasm, neoplasma, carcinoma, SEQ ID NOs: 32, 33, 105, 109, 114, 119-121, 184-187, 228, 229的序列检索, search for SEQ ID NOs: 32, 33, 105, 109, 114, 119-121, 184-187, 228, 229

### C. DOCUMENTS CONSIDERED TO BE RELEVANT

| Category* | Citation of document, with indication, where appropriate, of the relevant passages | Relevant to claim No. |
|---|---|---|
| A | WO 2022060767 A1 (MERCK SHARP & DOHME et al.) 24 March 2022 (2022-03-24)<br>see claims 1-55 | 1-15 (in part) |
| A | CN 113056483 A (FIVE PRIME THERAPEUTICS, INC.; BRISTOL-MYERS SQUIBB COMPANY) 29 June 2021 (2021-06-29)<br>see claims 1-63 | 1-15 (in part) |
| A | CN 110719917 A (MERCK SHARP & DOHME CORP.; AGENUS INC.) 21 January 2020 (2020-01-21)<br>see abstract, claims 1-42 | 1-15 (in part) |
| A | US 2022056128 A1 (ICAHN SHOOL OF MEDICINE AT MOUNT SINAI et al.) 24 February 2022 (2022-02-24)<br>see abstract | 1-15 (in part) |

☑ Further documents are listed in the continuation of Box C. ☑ See patent family annex.

| | |
|---|---|
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **08 December 2023** | **18 December 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
| --- |
| **PCT/CN2023/117653** |

**C.    DOCUMENTS CONSIDERED TO BE RELEVANT**

| Category* | Citation of document, with indication, where appropriate, of the relevant passages | Relevant to claim No. |
| --- | --- | --- |
| A | CHEN, Xiaozheng et al. "ILT4 Inhibition Prevents TAM- and Dysfunctional T Cell-Mediated Immunosuppression and Enhances the Efficacy of Anti-PD-L1 Therapy in NSCLC with EGFR Activation" *Theranostics*, Vol. 11, No. (7), 19 January 2021 (2021-01-19), pp. 3392-3416 see abstract | 1-15 (in part) |

Form PCT/ISA/210 (second sheet) (July 2022)

**INTERNATIONAL SEARCH REPORT**

International application No.

**PCT/CN2023/117653**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

International application No.

**PCT/CN2023/117653**

**Box No. II    Observations where certain claims were found unsearchable (Continuation of item 2 of first sheet)**

This international search report has not been established in respect of certain claims under Article 17(2)(a) for the following reasons:

1. ☑ Claims Nos.: **11, 13-15**
   because they relate to subject matter not required to be searched by this Authority, namely:

   Claims 11 and 13-15 relate to a method for treatment of the human or animal body by therapy, which falls within subject matter for which no international search is required (PCT Rule 39.1(iv)). However, the search for claims 11 and 13-15 is made and based on the subject matter that is reasonably expected to be amemded, i.e., the use of an effective amount of the anti-ILT4 antibody of any one of claims 1-7 in the preparation of a drug for blocking the combination of ILT4 with HLA-G in a human subject in need thereof; and the use of a therapeutically effective amount of the anti-ILT4 antibody of any one of claims 1-7 or the pharmaceutical composition of claim 8 in the preparation of a drug for treating diseases.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:

The present application sets forth the following 15 inventions:

1. Claims 1-15 (in part) relate to anti-ILT4 antibody huP2-G9 or P2-G9, a heavy chain variable region sequence thereof comprising SEQ ID NO: 184, 185 or 32, a light chain variable region sequence comprising SEQ ID NO: 187, 186 or 33, sequences of HCDR1, HCDR2, HCDR3, LCDR1, LCDR2 and LCDR3 respectively comprising SEQ ID NOs: 105, 120, 121, 114, 109 and 119, and sequences of a heavy chain and a light chain respectively comprising SEQ ID NOs: 228 and 229; and a corresponding pharmaceutical composition, coding nucleic acid, host cell, detection method and treatment method.

2. Claims 1-15 (in part) relate to anti-ILT4 antibody huP2-C6 or P2-C6, a heavy chain variable region sequence thereof comprising SEQ ID NO: 211, 205, 206, 207, 208, 209, 210 or 40, a light chain variable region sequence comprising SEQ ID NO: 212, 213, 214 or 41, sequences of HCDR1, HCDR2, HCDR3, LCDR1, LCDR2 and LCDR3 respectively comprising SEQ ID NOs: 135, 203, 136, 201, 202 or 204, 137, 138, 139 and 140, and sequences of a heavy chain and a light chain respectively comprising SEQ ID NOs: 234 and 235; and a corresponding pharmaceutical composition, coding nucleic acid, host cell, detection method and treatment method.

3. Claims 1-15 (in part) relate to anti-ILT4 antibody huP2-E3 or P2-E3, a heavy chain variable region sequence thereof comprising SEQ ID NO: 181, 176, 177, 178, 179, 180 or 28, a light chain variable region sequence comprising SEQ ID NO: 183, 182 or 29, sequences of HCDR1, HCDR2, HCDR3, LCDR1, LCDR2 and LCDR3 respectively comprising SEQ ID NOs: 111, 173, 112, 174 or 175, 113, 114, 115 and 116, and sequences of a heavy chain and a light chain respectively comprising SEQ ID NOs: 226 and 227; and a corresponding pharmaceutical composition, coding nucleic acid, host cell, detection method and treatment method.

4. Claims 1-15 (in part) relate to anti-ILT4 antibody huP2-E8 or P2-E8, a heavy chain variable region sequence thereof comprising SEQ ID NO: 193, 190, 191, 192 or 10, a light chain variable region sequence comprising SEQ ID NO: 194, 195, 196 or 11, sequences of HCDR1, HCDR2, HCDR3, LCDR1, LCDR2 and LCDR3 respectively comprising SEQ ID NOs: 69, 189, 70 or 188, 71, 61, 62 and 72, and sequences of a heavy chain and a light chain respectively comprising SEQ ID NOs: 230 and 231; and a corresponding pharmaceutical composition, coding nucleic acid, host cell, detection method and treatment method.

5. Claims 1-15 (in part) relate to anti-ILT4 antibody P2 or P2m, a heavy chain variable region sequence thereof comprising SEQ ID NO: 163, a light chain variable region sequence comprising SEQ ID NO: 225 or 164, sequences of HCDR1, HCDR2, HCDR3, LCDR1, LCDR2 and LCDR3 respectively comprising SEQ ID NOs: 165, 166, 167, 224 or 168, 169 and 170, and sequences of a heavy chain and a light chain respectively comprising SEQ ID NOs:

Form PCT/ISA/210 (continuation of first sheet) (July 2022)

## INTERNATIONAL SEARCH REPORT

International application No.

**PCT/CN2023/117653**

**Box No. III      Observations where unity of invention is lacking (Continuation of item 3 of first sheet)**

238 and 239; and a corresponding pharmaceutical composition, coding nucleic acid, host cell, detection method and treatment method.

6. Claims 1-15 (in part) relate to anti-ILT4 antibody huP1-G7 or P1-G7, a heavy chain variable region sequence thereof comprising SEQ ID NO: 197, 198 or 36, a light chain variable region sequence comprising SEQ ID NO: 199, 200 or 37, sequences of HCDR1, HCDR2, HCDR3, LCDR1, LCDR2 and LCDR3 respectively comprising SEQ ID NOs: 105, 125, 126, 127, 109 and 128, and sequences of a heavy chain and a light chain respectively comprising SEQ ID NOs: 232 and 233; and a corresponding pharmaceutical composition, coding nucleic acid, host cell, detection method and treatment method.

7. Claims 1-15 (in part) relate to anti-ILT4 antibody huP1-D7 or P1-D7, a heavy chain variable region sequence thereof comprising SEQ ID NO: 217, 218, 219, 220, 221 or 42, a light chain variable region sequence comprising SEQ ID NO: 222, 223 or 43, sequences of HCDR1, HCDR2, HCDR3, LCDR1, LCDR2 and LCDR3 respectively comprising SEQ ID NOs: 141, 215 or 142, 216 or 143, 144, 145 and 146, and sequences of a heavy chain and a light chain respectively comprising SEQ ID NOs: 236 and 237; and a corresponding pharmaceutical composition, coding nucleic acid, host cell, detection method and treatment method.

8. Claims 1-15 (in part) relate to anti-ILT4 antibody P1-C4, a heavy chain variable region sequence thereof comprising SEQ ID NO: 34, a light chain variable region sequence comprising SEQ ID NO: 35, and sequences of HCDR1, HCDR2, HCDR3, LCDR1, LCDR2 and LCDR3 respectively comprising SEQ ID NOs: 99, 122, 123, 114, 109 and 124; and a corresponding pharmaceutical composition, coding nucleic acid, host cell, detection method and treatment method.

9. Claims 1-15 (in part) relate to anti-ILT4 antibody P2-E7, a heavy chain variable region sequence thereof comprising SEQ ID NO: 18, a light chain variable region sequence comprising SEQ ID NO: 19, and sequences of HCDR1, HCDR2, HCDR3, LCDR1, LCDR2 and LCDR3 respectively comprising SEQ ID NOs: 73, 87, 88, 61, 89 and 90; and a corresponding pharmaceutical composition, coding nucleic acid, host cell, detection method and treatment method.

10. Claims 1-15 (in part) relate to anti-ILT4 antibody P2-G4, a heavy chain variable region sequence thereof comprising SEQ ID NO: 20, a light chain variable region sequence comprising SEQ ID NO: 21, and sequences of HCDR1, HCDR2, HCDR3, LCDR1, LCDR2 and LCDR3 respectively comprising SEQ ID NOs: 91, 92, 93, 85, 62 and 94; and a corresponding pharmaceutical composition, coding nucleic acid, host cell, detection method and treatment method.

11. Claims 1-15 (in part) relate to anti-ILT4 antibody P2-E11, a heavy chain variable region sequence thereof comprising SEQ ID NO: 24, a light chain variable region sequence comprising SEQ ID NO: 25, and sequences of HCDR1, HCDR2, HCDR3, LCDR1, LCDR2 and LCDR3 respectively comprising SEQ ID NOs: 99, 100, 101, 102, 103 and 104; and a corresponding pharmaceutical composition, coding nucleic acid, host cell, detection method and treatment method.

12. Claims 1-15 (in part) relate to anti-ILT4 antibody P1-E11, a heavy chain variable region sequence thereof comprising SEQ ID NO: 44, a light chain variable region sequence comprising SEQ ID NO: 45, and sequences of HCDR1, HCDR2, HCDR3, LCDR1, LCDR2 and LCDR3 respectively comprising SEQ ID NOs: 147, 148, 149, 150, 151 and 152; and a corresponding pharmaceutical composition, coding nucleic acid, host cell, detection method and treatment method.

13. Claims 1-15 (in part) relate to anti-ILT4 antibody P2-B9, a heavy chain variable region sequence thereof comprising SEQ ID NO: 46, a light chain variable region sequence comprising SEQ ID NO: 47, and sequences of HCDR1, HCDR2, HCDR3, LCDR1, LCDR2 and LCDR3 respectively comprising SEQ ID NOs: 153, 154, 155, 114, 133 and 119; and a corresponding pharmaceutical composition, coding nucleic acid, host cell, detection method and treatment method.

14. Claims 1-15 (in part) relate to anti-ILT4 antibody P2-C2, a heavy chain variable region sequence thereof comprising SEQ ID NO: 48, a light chain variable region sequence comprising SEQ ID NO: 49, and sequences of HCDR1, HCDR2, HCDR3, LCDR1, LCDR2 and LCDR3 respectively comprising SEQ ID NOs: 58, 156, 157, 158, 62 and 159; and a corresponding pharmaceutical composition, coding nucleic acid, host cell, detection method and treatment method.

15. Claims 1-15 (in part) relate to anti-ILT4 antibody P2-E10, a heavy chain variable region sequence thereof comprising SEQ ID NO: 50, a light chain variable region sequence comprising SEQ ID NO: 51, and sequences of HCDR1, HCDR2, HCDR3, LCDR1, LCDR2 and LCDR3 respectively comprising SEQ ID NOs: 105, 160, 161, 114,

Form PCT/ISA/210 (continuation of first sheet) (July 2022)

## INTERNATIONAL SEARCH REPORT

International application No.

**PCT/CN2023/117653**

**Box No. III    Observations where unity of invention is lacking (Continuation of item 3 of first sheet)**

109 and 162; and a corresponding pharmaceutical composition, coding nucleic acid, host cell, detection method and treatment method.

The same or corresponding technical feature between the inventions is an anti-ILT4 antibody; however, the technical feature falls within the prior art (see, for example, documents WO 2022060767 A1 and WO 2018187518 A1). Therefore, the inventions do not have a same or corresponding special technical feature, do not fall within a single general inventive concept, and therefore lack unity of invention and do not comply with PCT Rule 13.1, 13.2 and 13.3.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **Parts of claims 1-15 that relate to SEQ ID NOs: 32, 33, 105, 109, 114, 119-121, 184-187, 228 and 229**

**Remark on Protest**      ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

| | | | |
|---|---|---|---|
| **INTERNATIONAL SEARCH REPORT**<br>**Information on patent family members** | | International application No.<br>**PCT/CN2023/117653** | |

| Patent document<br>cited in search report | Publication date<br>(day/month/year) | Patent family member(s) | Publication date<br>(day/month/year) |
|---|---|---|---|
| WO 2022060767 A1 | 24 March 2022 | US 2023365678 A1 | 16 November 2023 |
| | | AU 2021345096 A1 | 20 April 2023 |
| | | KR 20230069965 A | 19 May 2023 |
| | | JP 2023541656 A | 03 October 2023 |
| | | EP 4213879 A1 | 26 July 2023 |
| | | CA 3192390 A1 | 24 March 2022 |
| CN 113056483 A | 29 June 2021 | CA 3104530 A1 | 16 January 2020 |
| | | CL 2021000011 A1 | 24 May 2021 |
| | | US 2020079848 A1 | 12 March 2020 |
| | | US 11401328 B2 | 02 August 2022 |
| | | TW 202012438 A | 01 April 2020 |
| | | AU 2019302454 A1 | 25 February 2021 |
| | | MX 2021000009 A | 09 March 2021 |
| | | SG 11202100096 XA | 25 February 2021 |
| | | PE 20211604 A1 | 23 August 2021 |
| | | EP 3820904 A2 | 19 May 2021 |
| | | WO 2020014132 A2 | 16 January 2020 |
| | | WO 2020014132 A3 | 20 February 2020 |
| | | KR 20210030405 A | 17 March 2021 |
| | | BR 112020027095 A2 | 30 March 2021 |
| | | CO 2021000044 A2 | 19 April 2021 |
| | | US 2023174647 A1 | 08 June 2023 |
| | | JP 2022513420 A | 08 February 2022 |
| | | IL 279894 A | 01 March 2021 |
| CN 110719917 A | 21 January 2020 | ECSP 19072235 A | 27 December 2019 |
| | | TN 2019000272 A1 | 07 January 2021 |
| | | EP 3606958 A1 | 12 February 2020 |
| | | TW 201839014 A | 01 November 2018 |
| | | TWI 796329 B | 21 March 2023 |
| | | AR 111362 A1 | 03 July 2019 |
| | | DOP 2019000253 A | 15 December 2019 |
| | | US 2022002403 A1 | 06 January 2022 |
| | | WO 2018187518 A1 | 11 October 2018 |
| | | IL 269593 A | 28 November 2019 |
| | | PE 20191813 A1 | 26 December 2019 |
| | | SG 11201909081 YA | 30 October 2019 |
| | | UA 126865 C2 | 15 February 2023 |
| | | CO 2019011155 A2 | 21 October 2019 |
| | | CA 3057378 A1 | 11 October 2018 |
| | | US 2022033496 A1 | 03 February 2022 |
| | | PH 12019502275 A1 | 21 September 2020 |
| | | AU 2018248294 A1 | 10 October 2019 |
| | | AU 2018248294 B2 | 05 August 2021 |
| | | GEP 20227440 B | 25 November 2022 |
| | | JOP 20190236 A1 | 06 October 2019 |
| | | JOP 20190236 B1 | 17 September 2023 |
| | | CL 2019002855 A1 | 21 February 2020 |
| | | EA 201992402 A1 | 19 February 2020 |
| | | MX 2019011927 A | 10 February 2020 |
| | | JP 2022084773 A | 07 June 2022 |

Form PCT/ISA/210 (patent family annex) (July 2022)

## INTERNATIONAL SEARCH REPORT
### Information on patent family members

International application No.

**PCT/CN2023/117653**

| Patent document cited in search report | | | Publication date (day/month/year) | Patent family member(s) | | | Publication date (day/month/year) |
|---|---|---|---|---|---|---|---|
| | | | | AU | 2021225143 | A1 | 30 September 2021 |
| | | | | CR | 20190459 | A | 14 February 2020 |
| | | | | JP | 2020519235 | A | 02 July 2020 |
| | | | | JP | 7045392 | B2 | 31 March 2022 |
| | | | | KR | 20190136064 | A | 09 December 2019 |
| | | | | KR | 102357823 | B1 | 28 January 2022 |
| | | | | BR | 112019021000 | A2 | 05 May 2020 |
| | | | | NI | 201900103 | A | 31 October 2019 |
| | | | | US | 2018298096 | A1 | 18 October 2018 |
| | | | | US | 11053315 | B2 | 06 July 2021 |
| US | 2022056128 | A1 | 24 February 2022 | AU | 2019343131 | A1 | 29 April 2021 |
| | | | | KR | 20210108940 | A | 03 September 2021 |
| | | | | CA | 3111862 | A1 | 26 March 2020 |
| | | | | JP | 2022501428 | A | 06 January 2022 |
| | | | | EP | 3852805 | A1 | 28 July 2021 |
| | | | | EP | 3852805 | A4 | 15 June 2022 |
| | | | | WO | 2020061059 | A1 | 26 March 2020 |

Form PCT/ISA/210 (patent family annex) (July 2022)

# EP 4 585 618 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202211093055
- WO 2018187518 A1
- WO 2019126514 A2
- US 5821337 A
- US 7527791 B
- US 6982321 B
- US 7087409 B

- US 5959177 A
- US 6040498 A
- US 6420548 B
- US 7125978 B
- US 6417429 B
- WO 2020156509 A1

### Non-patent literature cited in the description

- *J. biol. chem.*, 1968, vol. 243, 3558
- **MARTIN, ACR**. *Protein Sequence and Structure Analysis of Antibody Variable Domains*, 2001
- **LEFRANC, M.P. et al.** *Dev. Comp. Immunol.*, 2003, vol. 27, 55-77
- *Front Immunol*, 16 October 2018, vol. 9, 2278
- *Prot. Sci.*, 2000, vol. 9, 487-496
- *Protein Engineering*, 1994, vol. 7, 697
- **ALMAGRO ; FRANSSON**. *Front. Biosci.*, 2008, vol. 13, 1619-1633
- **RIECHMANN et al.** *Nature*, 1988, vol. 332, 323-329
- **QUEEN et al.** *Proc. Nat'l Acad. Sci. USA*, 1989, vol. 86, 10029-10033
- **KASHMIRI et al.** *Methods*, 2005, vol. 36, 25-34
- **PADLAN**. *Mol. Immunol.*, 1991, vol. 28, 489-498
- **DALL' ACQUA et al.** *Methods*, 2005, vol. 36, 43-60

- **OSBOURN et al.** *Methods*, 2005, vol. 36, 61-68
- **KLIMKA et al.** *Br. J. Cancer*, 2000, vol. 83, 252-260
- **SIMS et al.** *J. Immunol.*, 1993, vol. 151, 2296
- **CARTER et al.** *Proc. Natl. Acad. Sci. USA*, 1992, vol. 89, 4285
- **PRESTA et al.** *J. Immunol.*, 1993, vol. 151, 2623
- **BACA et al.** *J. Biol. Chem.*, 1997, vol. 272, 10678-10684
- **ROSOK et al.** *J. Biol. Chem.*, 1996, vol. 271, 22611-22618
- **YAZAKI, P. ; WU, A.M.** Methods in Molecular Biology. Humana Press, 2004, vol. 248, 255-268
- Antibodies: A Laboratory Manual and Molecular Cloning: A Laboratory Manual. Cold Spring Harbor Laboratory